# EUROPÄISCHE PATENTSCHRIFT

(11) **EP 3 210 669 B1**
(45) Veröffentlichungstag und Bekanntmachung des Hinweises auf die Patenterteilung: **07.11.2018**
(21) Anmeldenummer: 17157310.8
(22) Anmeldetag: 22.02.2017
(51) Int. Cl.: B01L 1/02, B01L 9/06, B01L 3/00, G21F 7/005

(54) **VERFAHREN ZUM ÜBERFÜHREN EINER MEHRZAHL VON BEHÄLTERN UND/ODER VERSCHLUSSELEMENTEN IN EINEN REINRAUM SOWIE TRANSPORT- UND VERPACKUNGSBEHÄLTER**
METHOD FOR TRANSFERRING A NUMBER OF CONTAINERS AND/OR CLOSURE ELEMENTS IN A CLEAN ROOM AND TRANSPORT AND PACKAGING CONTAINER
PROCÉDÉ DE TRANSFERT D'UNE PLURALITÉ DE RÉCIPIENTS ET/OU D'ÉLÉMENTS DE FERMETURE DANS UNE SALLE BLANCHE ET RÉCIPIENT DE TRANSPORT ET D'EMBALLAGE

(30) Priorität: 26.02.2016 DE 102016103404
(43) Veröffentlichungstag der Anmeldung: 30.08.2017
(73) Patentinhaber: Schott AG, 55122 Mainz (DE)
(72) Erfinder: DEUTSCHLE, Gregor Fritz, 65510 Idstein (DE); PAWLOWSKI, Edgar, 55271 Stadecken-Elsheim (DE)
(74) Vertreter: 2K Patentanwälte Blasberg Kewitz & Reichel

(56) Entgegenhaltungen:
- WO-A1-95/34078
- DE-A1-102013 114 404
- DE-T2- 69 307 433
- DE-T2- 69 614 592
- DE-U1-202013 007 581
- US-A- 5 372 787

## Beschreibung

### GEBIET DER ERFINDUNG

Die vorliegende Erfindung betrifft ein Verfahren zum Überführen einer Mehrzahl von Behältern zur Aufbewahrung von Substanzen für medizinische, pharmazeutische oder kosmetische Zwecke und/oder von Verschlusselementen hierfür in einen Reinraum, und betrifft weiter einen Transport- und Verpackungsbehälter und ein Verpackungsgebilde hierfür sowie Verwendungen hiervon.

### STAND DER TECHNIK

Als Behälter (Container) zur Aufbewahrung und Lagerung von medizinischen, pharmazeutischen oder kosmetischen Präparaten mit Verabreichung in flüssiger Form, insbesondere in vordosierten Mengen, werden in großem Umfang Medikamentenbehälter, wie beispielsweise Fläschchen (vials), Ampullen, Spritzen oder Karpulen, eingesetzt, die aus Kunststoff oder Glas hergestellt sind und kostengünstig in großen Mengen erhältlich sind. Für eine möglichst wirtschaftliche Befüllung oder Weiterverarbeitung der Behälter unter sterilen Bedingungen werden in zunehmendem Maße Konzepte eingesetzt, bei denen die Behälter gleich beim Hersteller der Behälter in einen Transport- und Verpackungsbehälter oder in eine Verpackungseinheit steril verpackt werden und diese dann bei einem Pharmaunternehmen unter sterilen Bedingungen, insbesondere in einem Isolator, einem Reinraum oder Reinstraum oder unter RABS-Umgebung (Restricted Access Barriers Systems), ausgepackt und dann weiter verarbeitet werden.

Zu diesem Zweck werden im Stand der Technik immer häufiger sog. genestete (nested) Verpackungskonzepte eingesetzt, bei denen eine Mehrzahl von Medikamentenbehältern zum Transport in einer regelmäßigen Anordnung, beispielsweise in einer Matrixanordnung, in einer Haltestruktur (sog. nest) angeordnet sind, was Vorteile bei der automatisierten Weiterverarbeitung der Behälter ermöglicht, da die Behälter an kontrollierten Positionen und in einer vorgegebenen Anordnung an Bearbeitungsstationen übergeben werden können. Zum Transport wird die Haltestruktur dann in einen Transport- und Verpackungsbehälter (sog. tub) eingesetzt, dessen oberes Ende mittels einer Schutzfolie steril verschlossen ist. Der Transport- und Verpackungsbehälter ist üblicherweise in einem Umverpackungsbeutel steril verpackt und wird in dieser Form ausgeliefert.

Zur Übergabe der Medikamentenbehälter an einen Reinraum werden üblicherweise Materialschleusen eingesetzt, wie diese von der Handhabung von radioaktiven Substanzen bekannt sind. Dabei muss jedoch auch der Transport- und Verpackungsbehälter mit in den Reinraum übergeben werden. Eine direkte Übergabe nur der Haltestrukturen mit den daran gehaltenen Medikamentenbehältern in den Reinraum ist für genestete Verpackungskonzepte aus dem Stand der Technik nicht bekannt.

US 20150114853 A1 offenbart einen quaderförmigen Transport- und Verpackungsbehälter, in dem eine Mehrzahl von Behältern in einer vorbestimmten geometrischen Anordnung gehalten werden. Eine vordere Seitenwand des Transport- und Verpackungsbehälters kann zumindest teilweise geöffnet werden, sodass einzelne Behälter für eine Weiterverarbeitung gezielt aus dem Transport- und Verpackungsbehälter entnommen werden können und der Transport- und Verpackungsbehälter anschließend wieder verschlossen werden kann. Spezifische Maßnahmen zur Übergabe der Behälter in einen Reinraum sind jedoch nicht offenbart.

EP 2183166 B1 offenbart ein genestetes Verpackungskonzept. Ein Transport- und Verpackungsbehälter, der in einem Verpackungsbeutel aus Kunststoff steril verpackt ist, wird über eine Materialschleuse in einen Reinraum übergeben. Zur Übergabe wird der Verpackungsbeutel um die Materialschleuse des Reinraums herum vorübergehend an der Seitenwand des Reinraums gehalten und die Materialschleuse erst dann geöffnet, um das Eindringen von Partikeln und Keimen in den Reinraum zu verhindern. Der Transport- und Verpackungsbehälter muss mit in den Reinraum übergaben werden und kann erst im Reinraum für die Weiterverarbeitung der Medikamentenbehälter geöffnet werden.

US 20090100802 A1 offenbart eine Verpackungseinheit für Spritzenkörper, mit einem Transport- und Verpackungsbehälter, in dem eine Haltestruktur aufgenommen ist, an der die Spritzenkörper gehalten sind. Der Transport- und Verpackungsbehälter ist in einem Verpackungsbeutel aus einem gasundurchlässigen Kunststoff angeordnet, an den vor dem Zuschweißen ein Unterdruck angelegt wird, der bewirkt, dass der Verpackungsbeutel hinab in die Zwischenräume zwischen den an der Haltestruktur gehaltenen Spritzenkörpern gezogen wird. Zur Übergabe in einen Reinraum muss die Außenseite des Verpackungsbeutels aufwändig entkeimt werden.

US 8118167 B2 offenbart einen vergleichbaren Transport- und Verpackungsbehälter.

Das Öffnen der Transport- und Verpackungsbehälter und die Übergabe der darin aufbewahrten Behälter oder Haltestruktur mit den daran gehaltenen Behältern an eine Prozessstation unter sterilen Bedingungen ist jedoch umständlich, aufwändig, nicht ausreichend flexibel und gewährleistet nicht in jedem Fall die Einhaltung hoher Qualitätsstandards, insbesondere geringer Partikel- oder Keimkonzentrationen.

Für pharmazeutische oder medizinische Anwendungen werden die Transport- und Verpackungsbehälter zumeist nach dem "bag in bag"-Prinzip verpackt. Eine in einem Reinraum produzierte Verpackung wird in einem Umverpackungsbeutel verpackt, der auch im Reinraum hergestellt wurde. Meist werden die Verpackungen selbst zwei oder drei Mal mit einem jeweils weiteren Umverpackungsbeutel verpackt. Beim Transport kann die erste Verpackung (Umverpackungsbeutel 1) verschmutzen. Dann geht es durch eine Materialschleuse zu einem ersten Raum oder Reinraum mit einer vergleichsweise hohen Partikelkonzentration, wo die zweite Verpackung (Umverpackungsbeutel 2) entfernt wird, bis die sterile Verpackung schließlich in einen Reinraum mit einer niedrigeren Partikelkonzentration übergeben wird, wo die weitere Verarbeitung der Behälter und aufzunehmenden Substanzen erfolgt.

WO 95/34078 A1 offenbart ein Verfahren zum Überführen einer Mehrzahl von Pharmabehältern aus einem Ladewagen in einen Reinraum. Hierzu wird die Tür des Ladewagens mit der Tür des Reinraums gekoppelt und dann die Tür des Reinraums geöffnet. Damit die Tür des Ladewagens mit der Tür des Reinraums gekoppelt werden kann, muss diese jedoch gasundurchlässig ausgebildet sein.

DE 695 06 008 T2 offenbart ein entsprechendes Verfahren, bei dem vor der Übergabe in den Reinraum zunächst zwei Türen miteinander gekoppelt werden. Diese müssen gasundurchlässig ausgebildet sein.

DE 696 14 592 T2 offenbart vor der Übergabe von Vials aus einem Ladewagen in einen Gefriertrockner zunächst zwei Türen miteinander gekoppelt werden. Diese müssen gasundurchlässig ausgebildet sein.

Weitere entsprechende Verfahren sind in den Druckschriften DE 693 07 433 T2 und US 2005 / 0 042 710 A1 offenbart. Ein Abdichten der Zugriffsöffnung eines Transport- und Verpackungsbehälters mittels einer gasdurchlässigen Schutzfolie und eine Kopplung einer solchen gasdurchlässigen Schutzfolie mit einer Schleusentür sind in den vorgenannten Druckschriften nicht offenbart.

US 5 372 787 A offenbart einen quaderförmigen Transport- und Verpackungsbehälter, aus dem Pharmabehälter nicht über eine Seitenwand im Sinne dieser Anmeldung entnommen werden können, sondern erst nach Abnehmen eines oberen Deckels über die Oberseite des Transport- und Verpackungsbehälters.

DE 10 2013 114404 A1 der Anmelderin offenbart in der Fig. 5c einen quaderförmigen Transport- und Verpackungsbehälter, bei dem eine vordere Seitenwand durch eine sterile und gasdurchlässige Schutzfolie ersetzt ist, die die Schutzfolie eigentliche Seitenwand ausbildet. Eine Zugriffsöffnung in einer Seitenwand eines quaderförmigen Behälters im Sinne der voliegenden Anmeldung ist nicht offenbart

DE 20 2013 007581 U1 offenbart ebenfalls einen quaderförmigen Transport- und Verpackungsbehälter, ähnlich zur US 5 372 787 A. Objekte können aus dem Transport- und Verpackungsbehälter lediglich nach Abheben eines oberen Deckels entnommen werden.

### ZUSAMMENFASSUNG DER ERFINDUNG

Aufgabe der vorliegenden Erfindung ist es, ein verbessertes Verfahren zum Überführen einer Mehrzahl von Behältern, die zur Aufbewahrung von Substanzen für medizinische, pharmazeutische oder kosmetische Zwecke dienen, und/oder von Verschlusselementen hierfür aus einem Transport- und Verpackungsbehälter in einen Raum mit kontrolliert niedriger Partikel- oder Keimkonzentration, insbesondere in einen Reinraum, bereitzustellen, das einfach und kostengünstig ausgeführt werden kann und die Einhaltung hoher Qualitätsstandards zuverlässig gewährleistet. Gemäß weiteren Gesichtspunkten nach der vorliegenden Erfindung sollen ferner ein entsprechend ausgelegter Transport- und Verpackungsbehälter, ein Verpackungsgebilde für einen Transport- und Verpackungsbehälter sowie eine Verwendung hiervon bereitgestellt werden.

Diese Aufgaben werden durch ein Verfahren nach Anspruch 1 und einen Transport- und Verpackungsbehälter nach Anspruch 13 gelöst. Weitere vorteilhafte Ausführungsformen sind Gegenstand der rückbezogenen Unteransprüche,

Gemäß der vorliegenden Erfindung wird ein Verfahren zum Überführen einer Mehrzahl von Behältern zur Aufbewahrung von Substanzen für medizinische, pharmazeutische oder kosmetische Zwecke und/oder von Verschlusselementen für solche Behälter, die in einem Transport- und Verpackungsbehälters angeordnet sind, in einen Reinraum bereitgestellt, wobei der Transport- und Verpackungsbehälter zumindest eine Seitenwand Zugriffsöffnung aufweist, in der eine Zugriffsöffnung ausgebildet ist, die mittels einer gasdurchlässigen Schutzfolie oder gasdurchlässigen Abdeckung steril abgedichtet ist, in der eine Öffnung ausgebildet ist, die durch einen rahmenartigen Deckel mit einer Zugriffsöffnung zum Innenraum des Transport- und Verpackungsbehälters verschlossen ist, die mittels einer gasdurchlässigen Schutzfolie steril abgedichtet ist, mit den Schritten: Anordnen des Transport- und Verpackungsbehälters mit der Mehrzahl von darin aufgenommenen Behältern und/oder Verschlusselementen, sodass die Seitenwand des Transport- und Verpackungsbehälters unmittelbar an einer Seitenwand des Reinraums und in unmittelbarer Nähe zu einer Schleusentür des Reinraums angeordnet ist; Öffnen der Schleusentür, wobei durch Koppeln der gasdurchlässigen Schutzfolie mit der Schleusentür gleichzeitig die gasdurchlässige Schutzfolie von der Seitenwand oder dem rahmenartigen Deckel des Transport- und Verpackungsbehälters getrennt wird und die Zugriffsöffnung des Transport- und Verpackungsbehälters mit einem Innenraum des Reinraums in Verbindung steht; Überführen der Mehrzahl von Behältern und/oder Verschlusselementen aus dem Transport- und Verpackungsbehälters in den Innenraum des Reinraums; und Schließen der Schleusentür.

Ein Reinraum im Sinne der vorliegenden Erfindung, in den die Behälter zum Zwecke einer Weiterverarbeitung übergeben werden sollen, ist ein abgegrenzter Raum mit kontrolliert niedriger Partikel- bzw. Keimkonzentration, was in bekannter Weise durch geeignete Luftströmungen und Filterung der Luft erreicht werden kann. Zur Übergabe ist an einer Seitenwand des Reinraums eine Materialschleuse vorgesehen, insbesondere eine Schleusentür, die eine Zugangsöffnung zum Reinraum abgedichtet verschließt und kontrolliert geöffnet werden kann. Derartige Materialschleusen bzw. Schleusentüren können unterschiedliche geometrische Formen haben, beispielsweise kreis- oder rechteckförmig sein, und sind in einer geschlossenen Stellung abgedichtet an der Seitenwand des Reinraums angeordnet. Zu diesem Zweck ist um den Rand der Materialschleuse bzw. Schleusentür und/oder der Zugangsöffnung des Reinraums eine geeignete Dichtung, insbesondere eine elastische Dichtung, vorgesehen. Derartige Schleusentüren können durch eine Schwenkbewegung oder auch durch Verstellbewegungen senkrecht und/oder parallel zu der von der Seitenwand des Reinraums aufgespannten Ebene geöffnet und wieder geschlossen werden.

Zur Übergabe mit praktisch vernachlässigbarem Eintrag von Partikeln und Keimen in den Reinraum kann es grundsätzlich ausreichend sein, die Seitenwand des Transport- und Verpackungsbehälters vergleichsweise nahe, jedoch ohne Berührung in der Nähe der Schleusentür bzw. Seitenwand des Reinraums anzuordnen, insbesondere wenn der Luftdruck innerhalb des Reinraums und außerhalb des Reinraums identisch ist oder im Inneren des Reinraums ein nur geringer Überdruck vorherrscht. Beispielsweise kann die Breite eines Spalts zwischen der Seitenwand des Transport- und Verpackungsbehälters und der Schleusentür bzw. Seitenwand des Reinraums weniger als etwa ein Viertel, bevorzugter weniger als etwa ein Achtel und noch bevorzugter weniger als etwa ein Zehntel der maximalen Breite der Schleusentür betragen.

Wenn das Öffnen der Materialschleuse bzw. Schleusentür und des Transport- und Verpackungsbehälters, die Übergabe der Behälter und das erneute Schließen der Materialschleuse bzw. Schleusentür ausreichend schnell erfolgt, kann die Wahrscheinlichkeit des Eindringens von Partikeln und Keimen in den Reinraum auf ein akzeptabel niedriges Niveau reduziert werden. Zu diesem Zweck wird bevorzugt, wenn die Materialschleuse bzw. Schleusentür und der Transport- und Verpackungsbehälter zur Übergabe der Behälter gleichzeitig geöffnet und bevorzugt auch gleichzeitig wieder geschlossen werden, insbesondere durch gleichzeitiges synchrones Verschwenken oder geeignete vertikales und/oder horizontale Verstellung.

Gemäß einer weiteren Ausführungsform wird die Seitenwand des Transport- und Verpackungsbehälters so nahe an einer Seitenwand des Reinraums angeordnet, dass ein Spalt zwischen der Seitenwand des Transport- und Verpackungsbehälters und der Seitenwand des Reinraums mittels eines Dichtungselements abgedichtet ist Durch Koppeln der gasdurchlässigen Schutzfolie oder Abdeckung mit der Schleusentür wird beim Öffnen der Schleusentür gleichzeitig die gasdurchlässige Schutzfolie oder Abdeckung von der Seitenwand des Transport- und Verpackungsbehälters getrennt oder abgezogen, um die Zugriffsöffnung des Transport- und Verpackungsbehälters für die Überführung der Mehrzahl von Behältern aus dem Transport- und Verpackungsbehälter in den Innenraum des Reinraums freizugeben.

Aufgrund der Abdichtung des Spalts zwischen der Seitenwand des Transport- und Verpackungsbehälters und der Seitenwand des Reinraums ist ein Eindringen von Partikeln und Keimen von der Außenseite des Transport- und Verpackungsbehälters in den Innenraum des Reinraums ausgeschlossen. Wird die Seitenwand des Transport- und Verpackungsbehälters, die in Anlage zu der Seitenwand des Reinraums gebracht wird, zuvor, oder alternativ auch erst nach einem vorübergehenden Ankoppeln der vorderen Seitenwand des Transsportbchälters an die Seitenwand des Reinraums, geeignet entkeimt oder war diese zuvor steril verpackt, etwa in einem sterilen Kunststoffbeutel, so ist das Eindringen von Partikeln und Keimen auch von dieser Seitenwand des Transport- und Verpackungsbehälters in den Innenraum des Reinraums ausgeschlossen. Selbst wenn auf dieser Seitenwand des Transportbehälters Partikel und Keime vorhanden sein sollten, ist deren Eindringen in den Innenraum des Reinraums dennoch zuverlässig verhindert, weil die auf dieser Seitenwand vorgesehene Schutzfolie oder Abdeckung des Transport- und Verpackungsbehälters vor dem Öffnen der Schleusentür mit dieser gekoppelt wird. Bevorzugt wird zu diesem Zweck weiterhin ein Spalt zwischen dieser Seitenwand und der Außenseite der Schleusentür mittels eines Dichtungselements abgedichtet.

Damit die gasdurchlässige Schutzfolie beim Öffnen der Schleusentür von der Seitenwand getrennt werden kann, insbesondere abgezogen werden kann, muss die Stärke der Kraft, mit der die Schutzfolie oder Abdeckung mit der Schleusentür gekoppelt wird, größer sein als die Kraft, mit der die Schutzfolie oder Abdeckung auf der Seitenwand des Transport- und Verpackungsbehälters sitzt, beispielsweise anhaftet, aufgedrückt oder mit dieser verrastet ist.

Zu diesem Zweck kann die Kopplung insbesondere durch Verrastung, durch vorübergehende Fixierung der Schutzfolie an der Außenseite der Schleusentür mittels verstellbaren Greifern oder durch Ansaugen der Schutzfolie gegen die Außenseite der Schleusentür mittels Saugeinrichtungen erfolgen. Hierzu sind an der Schleusentür zweckmäßig geeignet verstellbare oder aktivierbare Halte- oder Kopplungseinrichtungen zum vorübergehenden Koppeln der Schutzfolie mit der Schleusentür vorgesehen, die über die Schleusentür selbst mit Energie für die Verstellung bzw. Aktivierung versorgt werden.

Gemäß einer weiteren Ausführungsform ist das Dichtungselement ein elastisches Dichtungselement und ist dieses auf der Seitenwand des Transport- und Verpackungsbehälters und/oder auf der Seitenwand des Reinraums angeordnet. Das Dichtungselement ist zweckmäßig umlaufend ausgebildet, um an geeigneter Stelle für die vorgenannte Abdichtung zu sorgen, insbesondere um den Spalt zwischen der Seitenwand des Transport- und Verpackungsbehälters und der Seitenwand des Reinraums und/oder den Spalt zwischen der Abdeckung und der Schleusentür abzudichten.

Erfindungsgemäß ist die Schutzfolie der Seitenwand des Transport- und Verpackungsbehälters gasdurchlässig, insbesondere eine sterile, gasdurchlässige Schutzfolie, insbesondere eine gasdurchlässige Kunststofffolie, bestehend aus einem Geflecht von Kunststofffasern, beispielsweise aus Polypropylen-Fasem. Die sterile, gasdurchlässige Schutzfolie ist bevorzugt eine Tyvek®-Schutzfolie, die auf einen Rand der Seitenwand des Transport- und Verpackungsbehälters aufgeklebt ist, sodass diese einfach abgezogen werden kann.

Die Abdeckung kann gemäß einer Ausführungsform der Erfindung auch als Deckel aus einem gasundurchlässigen Material, beispielsweise aus einem Kunststoff, ausgebildet sein, der in geeigneter Weise mit der Seitenwand des Transport- und Verpackungsbehälters lösbar verbunden ist, um die Zugriffsöffnung steril zu verschließen. Zu diesem Zweck kann der Deckel mit der Seitenwand des Transport- und Verpackungsbehälters geeignet verrastet sein. Dabei ist der Deckel in der Art eines Rahmens ausgebildet, mit einer Öffnung, die mit einer gasdurchlässigen Schutzfolie, wie vorstehend beschrieben, steril verschlossen ist. Diese Schutzfolie kann insbesondere auf den Rahmen aufgeklebt sein und so einfach abgezogen werden. Grundsätzlich kann diese Öffnung auch als Zugriffsöffnung zum Innenraum des Transport- und Verpackungsbehälters zur Entnahme der Behälter und/oder Verschlusselemente dienen.

Gemäß einer weiteren Ausführungsform umfasst das Verfahren weiter den Schritt, dass die in dem Transport- und Verpackungsbehälter aufgenommenen Behälter und/oder Verschlusselemente durch die sterile, gasdurchlässige Schutzfolie oder Abdeckung hindurch durch Einströmen eines Gases oder Dampfes sterilisiert werden. Diese Entkeimung kann bevorzugt über die vordere und/oder hintere Seitenwand des Transport- und Verpackungsbehälters erfolgen, sodass der Innenraum eines Transport- und Verpackungsbehälters auch dann noch zuverlässig entkeimt werden kann, wenn mehrere Transport- und Verpackungsbehälter gestapelt übereinander angeordnet sind. Wenn auf der vorderen und hinteren Stirnseite des Transport- und Verpackungsbehälters eine gasdurchlässige Schutzfolie oder Abdeckung vorgesehen ist, wie vorstehend beschrieben, kann eine Entkeimung auch beispielsweise über die hintere Seitenwand erfolgen, bevor die Schleusentür zum Reinraum geöffnet wird, um das Risiko des Eindringens von Partikeln oder Keimen in den Reinraum noch weiter zu reduzieren.

Gemäß einer weiteren Ausführungsform ist an der Schleusentür in Entsprechung zu einem Kleberand, entlang dem die sterile, gasdurchlässige Schutzfolie auf den Rand der Seitenwand des Transport- und Verpackungsbehälters oder auf den rahmenartigen Deckel aufgeklebt ist, eine Heizeinrichtung angeordnet, wobei durch Aktivieren der Heizeinrichtung der Kleberand erwärmt und erweicht wird, und die sterile, gasdurchlässige Schutzfolie so mit der Schleusentür gekoppelt wird, dass die Schutzfolie nach dem Erweichen des Kleberands durch Öffnen der Schleusentür von der Seitenwand des Transport- und Verpackungsbehälters oder von dem rahmenartigen Deckel abgezogen wird. Hierzu kann es ausreichend sein, wenn die Stärke der Kopplung der Schutzfolie mit der Schleusentür stärker ist als die verbleibende, geringe Haftkraft der Schutzfolie an dem Kleberand nach dem Erweichen des Klebers.

Gemäß einer weiteren Ausführungsform ist die gasdurchlässige Schutzfolie auf der Seitenwand des Transport- und Verpackungsbehälters oder auf einem darauf ausgebildeten rahmenartigen Vorsprung angeordnet, sind in der Seitenwand oder dem rahmenartigen Vorsprung eine Mehrzahl von Ausnehmungen ausgebildet, und sind korrespondierend zu den Ausnehmungen auf der Außenseite der Schleusentür verstellbare Greifer angeordnet, wobei die Greifer so verstellt werden, dass der Transport- und Verpackungsbehälter in einer ersten Stellung der Greifer ungehindert in die Nähe der Schleusentür des Reinraums gebracht wird, dasss die Greifer anschließend in eine zweite Stellung verstellt werden, in welcher die Greifer in die entsprechenden Ausnehmungen auf der Außenseite der Schleusentür eingreifen und dabei die Schutzfolie hintergreifen, um die Schutzfolie vorübergehend an der Schleusentür zu fixieren, und wobei die Schleusentür in der zweiten Stellung der Greifer geöffnet wird, wodurch die Schutzfolie von der Seitenwand, von der Abdeckung oder von dem rahmenartigen Vorsprung abgezogen wird, und wobei die Greifer nach Schließen der Schleusentür zurück in die erste Stellung verstellt werden, wodurch die Zugriffsöffnung des Transport- und Verpackungsbehälters wieder verschlossen wird.

Gemäß einer weiteren Ausführungsform sind die Behälter und/oder Verschlusselemente gemeinsam in einer Haltestruktur gehalten und werden diese aus dem Transport- und Verpackungsbehälters in den Innenraum des Reinraums durch Verschieben der Haltestruktur überführt. Dies ermöglicht eine rasche Überführung der Behälter und/oder Verschlusselemente in den Reinraum, da gleichzeitig sämtliche Behälter durch Handhabung der Haltestruktur weiter transportiert werden können. Eine solche Haltestruktur kann insbesondere als Nest ausgebildet sein, wie diese aus genesteten Verpackungskonzepten aus dem Stand der Technik bekannt ist.

Bei den Verschlusselementen, die in den Reinraum überführt werden sollen, kann es sich insbesondere um Stopfen, Kolbenpfropfen oder Verschlusskappen handeln, die in dem Reinraum weiterverwendet werden sollen.

Gemäß einer weiteren Ausführungsform weist die Haltestruktur ein kastenförmiges Unterteil mit einem Boden, auf dem die Behälter unmittelbar abgestützt sind, und ein kastenförmiges Oberteil auf, das unmittelbar oder mittelbar auf den oberen Enden der Behälter aufliegt und ein Eindringen von Partikeln oder Keimen in die Haltestruktur und insbesondere in Befüllöffnungen an den oberen Enden von noch nicht verschlossenen Behältern verhindert.

Gemäß einer weiteren Ausführungsform wird die Haltestruktur zum Überführen aus dem Transport- und Verpackungsbehälters in den Innenraum des Reinraums auf Führungsschienen geführt, was insbesondere dann von Vorteil ist, wenn der Boden des Transport- und Verpackungsbehälters nicht bündig mit dem unteren Rand der Zugriffsöffnung auf der Seitenwand des Transport- und Verpackungsbehälters ist.

Gemäß einer bevorzugten weiteren Ausführungsform fluchtet ein unterer Rand der Zugriffsöffnung des Transport- und Verpackungsbehälters mit dem Boden des Transport- und Verpackungsbehälters, sodass die Haltestruktur zum Überführen in den Reinraum auch unmittelbar auf dem Boden des Transport- und Verpackungsbehälters verschoben werden kann.

Gemäß einer weiteren Ausführungsform wird der Transport- und Verpackungsbehälter in einem sterilen Verpackungsbeutel steril verpackt bereitgestellt und ist dem Reinraum ein erster Raum, insbesondere ein erster Reinraum, mit einer höheren Partikelkonzentration vorgeschaltet, wobei das Verfahren die weiteren Schritte umfasst: Entkeimen einer Außenseite des Verpackungsbeutels in dem ersten Raum, insbesondere in dem ersten Reinraum; und Entnehmen des Transport- und Verpackungsbehälters aus dem sterilen Verpackungsbeutel nach dem Entkeimen. Bei dem ersten Raum kann es sich grundsätzlich um eine normale Arbeitsumgebung handeln, beispielsweise eine Arbeitshalle eines Pharmaabfüllbetriebs, in der der eigentliche Reinraum angeordnet ist, in den die Behälter zu deren Weiterverarbeitung darin steril übergeben werden sollen. Grundsätzlich kann jedoch auch bereits dieser erste Raum ein abgegrenzter Raum mit einer kontrollieren Umgebung sein, insbesondere mit einer kontrollierten Partikel- und Keimkonzentration, der über eine Materialschleuse mit dem eigentlichen Reinraum verbunden ist, in dem die Behälter weiterverarbeitet werden sollen.

Gemäß einer weiteren Ausführungsform wird die Mehrzahl von Behältern nach einer Bearbeitung in dem Reinraum mittels der folgenden Schritte in den Transport- und Verpackungsbehälter eingebracht: Anordnen des Transport- und Verpackungsbehälters in dem ersten Raum, insbesondere in den ersten Reinraum, mit der höheren Partikelkonzentration, sodass eine Seitenwand des Transport- und Verpackungsbehälters nahe einer Schleusentür des Reinraums angeordnet ist; gleichzeitiges Öffnen der Seitenwand des Transport- und Verpackungsbehälters und der Schleusentür, sodass die Zugriffsöffnung des Transport- und Verpackungsbehälters mit dem Innenraum des Reinraums in Verbindung steht; Überführen der Mehrzahl von Behältern aus dem Innenraum des Reinraums in den Transport- und Verpackungsbehälter; Schließen der Schleusentür; Schließen des Transport- und Verpackungsbehälters mittels einer Abdeckung, insbesondere bewirkt durch das gleichzeitige Schließen der Schleusentür; Entkeimen der Außenseite des Transport- und Verpackungsbehälters in dem ersten Raum, insbesondere in dem ersten Reinraum, mit der höheren Partikelkonzentration; Einbringen des Transport- und Verpackungsbehälters nach dem Entkeimen der Außenseite in einen sterilen Verpackungsbeutel; und Verschließen des sterilen Verpackungsbeutels.

Gemäß einer weiteren bevorzugten Ausführungsform erfolgt die Übergabe der Behälter und/oder Verschlusselemente in den Reinraum, ohne dass die Haltestruktur senkrecht nach oben aus dem Transport- und Verpackungsbehälter herausgehoben werden muss. Die Orientierung der Haltestruktur bleibt vielmehr während der Übergabe erhalten, ohne dass die Haltestruktur hierzu verdreht werden muss. Bevorzugt erfolgt die Übergabe ausschließlich durch Verschieben der Haltestruktur in einer horizontalen Ebene, ohne dass die Haltestruktur hierzu angehoben zu werden braucht. Gemäß weiteren Ausführungsformen schließt dies nicht aus, dass die Haltestruktur nicht doch in geringem Umfange angehoben wird, etwa um diese über einen unteren Rand der Zugriffsöffnung drüber zu heben. In jedem Fall ist dieses Anheben im Vergleich zu einer vertikalen Erstreckung der Behälter und/oder Verschlusselemente im Wesentlichen vernachlässigbar, insbesondere kleiner als etwa ein Drittel, bevorzugter ein Viertel einer maximalen axialen Länge der Behälter und/oder Verschlusselement, die in den Reinraum überführt werden sollen. Deshalb sind erfindungsgemäß keine aufwändigen mechanischen Mechanismen zum Verschieben der Haltestruktur erforderlich.

Gemäß einem weiteren Gesichtspunkt der vorliegenden Erfindung, der auch mittels eines gesonderten Satzes von Patentansprüchen unabhängig zu dem vorgenannten Verfahren beansprucht werden kann, wird ein Transport- und Verpackungsbehälter zur Aufbewahrung von Behältern für Substanzen für medizinische, pharmazeutische oder kosmetische Zwecke und/oder von Verschlusselementen hierfür in einer vertikalen Ausrichtung bereitgestellt, insbesondere zur Verwendung in einem Verfahren, wie vorstehend ausgeführt, wobei der Transport- und Verpackungsbehälter quaderförmig ist und eine Oberseite, eine Unterseite und vier Seitenwände aufweist, die Behälter und/oder von Verschlusselemente in der vertikalen Ausrichtung senkrecht zu der Oberseite und der Unterseite ausgerichtet sind, jeweils zwei Seitenwände parallel und beabstandet zueinander gegenüberliegend angeordnet sind, wobei zumindest eine der Seitenwände mittels einer Abdeckung steril abgedichtet ist, und wobei die jeweilige Abdeckung abnehmbar ist, um einen Innenraum des Transport- und Verpackungsbehälters zur Entnahme der in dem Transport- und Verpackungsbehälter aufgenommenen Behälter freizugeben.

Erfindungsgemäß ist die Abdeckung eine gasdurchlässige Schutzfolie, um ein Sterilisieren des Innenraums des Transport- und Verpackungsbehälters und/oder der darin aufgenommenen Behälter und/oder Verschlusselemente durch Einströmen eines Gases durch die gasdurchlässige Schutzfolie oder Abdeckung zu ermöglichen. Die Schutzfolie dichtet die Zugriffsöffnung in der zumindest einen Seitenwand oder in einem rahmenartigen Deckel, der eine Öffnung in der zumindest einen Seitenwand verschließt, steril ab, sodass der Innenraum des Transport- und Verpackungsbehälters und/oder der darin aufgenommenen Behälter und/oder Verschlusselemente durch Einströmen eines Gases durch die gasdurchlässige Schutzfolie sterilisiert werden können und die Behälter und/oder Verschlusselemente durch Ablösen der gasdurchlässigen Schutzfolie von der zumindest einen Seitenwand oder dem rahmenartigen Deckel durch die Zugriffsöffnung seitlich und in der vertikalen Ausrichtung aus dem Transport- und Verpackungsbehälter entnommen werden können.

Bei der Seitenwand handelt es sich nicht um die Oberseite oder Unterseite des Transport- und Verpackungsbehälters, deren Flächeninhalt im Wesentlichen der bestimmungsgemäßen Standfläche des Transport- und Verpackungsbehälters bei vertikaler Ausrichtung der Behälter entspricht, sondern um eine Außenseite, die senkrecht oder im Wesentlichen senkrecht zu dieser Standfläche orientiert ist. Ist die Standfläche des Transport- und Verpackungsbehälters rechteckig ausgebildet, so entspricht diese Seitenwand bevorzugt der vorderen oder hinteren Seitenwand des Transport- und Verpackungsbehälters, die eine kleinere Standfläche als die Oberseite und Unterseite des Transport- und Verpackungsbehälters hat.

Gemäß einer weiteren Ausführungsform ist an dem Transport- und Verpackungsbehälter zumindest eine Kopplungseinrichtung zur vorübergehenden Kopplung des Transport- und Verpackungsbehälters an einer Seitenwand eines Reinraums vorgesehen, wobei die jeweilige Kopplungseinrichtung außerhalb der Abdeckung und einer Zugriffsöffnung des Transport- und Verpackungsbehälters vorgesehen ist.

Zur mechanischen Kopplung eignen sich dabei insbesondere verstellbare mechanische Kopplungseinrichtungen, wie beispielsweise Vertiefungen, Ausnehmungen oder Vorsprünge, die insbesondere auf der vorderen Seitenwand oder der Ober- und/oder Unterseite des Transport- und Verpackungsbehälters vorgesehen sein können und formschlüssig mit verstellbaren Halteklauen zusammenwirken können. Angedacht sind auch verstellbare Klemmschienen oder geeignet ausgebildete Elemente eines Bajonett-Verschlusses oder dergleichen. Die Kopplung kann jedoch grundsätzlich auch hydraulisch, pneumatisch, elektrisch oder magnetisch erfolgen.

Gemäß einer weiteren Ausführungsform ist um die gasdurchlässige Schutzfolie oder den rahmenartigen Deckel herum ein flacher oder ebener, umlaufender Anlageabschnitt ausgebildet, sodass bei Anlage des umlaufenden Anlageabschnitts an einem korrespondierend ausgebildeten umlaufenden Anlageabschnitt auf einer Seitenwand eines Reinraums ein Spalt zwischen der Schutzfolie oder dem rahmenartigen Deckel und der Seitenwand des Reinraums mittels eines elastischen Dichtungselements gegen die Umgebung hin abgedichtet werden kann.

Gemäß einer weiteren Ausführungsform ist die sterile, gasdurchlässige Schutzfolie eine gasdurchlässige Kunststofffolie, bestehend aus einem Geflecht von Kunststofffasern, beispielsweise aus Polypropylen-Fasern, und ist die sterile, gasdurchlässige Schutzfolie bevorzugt eine Tyvek®-Schutzfolie, die auf den flachen, umlaufenden Anlageabschnitt der jeweiligen Seitenwand des Transport- und Verpackungsbehälters aufgeklebt ist. Die gasdurchlässige Schutzfolie kann auf einen rahmenartig ausgebildeten Deckel aus einem gasundurchlässigen Material, insbesondere aus einem Kunststoff, angeordnet sein, in welchem eine Öffnung zum Zugriff auf den Innenraum des Transport- und Verpackungsbehälters ausgebildet ist, die mit einer gasdurchlässigen Schutzfolie, wie vorstehend beschrieben, steril verschlossen ist.

Gemäß einer weiteren Ausführungsform ist ein umlaufender Randbereich der gasdurchlässigen Schutzfolie durch einen umlaufenden, linienförmigen Schwächungsbereich in einen äußeren umlaufenden Abschnitt und einen inneren umlaufenden Abschnitt unterteilt. Beim Ankoppeln der Vorderseite des Transportbehälters an die Seitenwand des Reinraums kann so der äußere umlaufende Abschnitt zwischen dem Transportbehälter und der Seitenwand geklemmt gehalten werden, während der innere Abschnitt der Schutzfolie beim Öffnen der Schleusentür des Reinraums aufgrund einer vorübergehenden Kopplung der Schutzfolie mit der Schleusentür entlang des linienförmigen Schwächungsbereichs ohne weiteres abgezogen werden kann. Der Schwächungsbereich kann durch Ausbilden von punktförmigen Perforierungen oder Vertiefungen, beispielsweise durch Stanzen, Umformung, Laserablation oder dergleichen, entlang dem Rand der Abdeckung oder Schutzfolie ausgebildet sein. Im Falle einer Schutzfolie erstreckt sich der linienförmige Schwächungsbereich zweckmäßig innerhalb eines Kleberands, entlang dem die Schutzfolie mit der Vorderseite des Transportbehälters verbunden ist.

Gemäß einem weiteren Gesichtspunkt der vorliegenden Erfindung, der auch mittels eines gesonderten Satzes von Patentansprüchen unabhängig zu dem vorgenannten Verfahren beansprucht werden kann, wird ein Transport- und Verpackungsbehälter zur Aufbewahrung von Behältern für Substanzen für medizinische, pharmazeutische oder kosmetische Zwecke und/oder von Verschlusselementen hierfür, insbesondere zur Verwendung in dem vorstehend ausgeführten Verfahren, bereitgestellt, wobei der Transport- und Verpackungsbehälter wie hierin offenbart ausgebildet ist und in einem sterilen Verpackungsbeutel steril verpackt ist.

Gemäß einem weiteren Gesichtspunkt der vorliegenden Erfindung, der auch mittels eines gesonderten Satzes von Patentansprüchen unabhängig zu dem vorgenannten Verfahren beansprucht werden kann, wird eine Verwendung des Transport- und Verpackungsbehälters wie hierin offenbart in einem Verfahren wie hierin offenbart bereitgestellt.

### FIGURENÜBERSICHT

Nachfolgend wird die Erfindung in beispielhafter Weise und unter Bezugnahme auf die beigefügten Zeichnungen beschrieben werden, woraus sich weitere Merkmale, Vorteile und zu lösende Aufgaben ergeben werden. Es zeigen:
- Fig. 1a bis 1g: einen Transport- und Verpackungsbehälter gemäß verschiedenen Ausführungsformen nach der vorliegenden Erfindung;
- Fig. 1h: ein Verpackungsgebilde mit einem Transport- und Verpackungsbehälter gemäß der vorliegenden Erfindung;
- Fig. 2a bis 2d: ein Verfahren zum Überführen einer Mehrzahl von Behältern aus einem Transport- und Verpackungsbehälter in einen Reinraum gemäß einer ersten Ausführungsform der vorliegenden Erfindung;
- Fig. 3a bis 3e: ein Verfahren zum Überführen einer Mehrzahl von Behältern aus einem Transport- und Verpackungsbehälter in einen Reinraum gemäß einer weiteren Ausführungsform der vorliegenden Erfindung;
- Fig. 4a bis 4c: ein Verfahren zum Überführen einer Mehrzahl von Behältern aus einem Transport- und Verpackungsbehälter in einen Reinraum gemäß einer weiteren Ausführungsform der vorliegenden Erfindung;
- Fig. 5a bis 5e: eine Haltestruktur gemäß einer ersten Ausführungsform zur Verwendung bei einem Verfahren gemäß der vorliegenden Erfindung;
- Fig. 6a bis 6c: Einzelheiten von Haltestrukturen gemäß weiteren Ausführungsformen zur Verwendung bei einem Verfahren gemäß der vorliegenden Erfindung;
- Fig. 7a: ein Verfahren zum Überführen einer Mehrzahl von Behältern zur Aufbewahrung von Substanzen für medizinische, pharmazeutische oder kosmetische Zwecke in einen Reinraum gemäß einer weiteren Ausführungsform der vorliegenden Erfindung;
- Fig. 7b: ein Verfahren zum Überführen einer Mehrzahl von Behältern zur Aufbewahrung von Substanzen für medizinische, pharmazeutische oder kosmetische Zwecke in einen Reinraum gemäß einer weiteren Ausführungsform der vorliegenden Erfindung;
- Fig. 7c: ein Verfahren zum Überführen einer Mehrzahl von Behältern zur Aufbewahrung von Substanzen für medizinische, pharmazeutische oder kosmetische Zwecke aus einem Reinraum in einen Transport- und Verpackungsbehälter zur Ausbildung eines Verpackungsgebildes gemäß der vorliegenden Erfindung; und
- Fig. 8a bis 8c: weitere Möglichkeiten der vorübergehenden Kopplung eines Transport- und Verpackungsbehälter gemäß der vorliegenden Erfindung mit der Seitenwand eines Reinraums.

In den Figuren bezeichnen identische Bezugszeichen identische oder im Wesentlichen gleichwirkende Elemente oder Elementgruppen.

### AUSFÜHRLICHE BESCHREIBUNG VON AUSFÜHRUNGSBEISPIELEN

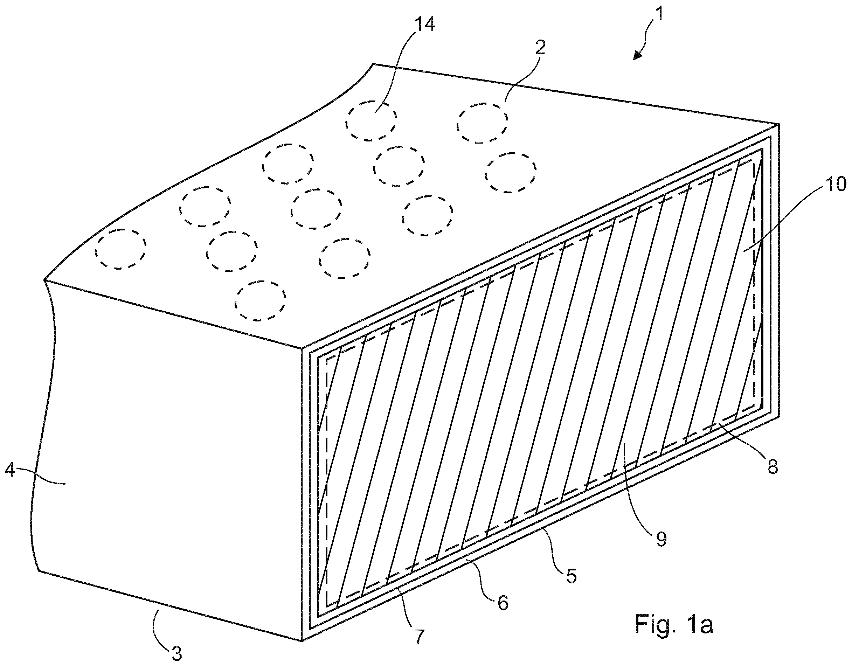

Die Fig. 1a zeigt einen Transport- und Verpackungsbehälter (nachfolgend auch verkürzt als Transportbehälter bezeichnet) 1 gemäß einer ersten Ausführungsform der vorliegenden Erfindung. Der Transportbehälter 1 ist insgesamt quaderförmig ausgebildet und weist eine Oberseite 2, eine Unterseite 3, zwei Längsseiten 4, eine vordere Seitenwand 5 und eine hintere Seitenwand (nicht gezeigt) auf. Auf der vorderen Seitenwand 5 ist eine Abdeckung 10 angebracht, die eine Zugriffsöffnung 9 zum Innenraum des Transportbehälters 1 steril abdeckt. Gemäß der Fig. 1a ist umlaufend um den Rand der Abdeckung 10 herum ein flacher Rand 6 ausgebildet, wobei der Rand der Abdeckung 10 beabstandet zum Rand der vorderen Seitenwand 5 ist. Der Transportbehälter 1 ist zweckmäßig aus einem Kunststoff ausgebildet, insbesondere mittels eines Spritzgussverfahrens, der ausreichend steif und mechanisch stabil ist, um den Innenraum mechanisch zu schützen.

Die Abdeckung 10 kann grundsätzlich als rahmenartiger Deckel aus gasundurchlässigen Material ausgebildet sein, in dem eine Zugriffsöffnung 9 ausgebildet ist, die mittels einer gasdurchlässigen Schutzfolie steril abgedichtet ist. Eine solche Abdeckung 10 kann in geeigneter Weise mit der vorderen Seitenwand 5 des Transportbehälters 1 lösbar verbunden sein. Hierzu kann der Deckel auf die vordere Seitenwand 5 aufgeschraubt sein, auf diese aufgesteckt sein oder beispielsweise in den Rand der Zugriffsöffnung 9 eingesteckt sein. Selbstverständlich kann die Abdeckung 10 auch auf die vordere Seitenwand 5 aufgeklebt sein.

Gemäß einer bevorzugten Ausführungsform ist die Abdeckung 10 eine sterile, gasdurchlässige Schutzfolie, insbesondere eine gasdurchlässige Kunststofffolie, bestehend aus einem Geflecht von Kunststofffasern, beispielsweise aus Polypropylen-Fasern (PP), und bevorzugt eine Tyvek®-Schutzfolie, die mittels eines Klebemittels entlang einem Kleberand 8 auf den Rand 6 auf der vorderen Seitenwand 5 des Transport- und Verpackungsbehälters 1 aufgeklebt ist. Eine solche Schutzfolie ist für Gas (beispielsweise Ethylenoxyd (ETO) oder H₂O₂) durchlässig, das durch die Schutzfolie in den Innenraum des Transportbehälters 1 einströmen und den Innenraum und die darin aufgenommenen Behälter sterilisieren kann. Für den Fall, dass die Abdeckung 10 gasundurchlässig ist, können auf einer der Seitenflächen des Transportbehälters 1, insbesondere auf der Oberseite 2, wie in der Fig. 1a dargestellt, oder auch in der Abdeckung 10 selbst eine Mehrzahl von Öffnungen oder Durchbrechungen 14 ausgebildet sein, die mittels einer sterilen, gasdurchlässigen Schutzfolie, wie vorstehend ausgeführt, steril verschlossen sind, sodass der Innenraum des Transportbehälters mit den darin aufgenommenen Behältern durch Einströmen eines Gases durch die Schutzfolie im Bereich der Durchbrechungen oder Öffnungen 14 hindurch sterilisiert werden kann.

Grundsätzlich möglich ist auch eine Entkeimung des Innenraums des Transportbehälters 1 mittels Dampfsterilisation, bei der ein heißer Dampf (beispielsweise 134°C/ 3 min) den Innenraum des Transportbehälters 1 durchströmt.

Der vorgenannte gasundurchlässig ausgebildete Deckel ist gemäß einer weiteren Ausführungsform der Erfindungrahmenartig ausgebildet, mit einer Zugriffsöffnung zum Innenraum des Transportbehälters, die mittels einer gasdurchlässigen Schutzfolie, wie vorstehend beschrieben, steril verschlossen ist, insbesondere auf den Rahmen aufgeklebt ist.

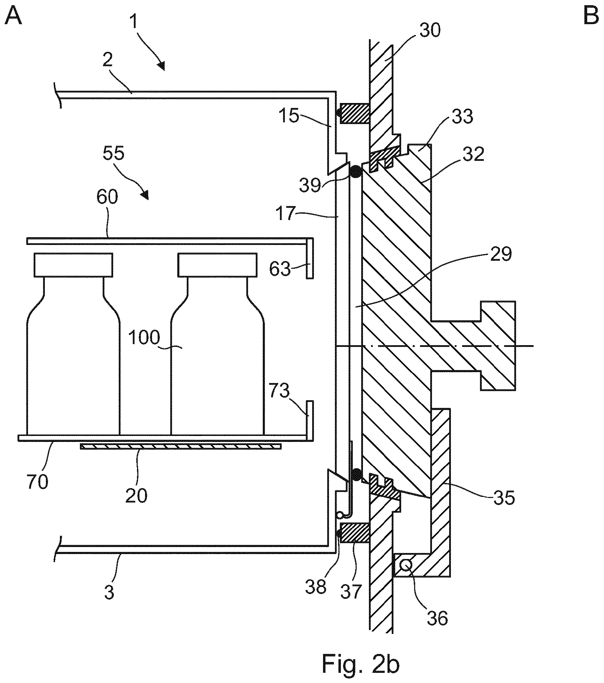

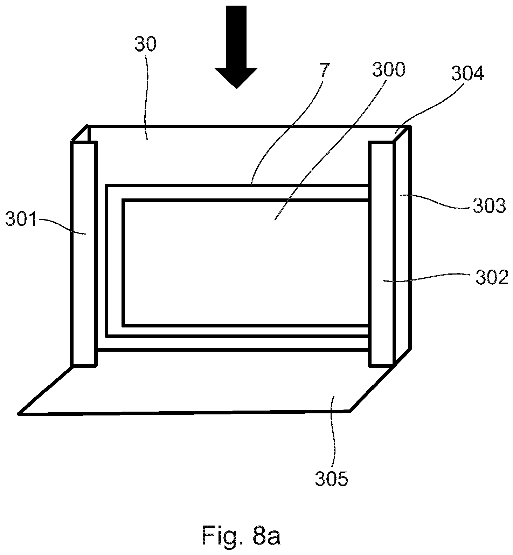

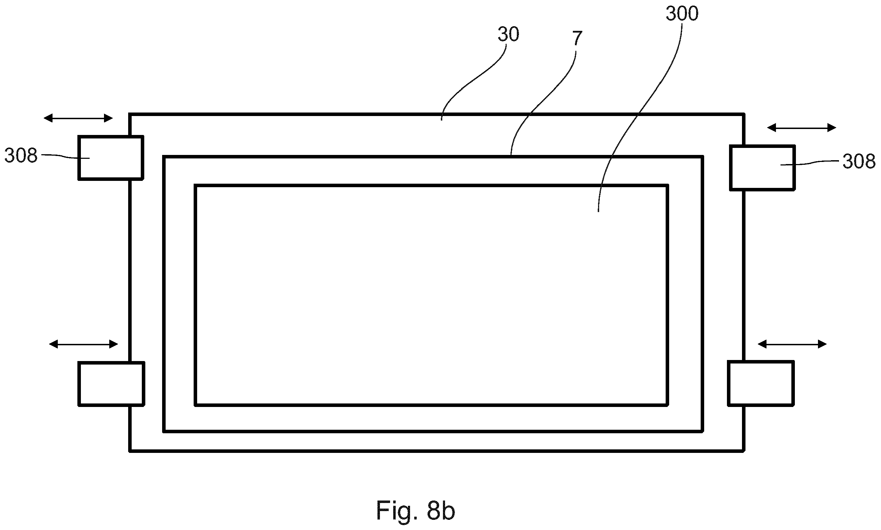

Gemäß der Fig. 1a ist auf dem Rand 6, beabstandet sowohl zum Rand der vorderen Seitenwand 5 als auch zum Rand der Abdeckung 10, ein elastisches Dichtungselement 7 vorgesehen, das beispielsweise in eine dort vorgesehene Nut eingesteckt ist oder mittels eines zwei-Komponenten(2K)-Spritzgussverfahrens einstückig mit dem Transportbehälter 1 ausgebildet ist. Dieses Dichtungselement 7 dient einer Abdichtung eines Spalts 29 (vgl. Fig. 2b) zwischen der Schleusentür 32 eines Reinraums B und der Abdeckung 10, wie nachfolgend ausgeführt. Seine Dichtungsfunktion kann jedoch auch durch eine entsprechende Dichtung auf der Außenseite der Schleusentür 32 des Reinraums B ersetzt oder gemeinsam mit dieser ausgeübt werden, wie in den Figuren 8a und 8b gezeigt.

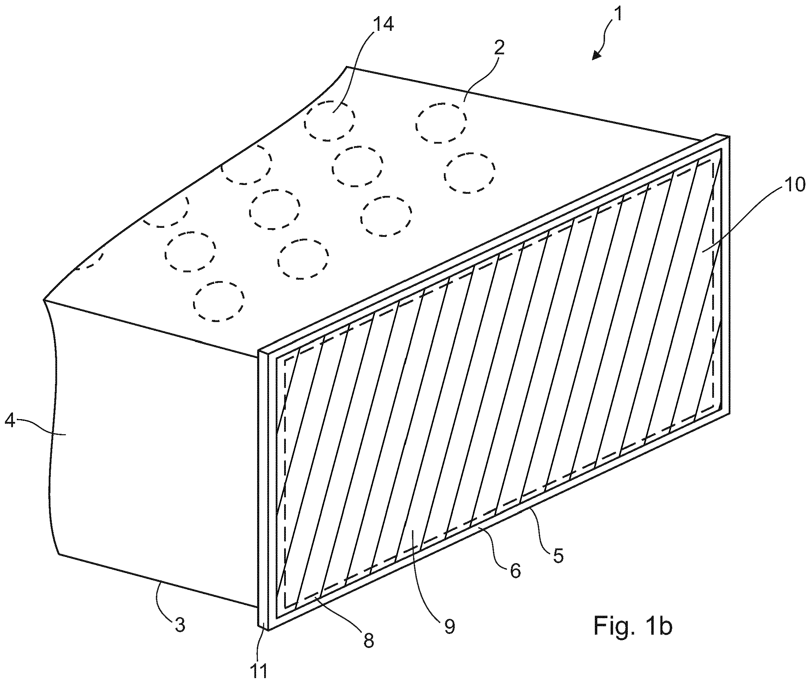

Die Fig. 1b zeigt eine weitere Ausführungsform eines solchen Transportbehälters 1, wobei die vordere Seitenwand 5 zu einem Flansch 11 verbreitert ist, der seitlich über die Unterseite des Transportbehälters 1 hinausragt. Ein solcher Flansch 11 kann als Kopplungseinrichtung zur vorübergehenden mechanischen Kopplung des Transportbehälters 1 mit einer Seitenwand 30 des Reinraums B (vgl. Fig. 2b) von Greifern oder dergleichen hintergriffen werden, wie nachfolgend ausgeführt. Auch bei dieser Ausführungsform ist auf der vorderen Seitenwand 5 ein flacher, umlaufender Anlageabschnitt 5 vorgesehen, der in Anlage an einer elastischen Dichtung auf der Seitenwand eines Reinraums einen Spalt zwischen der Schleusentür eines Reinraums und der Abdeckung 10 abdichten kann. Bei dieser Ausführungsform ist der untere Rand der Zugriffsöffnung 9 bevorzugt bündig mit dem Boden des Transportbehälters 1, sodass die Haltestruktur unmittelbar auf dem Boden des Transportbehälters 1 verschoben und so in den Reinraum überführt werden kann, wie nachfolgend ausgeführt.

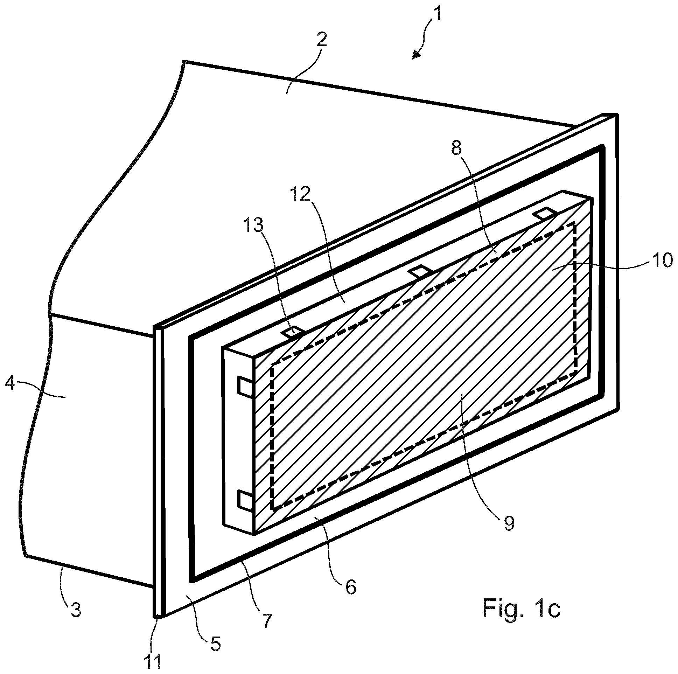

Die Fig. 1c zeigt eine weitere Ausführungsform eines solchen Transportbehälters 1, wobei abweichend zur Fig. 1b innerhalb des rechteckförmigen Bereichs der elastischen Dichtung 7, ein reckteckförmiger, rahmenartiger Vorsprung 12 von der vorderen Seitenwand 5 abragt, auf dem die Abdeckung 10 vorgesehen ist, insbesondere eine Schutzfolie aufgeklebt ist, wie vorstehend ausgeführt. Gemäß der Fig. 1c sind in dem Vorsprung 12 eine Mehrzahl von Ausnehmungen oder Vertiefungen 13 ausgebildet, sodass in diesen Bereichen die Abdeckung 10 von mechanischen Greifern hintergriffen und abgezogen werden kann, wie nachfolgend ausgeführt. Alternativ können derartige Ausnehmungen oder Vertiefungen 13 auch entlang dem Rand des Flansches 11 oder der vorderen Seitenwand 5 des Transportbehälters 1 in entsprechender Weise ausgebildet sein. Auch bei dieser Ausführungsform kann der untere Rand der Zugriffsöffnung 9 mit dem Boden des Transportbehälters 1 bündig abschließen.

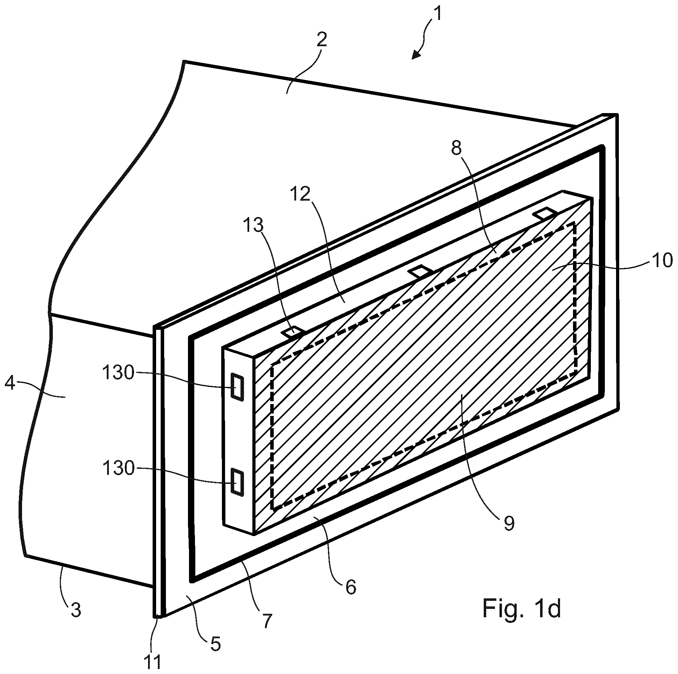

Die Fig. 1d zeigt eine weitere Ausführungsform, bei der zusätzlich in dem Vorsprung 12 eine Mehrzahl von Aussparungen oder Vertiefungen 130 ausgebildet sind, die als Kopplungseinrichtung zur vorübergehenden mechanischen Kopplung des Transportbehälters 1 mit einer Seitenwand 30 des Reinraums B (vgl. Fig. 2b) von Greifern oder dergleichen hintergriffen werden können. Dies ist schematisch in der Fig. 8b gezeigt, wonach auf der Seitenwand 30 des Reinraums seitlich zu der Öffnung 300 des Reinraums Riegel 308 vorgesehen sind, die in Richtung der Doppelpfeile verstellt werden können. In einer geöffneten Stellung dieser Riegel 308 kann der Transportbehälter 1 der Seitenwand 30 angenähert werden, bis eine geeignete Stellung erreicht ist, insbesondere eine unmittelbare Anlage der Abdeckung 10 (vgl. Fig. 1d) an der Schleusentür des Reinraums. In dieser Stellung werden die Riegel 308 dann einwärts verstellt, bis diese in die korrespondierend angeordneten Aussparungen oder Vertiefungen 130 auf dem Vorsprung 12 eingreifen. Die Riegel 308 können zusätzlich auch axial in Richtung der Seitenwand 30 verstellbar sein, um den Transportbehälter 1 an der Seitenwand fest zu klemmen. Auch bei dieser Ausführungsform kann der untere Rand der Zugriffsöffnung 9 mit dem Boden des Transportbehälters 1 bündig abschließen.

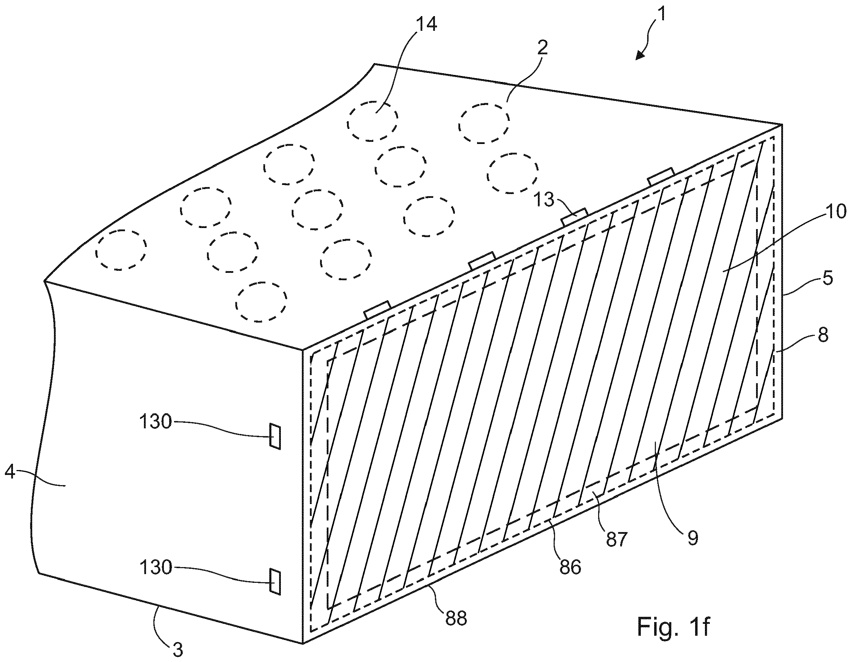

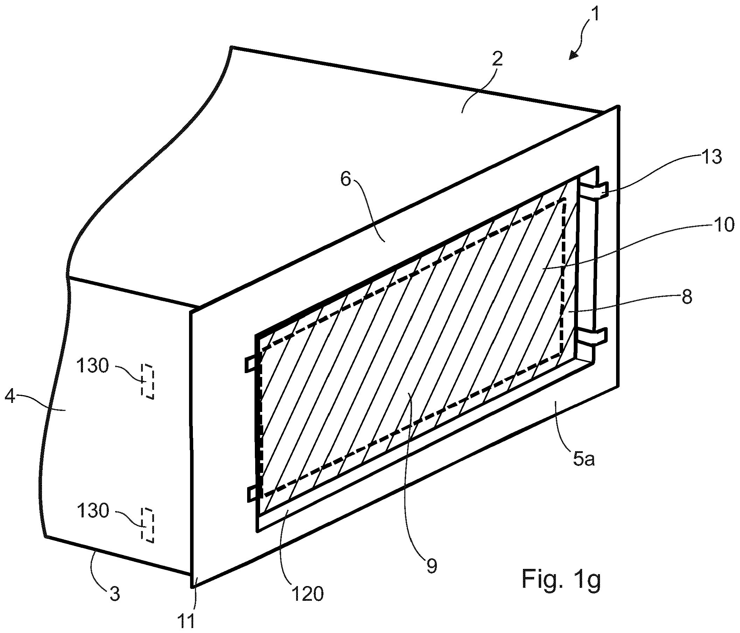

Derartige Aussparungen oder Vertiefungen 130 können auch auf der Seitenwand des Transportbehälters 1 vorgesehen sein, wie in den Figuren 1f und 1g gezeigt. Nach der Fig. 1f bedeckt die Abdeckung 10, insbesondere eine Schutzfolie wie vorstehend beschrieben, die gesamte vordere Seitenwand 5 des Transportbehälters 1, ohne dass der vorgenannte umlaufende Anlageabschnitt notwendig wäre.

Bei der Ausführungsform nach der Fig. le ist zusätzlich auf dem Kleberand eine Randverstärkung 85 zum besseren Entfernen der Abdeckung 10, insbesondere einer Schutzfolie, vorgesehen. Diese Randverstärkung 85 kann als Polymer oder als Rahmen aus einem Kunststoff oder Metallblech ausgebildet sein und mittels eines Klebers aufgeklebt sein, der insbesondere nicht thermisch lösbar zu sein braucht. Das Abziehen der Abdeckung 10 von der vorderen Seitenwand 5 erfolgt in der vorstehend beschriebenen Weise durch Eingriff von verstellbaren Greifern in die Aussparungen oder Vertiefungen 13 und feste Klemmung der Abdeckung 10, insbesondere als Schutzfolie, gegen die Randverstärkung 85, was insbesondere ein versehentliches Aufreißen einer Schutzfolie verhindern kann.

Die Fig. 1f zeigt eine weitere Ausführungsform, bei der sich die Abdeckung 10, insbesondere als Schutzfolie ausgebildet, bis zum Rand der vorderen Seitenwand 5 erstreckt. Dabei ist die Abdeckung 10, insbesondere die Schutzfolie, entlang dem Kleberand 8 auf die vorderen Stirnseiten der oberen und unteren Seitenwand 2, 3 und der Seitenwände 4 aufgeklebt, wobei der Randbereich der Abdeckung 10, wo diese mit dem Kleberand 8 verbunden ist, durch einen linienförmigen Schwächungsbereich 86 in einen äußeren umlaufenden Abschnitt 88 und einen inneren umlaufenden Abschnitt 87 unterteilt ist. Durch Andrücken des äußeren umlaufenden Abschnitts 88 gegen die Seitenwand des Reinraums kann der Bereich um den Schwächungsbereich 86 herum gegen die Umgebung abgedichtet werden, insbesondere, wenn in diesem Bereich ein elastisches Dichtungselement (entweder auf dem äußeren umlaufenden Abschnitt 88 der Abdeckung 10 oder auf der Seitenwand des Reinraums) vorgesehen ist oder die Abdeckung selbst in ausreichendem Maße elastisch ausgebildet ist. Der Schwächungsbereich 86 ist so ausgestaltet, dass die durch Ankoppeln des inneren Bereichs der Abdeckung 10 an die Schleusentür des Reinraums ausgeübte Haltekraft ausreichend ist, sodass beim Öffnen der Schleusentür, wie nachfolgend ausführlicher beschrieben, die Abdeckung 10 entlang dem linienförmigen Schwächungsbereich 86 von der Vorderseite des Transportbehälters 1 abgezogen werden kann. Ein derartiger Schwächungsbereich 86 kann durch Prägen, Ausbildung einer Mehrzahl von mulden-förmigen Vertiefungen durch Laserablation, Laserperforierung oder dergleichen entlang dem Rand der Abdeckung 10 ausgebildet werden. Da die Abdeckung 10 entlang dem inneren umlaufenden Abschnitt 87 nach wie vor über den Kleberand mit den vorderen Stirnseiten der oberen und unteren Seitenwand 2, 3 und der Seitenwände 4 verbunden ist, kann gleichwohl eine sterile Verpackung gewährleistet werden.

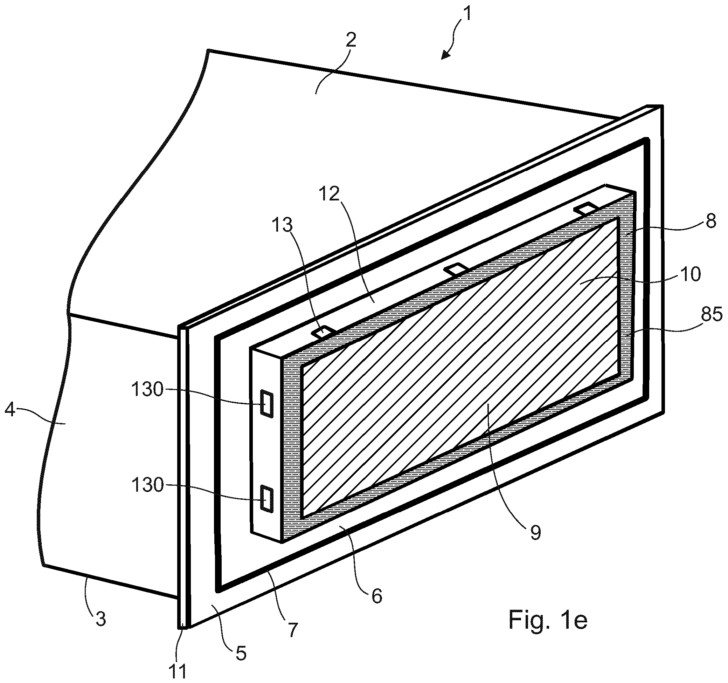

In der Seitenwand 4 sind als Kopplungseinrichtungen wirkende Aussparungen oder Vertiefungen 130 ausgebildet, zur vorübergehenden mechanischen Kopplung des Transportbehälters 1 mit der Seitenwand des Reinraums, wie nachfolgend ausgeführt. Weiter sind in der Oberseite 2 mehrere Aussparungen oder Vertiefungen 13 zum Abziehen der Abdeckung 10 vorgesehen, die sowohl den äußeren umlaufenden Abschnitt 88 als auch den inneren umlaufenden Abschnitt 87 der Abdeckung 10 hintergreifen. Auf dem Kleberand 8 kann eine Randverstärkung vorgesehen sein, wie vorstehend anhand der Fig. 1e ausgeführt. Vorteilhaft an dieser Ausführungsform ist insbesondere, dass sich die Zugriffsöffnung 9 des Transportbehälters 1 bis zur Unterseite 3 hin erstreckt und keine störende Schwelle vorliegt, sodass die Behälter unmittelbar auf dem Boden des Transportbehälters 1 abgestützt und über die Zugriffsöffnung 9 in den Reinraum verschoben werden können.

Zur mechanischen Kopplung einer solchen Schutzfolie mit einer Schleusentür können auf der Außenseite der Schutzfolie grundsätzlich auch mechanisch handhabbare, insbesondere greifbare, Strukturen vorgesehen sein. Beispielsweise können an definierten Stellen auf der Außenseite der Schutzfolie punktförmige Bereiche aus Kunststoff fest mit der Schutzfolie verbunden sein, beispielsweise auflaminiert oder mittels eines Spritzgussverfahrens aufgespritzt, an denen die mechanisch handhabbaren Strukturen befestigt sind, beispielsweise in Gestalt von ringförmigen Gebilden, Haken, Ösen oder Gebilden mit Vorsprüngen und/oder Vertiefungen, die aus Kunststoff gefertigt sind und entweder einstückig mit den punktförmigen Bereichen aus Kunststoff ausgebildet sind oder an diesen befestigt sind, beispielsweise durch Kleben oder thermisches Schweißen.

Die Fig. 1g zeigt eine weitere Ausführungsform, bei der die Abdeckung 10 in Bezug zur vorderen Seitenwand 5a mittels einer rahmenartig ausgebildeten Stufe 120 nach hinten zurückversetzt ist. Zur vorübergehenden mechanischen Kopplung des Transportbehälters 1 mit der Seitenwand des Reinraums kann der seitlich überstehende Flansch 11 in einen Klemmkanal 304 (vgl. Fig. 8a) von oben eingeschoben werden, der an der Seitenwand 30 des Reinraums seitlich zur Öffnung 300 des Reinraums ausgebildet ist. Der Klemmkanal 304 ist gemäß der Fig. 8a dabei von einer vorderen Begrenzungswand 302 und einer Seitenwand 303 einer sich vertikal erstreckenden Klemmschiene 301 ausgebildet, mit einer Weite, die auf die Tiefe des Flansches 11 am vorderen Ende des Transportbehälters 1 abgestimmt ist und eine ausreichende Klemmung des Transportbehälters 1 in dem Klemmkanal 304 ermöglicht. Zur Abstützung des Transportbehälters 1 bei der Übergabe der Behälter in den Reinraum ist unterhalb der Öffnung 300 des Reinraums eine Ablagefläche 305 vorgesehen, die auch als Anschlag wirkt, um die Klemmstellung festzulegen. Weil die Abdeckung 10 in Bezug zur vorderen Seitenwand 5a nach hinten zurückversetzt ist, wird diese beim Einschieben des Flansches 11 in den Klemmkanal 304 nicht unbeabsichtigt abgeschert.

Wie dem Fachmann ohne weiteres ersichtlich sein wird, kann der vorgenannte Klemmkanal in entsprechender Weise auch horizontal verlaufend ausgebildet sein.

Wie man der Fig. 1g entnehmen kann, sind zur optionalen oder alternativen Kopplung in der Seitenwand 4 des Transportbehälters 1 mehrere Vertiefungen oder Aussparungen 130 ausgebildet, wie vorstehend beschrieben.

Zum Abziehen der Abdeckung 10, insbesondere Schutzfolie, sind in der Stufe 120 L-förmige Aussparungen 13 ausgebildet, deren längerer Schenkel sich in Längsrichtung des Transportbehälters 1 erstreckt, und deren Boden die Abdeckung 10 hintergreift, sodass verstellbare Greifer, die auf der Außenseite der Schleusentür des Reinraums vorgesehen sind, die Abdeckung 10 hintergreifen und diese geeignet klemmen können, wie vorstehend beschrieben.

Wie dem Fachmann ohne weiteres ersichtlich sein wird, kann bei sämtlichen vorstehenden Ausführungsformen des Transportbehälters der Kleberand von einem linienförmigen Schwächungsbereich in einen äußeren umlaufenden Abschnitt und einen inneren umlaufenden Abschnitt unterteilt sein, um ein Abziehen der Abdeckung von der Vorderseite des Transportbehälters durch Koppeln der Abdeckung mit der Schleusentür weiter zu unterstützen, wobei der äußeren umlaufende Abschnitt beim Koppeln der Abdeckung mit der Schleusentür auch weiterhin zwischen dem Transportbehälter und der Seitenwand des Reinraums geklemmt sein kann.

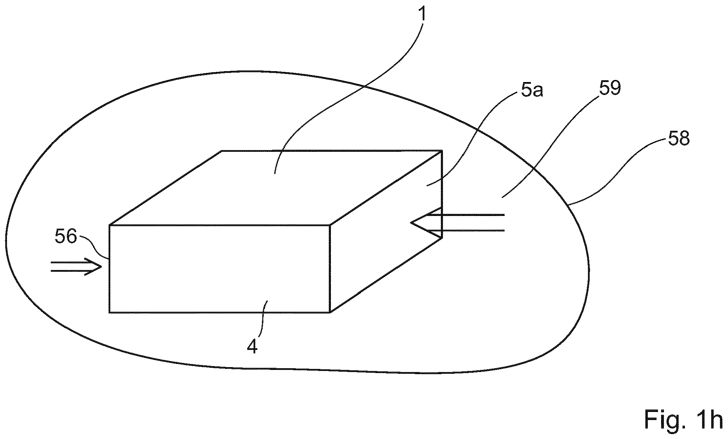

Zum sterilen Transport einer Mehrzahl von Behältern für medizinische, pharmazeutische oder kosmetische Zwecke und/oder von Verschlusselementen hierfür ist ein solcher Transportbehälter 1 gemäß der Fig. 1h in einen Verpackungsbeutel 58 steril eingeschweißt. Um einen keimfreien Transport und eine keimfreie Entnahme der Behälter aus dem Transportbehälter 1 zu ermöglichen, wird nach dem Einbringen der Behälter in den Transportbehälter 1 die Außenseite des Transportbehälters 1 sterilisiert, insbesondere vor einem Zuschweißen des Verpackungsbeutels 58, beispielsweise durch Bestrahlung, beispielsweise mit Elektronenstrahlen, Einwirken von Wärme, beispielsweise Dampf (unter kontrollierten Bedingungen), neuere Verfahren, wie beispielsweise oxidative Niedertemperatur-Sterilisation, oder mittels chemischer Mittel, insbesondere durch Beaufschlagung mit einem sterilisierenden Gas (z.B. ETO) oder heißem Dampf. Sofern noch nicht geschehen, kann auch die Innenseite des Verpackungsbeutels 58 vor dem Verschließen des Verpackungsbeutels 58 geeignet sterilisiert werden.

Wie durch die Pfeile in der Fig. 1h angedeutet, kann auch der Innenraum des Transportbehälters 1, ggf. gemeinsam mit den darin aufgenommenen Behältern und/oder Verschlusselementen, nach dem Verschließen der vorderen bzw. hinteren Seitenwand durch eine gasdurchlässige Schutzfolie, wie vorstehend beschrieben, durch Einströmen von Dampf, eines Gases oder eines chemischen Mittels durch die Schutzfolie hindurch sterilisiert werden.

Transportbehälter (tub) der vorgenannten Art sind bevorzugt aus einem Kunststoff gefertigt, der ausreichend stabil gegen Verformung und Verwindung ist, um die in seinem Innenraum aufgenommenen Behälter und/oder Verschlusselemente ausreichend mechanisch zu schützen. Ein solcher Transportbehälter kann durch Spritzgießen oder auch durch Thermoformen eines Kunststoffs hergestellt sein, wobei eine quadratische Grundfläche bevorzugt wird und die Höhe auf die Höhe der aufzunehmenden Behälter und/oder Verschlusselemente abgestimmt ist. Dabei werden Höhen von 3, 4, 5, 6 oder 6 ¾ Zoll bevorzugt.

Zur Aufnahme von Pharmabehältern wird bevorzugt, wenn nur eine Lage von Pharmabehältern in dem Transportbehälter aufgenommen ist. Zweckmäßig ist dann in dem Transportbehälter nur eine Haltestruktur mit einer Mehrzahl von daran gehaltenen Pharmabehältern und/oder Verschlusselementen aufgenommen. Grundsätzlich denkbar ist jedoch, dass auch mehrere Haltestrukturen in der Längsrichtung und/oder Querrichtung des Transportbehälters angeordnet sind, beispielsweise 3x3 Haltestrukturen oder 5x5 Haltestrukturen.

In dem Transportbehälter können grundsätzlich auch mehrere Haltestrukturen vertikal übereinander gestapelt aufgenommen sein, was insbesondere zur Lagerung und zum Transport von Verschlusselementen bevorzugt ist, für die weniger strenge Auflagen gelten als für Pharmabehälter. Die Haltestrukturen können dann in einem Regal auf Ablagen abgestützt sein oder auch unmittelbar, oder mit dazwischen befindlichen Zwischenlagen, übereinander gestapelt angeordnet sein.

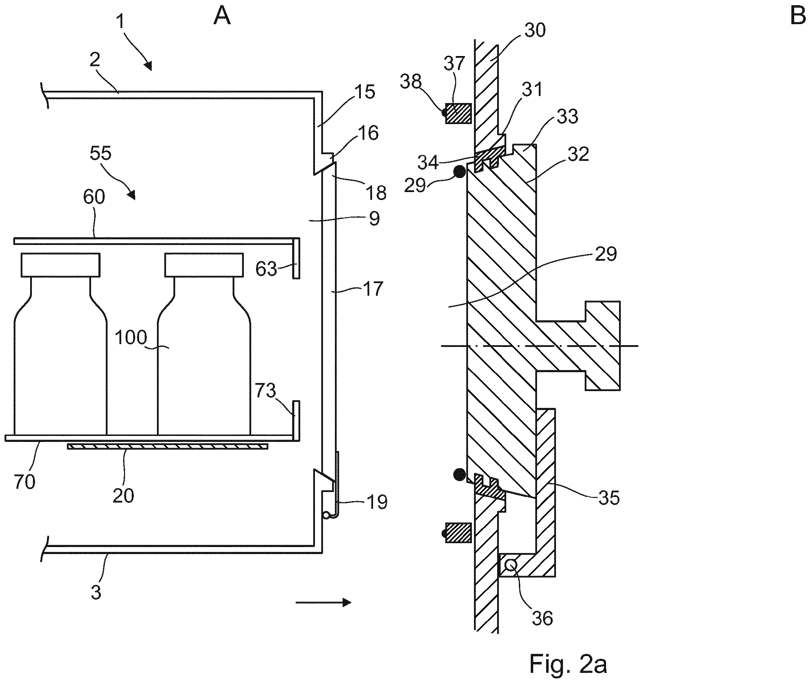

Nachfolgend wird anhand der Figuren 2a bis 2d ein Verfahren zum Überführen einer Mehrzahl von Behältern aus einem Transport- und Verpackungsbehälter in einen Reinraum B gemäß einer ersten Ausführungsform der vorliegenden Erfindung beschrieben. Dabei wird angenommen, dass der Transportbehälter 1 bereits aus dem Verpackungsbeutel 58 entnommen ist, was beispielsweise in einem weiteren Reinraum A mit einer höheren Partikelkonzentration als in dem Reinraum B erfolgen kann, in dem die Behälter später weiterverarbeitet werden sollen. Ein Verfahren gemäß der vorliegenden Erfindung kann in entsprechender Weise auch zum Überführen einer Mehrzahl von Verschlusselementen für Pharmabehälter oder einer Mehrzahl von Pharmabehältern gemeinsam mit solchen Verschlusselementen verwendet werden.

Die Haltestruktur für die Verschlusselemente und die Haltestruktur für die Pharmabehälter können dabei in dem Transport- und Verpackungsbehälter gestapelt übereinander angeordnet sein, beispielsweise in der Art, wie in der WO 2015/023924 A2 offenbart. Grundsätzlich können in dem Transport- und Verpackungsbehälter auch ausschließlich Verschlusselemente für Pharmabehälter aufgenommen sein, die in einer Haltestruktur in einer vorbestimmten regelmäßigen Anordnung gehalten sind, insbesondere in einer Matrix-Anordnung. Bei den Verschlusselementen kann es sich insbesondere um Stopfen, Kolbenpfropfen oder Verschlusskappen handeln, die in dem Reinraum weiterverwendet werden sollen, etwa zum Verschließen der Pharmabehälter nach einem Befüllen mit einem Wirkstoff oder einer Flüssigkeit.

Die Fig. 2a zeigt die Situation nach dem Einbringen des Transportbehälters 1 mit den darin aufgenommenen Behältern 100 in den Reinraum A, bzw. nach dem Öffnen des Verpackungsbeutels in dem Reinraum A, der die höhere Partikelkonzentration aufweist. Bei dem gezeigten Beispiel ist die vordere Seitenwand 15 des Transportbehälters 1 mit Hilfe eines gasundurchlässigen Deckels 17 verschlossen, dessen kegelstumpfartig abgeschrägter Rand 18 in den korrespondierend ausgebildeten abgeschrägten Rand 16 der vorderen Seitenwand 15 des Transportbehälters 1 eingesteckt ist. Das untere Ende des Deckels 17 ist mit einer elastischen Lasche 19 aus einem elastisch dehnbaren Kunststoff- oder Gummimaterial oder mit einem geeigneten längsverstellbaren Beschlag mit der vorderen Seitenwand 15 verbunden. In dem gasundurchlässigen Deckel 17 kann eine Öffnung ausgebildet sein, die mit einer gasdurchlässigen Schutzfolie steril verschlossen ist. Diese Öffnung kann dabei gemäß weiteren Ausführungsformen auch als Zugriffsöffnung zum Überführen der Behälter und/oder Verschlusselemente dienen.

Der rechte Bildteil der Fig. 2a zeigt den Bereich der Schleusentür 32 eines Reinraums B mit einer geringeren Partikelkonzentration als in dem Reinraum A. Die Schleusentür 32 verschließt eine in der Seitenwand 30 des Reinraums B ausgebildete Öffnung und kann durch Schwenken des Schwenkarms 35 mit der mit diesem verbundenen Schleusentür 32 um die Schwenkachse 36 geöffnet werden. Die Schleusentür 32 ist kegelstumpfförmig ausgebildet und liegt über die elastische Dichtung 34 an dem korrespondierend ausgebildeten abgeschrägten Rand 31 der Seitenwand 30 des Reinraums B an. Um die Schleusentür herum umlaufend ist ein rahmenförmiger Vorsprung 37 ausgebildet, auf dessen vorderer Seitenwand eine elastische Dichtung 38 vorgesehen ist. Gemäß der Fig. 2a kann optional auf der Außenseite der Schleusentür 32 eine weitere, umlaufend ausgebildete elastische Dichtung 39 vorgesehen sein. Die Schleusentür 32 ist hier nur als ein Beispiel für eine Materialschleuse zum Überführen der Behälter in den Reinraum B gedacht und kann selbstverständlich durch andere geeignete Verschlusseinrichtungen ersetzt werden, wie diese auf dem Gebiet der Rein- oder Reinstraumtechnologie gebräuchlich sind.

Gemäß der Fig. 2a wird die vordere Seitenwand 15 des Transportbehälters 1 in unmittelbarer Nähe zur Schleusentür 32 angeordnet, sodass die elastische Dichtung 38 auf dem umlaufenden Vorsprung 37 der flach ausgebildeten vorderen Seitenwand 15 des Transportbehälters 1 gegenüberliegt, welche den Deckel 17 umgibt. In dieser Stellung ist ein vergleichsweise schmaler Spalt 29 zwischen der vorderen Seitenwand 15 bzw. dem Deckel 17 des Transportbehälters 1 und der Seitenwand 30 bzw. der Schleusentür 32 des Reinraums B ausgebildet.

Grundsätzlich kann es gemäß der vorliegenden Erfindung für das Überführen der Behälter 100 aus dem Transportbehälter 1 in den Reinraum B ausreichend sein, bei genügend schmalem Spalt 29 den Deckel 17 von der vorderen Seitenwand 15 abzuziehen, beispielsweise durch seitliches Abziehen, gleichzeitig die Schleusentür zu öffnen, die Behälter 100 bzw. die Haltestruktur 55, in der die Behälter 100 gehalten bzw. aufgenommen sind, rasch in den Innenraum des Reinraums B zu überführen, und anschließend die Schleusentür 32 des Reinraums B rasch wieder zu verschließen. Wird diese Folge von Schritten rasch genug ausgeführt und ist der Spalt 29 ausreichend schmal, so kann erreicht werden, dass die Haltestruktur 55 bzw. die Behälter 100 und der Innenraum des Reinraums B beim Öffnen des Deckels 17 bzw. der Schleusentür 32 in praktisch vernachlässigbarem Umfang kontaminiert werden.

Gemäß einer bevorzugten Ausführungsform wird zum Überführen der Behälter 100 aus dem Transportbehälter 1 in den Reinraum B jedoch so vorgegangen, wie nachfolgend anhand der Figuren 2b bis 2d beschrieben. Gemäß der Fig. 2b wird die vordere Seitenwand 15 des Transportbehälters 1 zunächst in unmittelbare Anlage zu der elastischen Dichtung 38 gebracht, sodass der Spalt 29 zwischen dem Deckel 17 und der Schleusentür 32 durch den umlaufenden Vorsprung 37 und die darauf vorgesehene elastische Dichtung 38 gegen die Umgebung abgedichtet ist. Aufgrund der Konfiguration der Schleusentür 32 und des Deckels 17 ist in dieser Stellung gleichzeitig gewährleistet, dass der Deckel 17 unmittelbar an der optional vorgesehenen elastischen umlaufenden Dichtung 39 auf der Außenseite der Schleusentür 32 anliegt, sodass der Spalt 29 zwischen dem Deckel 17 und der Schleusentür 32 durch die umlaufende elastische Dichtung 39 abgedichtet ist.

In dieser Stellung wird der Deckel 17 vorübergehend mechanisch mit der Schleusentür 32 gekoppelt, was in der Fig. 2b aus Vereinfachungsgründen nicht dargestellt ist, jedoch durch Verrastung, mittels Greifern oder eines elektrisch, magnetisch oder pneumatisch betätigten Halte- oder Greifmittels realisiert werden kann, wie anhand der weiteren Ausführungsbeispiele nachfolgend beschrieben.

Nach dem mechanischen Koppeln des Deckels 17 mit der Schleusentür 32 wird diese durch Verschwenken des Schwenkarms 35 mit der daran befestigten Schleusentür 32 um die Schwenkachse 36 in den Innenraum des Reinraums B hinein geöffnet, wodurch der Innenraum des Transportbehälters 1 über die Zugriffsöffnung 9 mit dem Innenraum des Reinraums B in Verbindung steht. Aufgrund der Kopplung des Deckels 17 mit der Schleusentür 32 wird der Deckel 17 beim Öffnen der Schleusentür 32 automatisch von der vorderen Seitenwand 15 des Transportbehälters 1 gelöst, wodurch die Zugriffsöffnung des Transportbehälters 1 freigegeben wird.

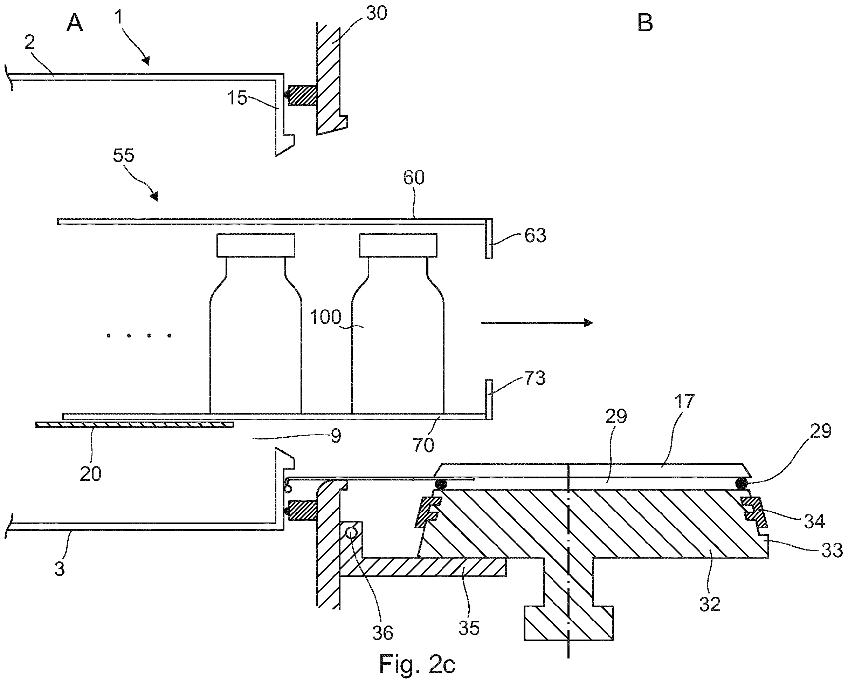

In der geöffneten Stellung der Schleusentür 32 und des Deckels 17 gemäß der Fig. 2b kann die Haltestruktur 55 mit den darin aufgenommenen Behältern 100 aus dem Transportbehälter 1 in den Innenraum des Reinraums B überführt werden. Wie in der Fig. 2c dargestellt, kann zu diesem Zweck das Unterteil 70 der Haltestruktur 55 auf der Führungsschiene 20, beispielsweise auf einem Vorsprung auf der Innenseite der Seitenwand des Transportbehälters 1, verschoben werden, wie durch den Pfeil in der Fig. 2c angedeutet. Weil der Innenraum des Transportbehälters 1 und die Behälter 100 zuvor beim Einbringen der Behälter 100 in den Transportbehälter 1 keimfrei gemacht wurden, beispielsweise durch Sterilisieren durch Einströmen eines Gases oder Dampfes, durch Bestrahlung oder dergleichen, können durch das Öffnen der Schleusentür 32 und das Überführen der Behälter 100 in den Innenraum des Reinraums B keine zusätzlichen Keime oder Verunreinigungen in den Innenraum des Reinraums B eingebracht werden. Weil der Spalt 29 zwischen der Außenseite des Deckels 10 und der Außenseite der Schleusentür 32 vor dem Öffnen der Schleusentür 32 durch die umlaufende Dichtung 39 abgedichtet wurde, können in der geöffneten Stellung der Schleusentür 32 gemäß der Fig. 2c Keime oder Verunreinigungen weder von der Außenseite des Deckels 10 noch von der Außenseite der Schleusentür 32 in den Innenraum des Reinraums B gelangen oder die Behälter 100 bei deren Überführung in den Reinraum B verunreinigen.

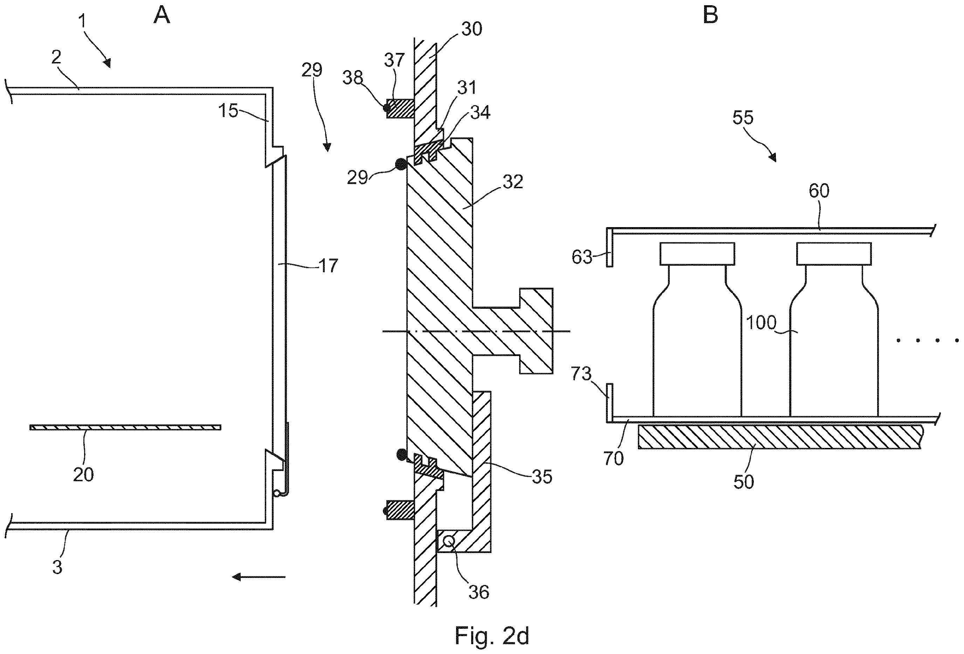

Nach Überführen der Haltestruktur 55 mit den darin aufgenommenen Behältern 100 aus dem Transportbehälter 1 in den Innenraum des Reinraums B wird die Schleusentür 32 erneut geschlossen, wie in der Fig. 2d dargestellt. Anschließend kann die vorübergehende mechanische Kopplung der Schleusentür 32 mit dem Deckel 17 wieder gelöst werden, sodass anschließend der Transportbehälter 1 von der Seitenwand 30 des Reinraums B wieder abgezogen werden kann, wie in der Fig. 2d gezeigt, und die Schleusentür 32 somit für ein Überführen von weiteren Behältern aus einem anderen Transportbehälter in den Reinraum B bereitsteht.

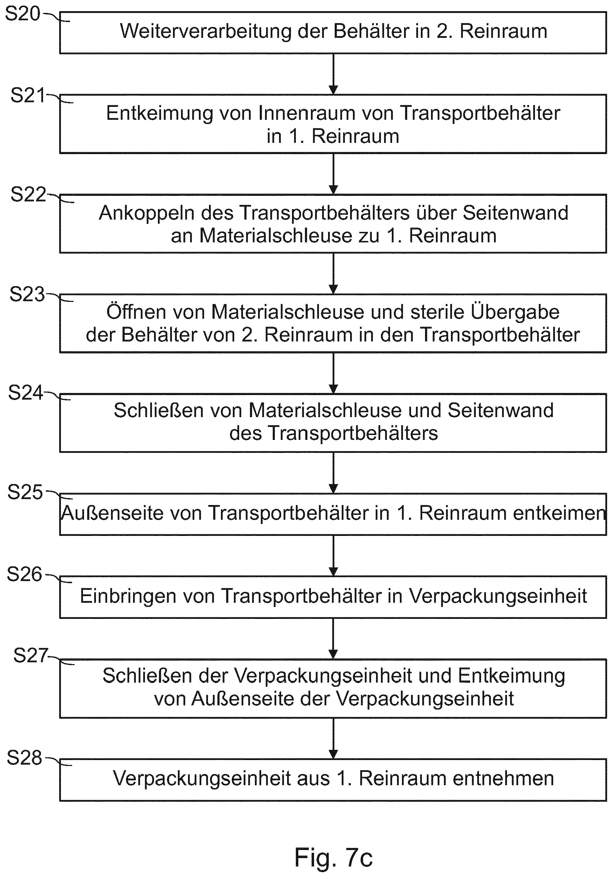

Gemäß der Fig. 2d kann der Transportbehälter 1 erneut mit dem Deckel 17 vorübergehend verschlossen werden und anschließend der Deckel 17 wieder abgenommen werden, beispielsweise um die Behälter 100 nach deren Weiterverarbeitung in dem Reinraum B mit der niedrigeren Partikelkonzentration erneut aufzunehmen, wie nachfolgend anhand der Fig. 7c beschrieben. Grundsätzlich kann der Transportbehälter 1 jedoch auch aus dem Reinraum A entnommen und anschließend entsorgt oder für einen erneuten Transport keimfrei gemacht werden.

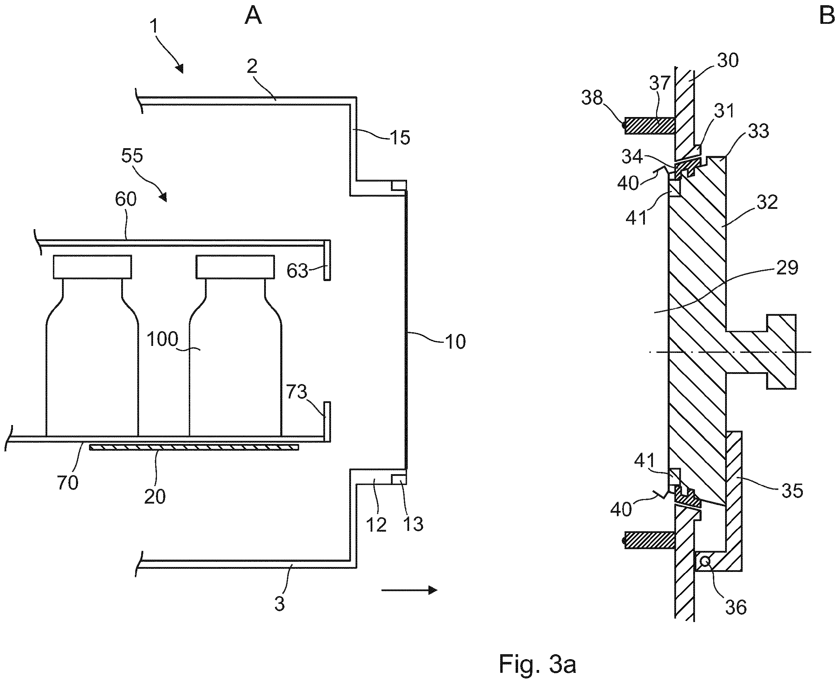

Während dem Ausführungsbeispiel gemäß den Figuren 2a bis 2d zugrunde lag, dass der Transportbehälter 1 mittels einer Kraft, beispielsweise der Kraft einer Förder- oder Verstelleinrichtung, permanent gegen die Seitenwand 30 des Reinraums B gedrückt wurde, wird nachfolgend anhand der Figuren 3a bis 3e ein weiteres Ausführungsbeispiel beschrieben, bei dem der Transportbehälter 1 zum Überführen der Haltestruktur 55 mit den darin aufgenommenen Behältern 100 aus dem Transportbehälter 1 in den Innenraum des Reinraums B vorübergehend an der Seitenwand 30 des Reinraums B fixiert oder verrastet wird.

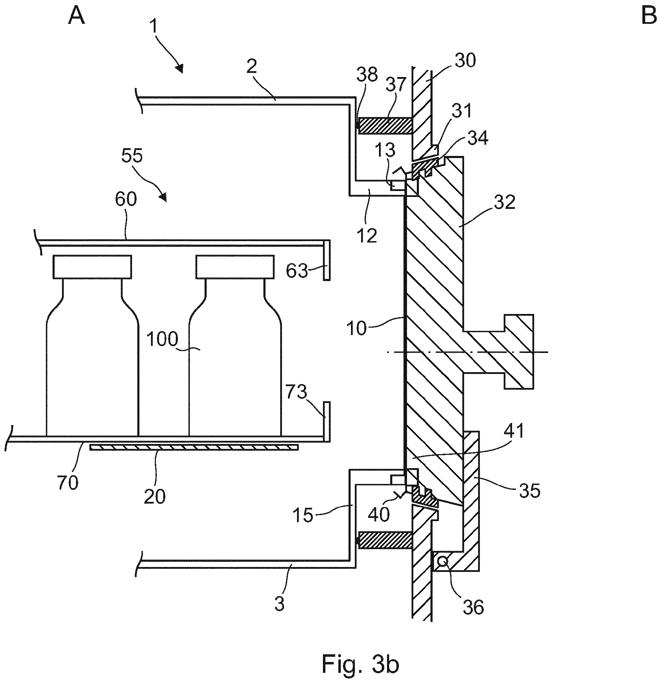

Dem Ausführungsbeispiel nach den Figuren 3a bis 3e liegt nachfolgend die Verwendung eines Transportbehälters gemäß einer der Figuren 1c bis 1e zugrunde, bei dem ein reckteckförmiger, rahmenartiger Vorsprung 12 auf der vorderen Seitenwand 5 vorgesehen ist, auf dem eine gasdurchlässige Schutzfolie 10 vorgesehen ist. Grundsätzlich können jedoch auch anders gestaltete Transportbehälter in entsprechender Weise verwendet werden. Wie man den Figuren 3a und 3b entnehmen kann, sind auf der Außenseite der Schleusentür 32 verstellbare Haltearme 40 zur vorübergehenden Fixierung der Schutzfolie 10 bzw. Abdeckung an der Außenseite der Schleusentür 32 vorgesehen. Gemäß den Figuren 3a und 3b befinden sich diese Haltearme 40 zunächst in einer geöffneten Stellung, in der diese noch nicht mit den Ausnehmungen 13 in dem rahmenartigen Vorsprung 12 des Transportbehälters 1 zusammenwirken.

Ausgehend von der Stellung gemäß der Fig. 3a wird der Transportbehälter 1 bei geöffneter Stellung der Haltearme 40 in die Nähe der Seitenwand 30 gebracht, solange, bis die Stellung gemäß der Fig. 3b erreicht ist, in der die vordere Seitenwand 15 des Transportbehälters an dem Dichtungselement 38 auf dem umlaufenden Vorsprung 37 anliegt und die Schutzfolie 10 sich in unmittelbarer Nähe zur Schleusentür 32 befindet oder an dieser unmittelbar anliegt. In dieser Stellung wird der Transportbehälter 1 mit der Seitenwand 30 des Reinraums B vorübergehend gekoppelt, beispielsweise verrastet, um diesen an der Seitenwand 30 vorübergehend zu fixieren. Zu diesem Zweck können beispielsweise die Riegel 308 gemäß der Fig. 8b einwärts verstellt werden, um in zugeordnete Vertiefungen oder Aussparungen 130 auf dem rahmenartigen Vorsprung 12 (vgl. Fig. 1d oder 1e) oder auf der Seitenwand 4 des Transportbehälters 1 (vgl. Fig. 1f) einzugreifen. Die vorübergehende Kopplung des Transportbehälters 1 mit der Seitenwand 30 kann auch mittels beliebiger anderer Formschlusselemente, pneumatisch, elektrisch oder magnetisch erfolgen.

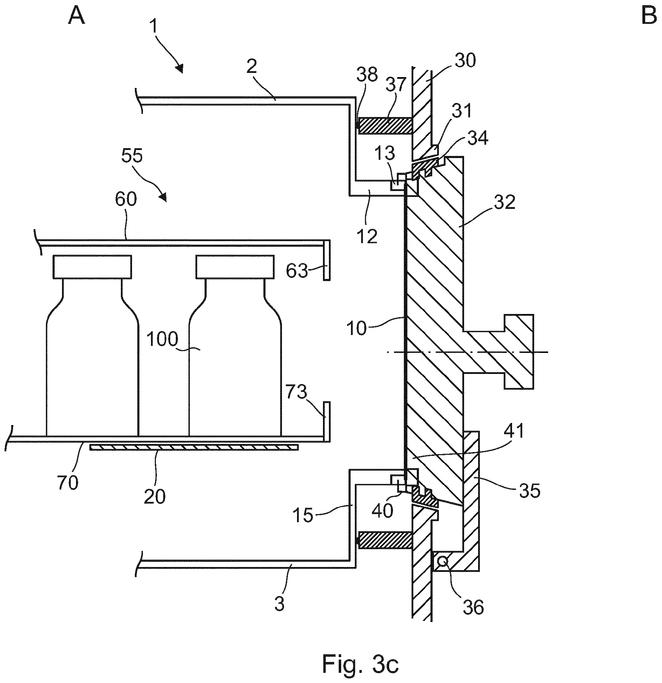

In einem nächsten Schritt werden gemäß der Fig. 3c die Haltearme 40 einwärts verstellt, wie durch die Pfeile in der Fig. 3c angedeutet, bis diese in die Ausnehmungen 13 in dem rahmenartigen Vorsprung 12 des Transportbehälters 1 eingreifen und so die Schutzfolie 10 bzw. Abdeckung hintergreifen, um die Schutzfolie 10 bzw. Abdeckung vorübergehend an der Außenseite der Schleusentür 32 zu fixieren. Die Schutzfolie 10 bzw. Abdeckung kann dabei insbesondere vollflächig gegen die Außenseite der Schleusentür 32 gedrückt oder gezogen werden, oder zwischen der Schutzfolie 10 bzw. Abdeckung und der Außenseite der Schleusentür 32 kann eine weitere elastische Dichtung ähnlich zu der Dichtung 39 gemäß der Fig. 2a vorgesehen sein, um einen Spalt zwischen der Schutzfolie 10 bzw. Abdeckung und der Außenseite der Schleusentür 32 abzudichten. In dieser Stellung verbleibt nur noch ein Spalt 29 zwischen der vorderen Seitenwand 15 des Transportbehälters und der Außenseite der Schleusentür 32 bzw. der Seitenwand 30, der mittels des umlaufenden Vorsprungs 37 und der auf diesem vorgesehen Dichtung 38 gegen die Umgebung abgedichtet ist, um ein Eindringen von Keimen in diesen Bereich zu verhindern.

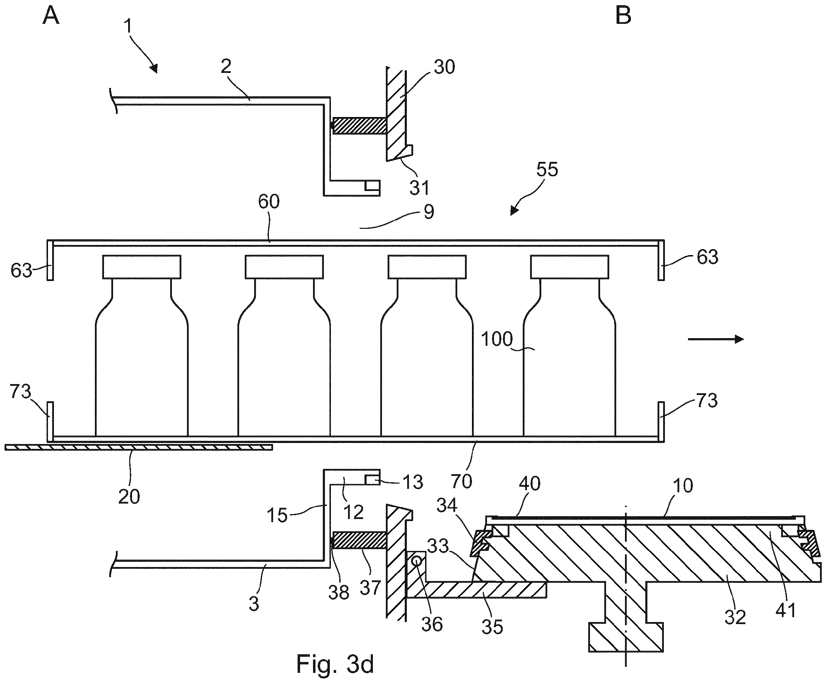

In dieser Stellung wird in einem nächsten Schritt, wie in der Fig. 3d abgebildet, die Schleusentür 32 geöffnet, sodass dann der Innenraum des Transportbehälters 1 über die Zugriffsöffnung 9 mit dem Innenraum des Reinraums B in Verbindung steht. In dieser Stellung kann dann die Haltestruktur 55 durch Verschieben entlang der Führungsschiene 20 in den Reinraum B überführt werden. Weil der Innenraum des Transportbehälters 1 beim Verpacken der Behälter 100 sterilisiert wurde, können in dieser Stellung keine weiteren Keime und Verunreinigungen in den Reinraum B eingebracht werden.

Bei dieser Ausführungsform kann durch das Öffnen der Schleusentür 32 die Schutzfolie 10 bzw. Abdeckung von dem rahmenartigen Vorsprung 12 des Transportbehälters 1 abgezogen werden, wenn die Halte- bzw. Klemmkraft der Haltearme 40 auf der Außenseite der Schleusentür 32 hierfür ausreichend ist.

Wenn die Schutzfolie 10 bzw. Abdeckung auf den rahmenartigen Vorsprung 12 mittels eines Klebers aufgeklebt ist, kann dieses Ablösen weiter durch Einwirken von Wärme und das dadurch bewirkte Erweichen des verwendeten Klebers unterstützt werden. Zu diesem Zweck kann gemäß der Fig. 3c auf der Außenseite der Schleusentür 32 an einer Position, die der Position des Kleberands 8 auf dem rahmenartigen Vorsprung 12 entspricht (vgl. Fig. 1a), eine Heizeinrichtung 41 vorgesehen sein, beispielsweise eine elektrische Heizeinrichtung, die bevorzugt umlaufend ausgebildet ist, in Entsprechung zu dem gegenüberliegenden Kleberand 8 auf der vorderen Seitenwand 15 des Transportbehälters 1. Nach Aktivieren der Heizeinrichtung 41 wird der Kleberand 8 geeignet erweicht, sodass dann die vorübergehend an der Schleusentür 32 fixierte Schutzfolie 10 bzw. Abdeckung beim Öffnen der Schleusentür 32 leichter von dem rahmenartigen Vorsprung 12 abgezogen werden.

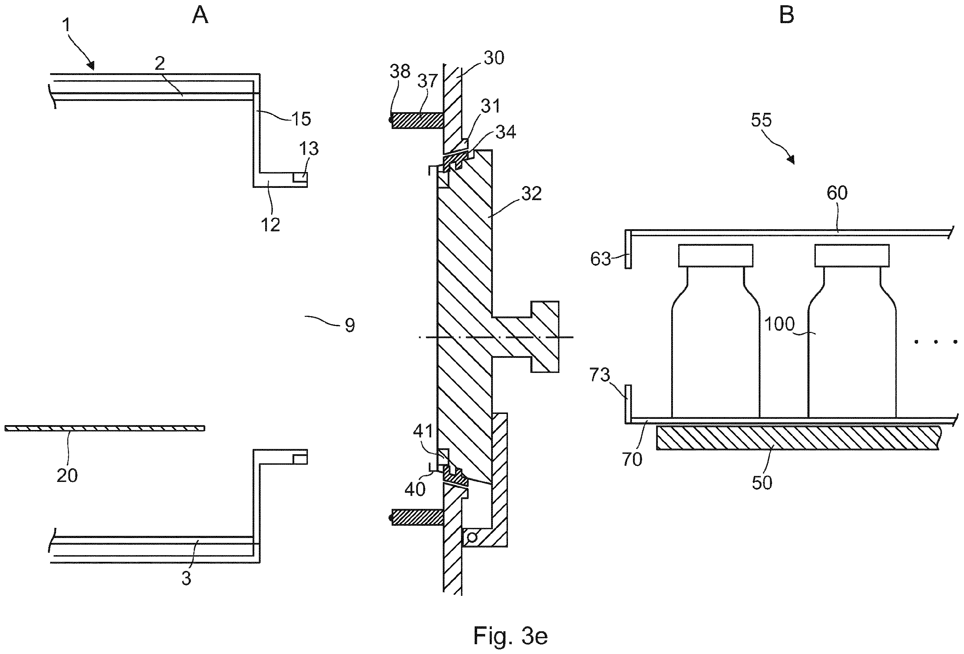

Nachdem die Haltestruktur 55 auf die Führungsschiene 50 in dem Reinraum B geschoben wurde, wie in der Fig. 3e dargestellt, wird anschließend die Schleusentür 32 wieder geschlossen. Die Behälter 100 können anschließend in dem Reinraum B aus der Haltestruktur 55 entnommen werden, wie nachfolgend ausführlicher beschrieben, und stehen dann für eine Weiterverarbeitung in dem Reinraum B zur Verfügung, beispielsweise für ein Befüllen der Behälter 100 unter Reinraum- oder Reinstraumbedingungen.

Weil die Außenseite der Schutzfolie 10 vor dem Öffnen der Schleusentür 32 in Anlage zur Außenseite der Schleusentür 32 gebracht wurde, können in der geöffneten Stellung der Schleusentür 32 gemäß der Fig. 3d Keime oder Verunreinigungen weder von der Außenseite der Schutzfolie 10 bzw. Abdeckung noch von der Außenseite der Schleusentür 32 in den Innenraum des Reinraums B gelangen oder die Behälter 100 bei deren Überführung in den Reinraum B verunreinigen. Dieser Effekt wird noch weiter begünstigt, wenn auf der Außenseite der Schleusentür 32 eine umlaufende Dichtung vorgesehen ist, wie diese bei der Ausführungsform gemäß den Figuren 2a bis 2d vorgesehen ist (Bezugszeichen 39), und wenn die Schutzfolie 10 bzw. Abdeckung vor dem Öffnen der Schleusentür 32 in Anlage mit dieser gebracht wird, um den Spalt 29 (vgl. Fig. 2b) zwischen der Außenseite der Schutzfolie 10 bzw. Abdeckung und der Außenseite der Schleusentür 32 abzudichten.

Nach Schließen der Schleusentür 32, wie in der Fig. 3e dargestellt, wird der Transportbehälter 1 abschließend wieder von der Seitenwand 30 des Reinraums B gelöst und in dem Raum A zurück verstellt. Nach dem Lösen des Transportbehälters 1 in dem Raum A kann die Zugriffsöffnung 9 unverschlossen bleiben, wie in der Fig. 3e gezeigt, oder alternativ erneut verschlossen werden, beispielsweise vorübergehend mit einem Deckel (nicht gezeigt), sodass keine weiteren Verunreinigungen in das Innere des Transportbehälters 1 gelangen können.

Wie dem Fachmann ohne weiteres erkennbar sein wird, können die Behälter 100 durch Umkehr der Reihenfolge der Schritte gemäß den Figuren 3e bis 3a in entsprechender Weise aus dem Reinraum B zurück in den gleichen oder einen anderen Transportbehälter 1 übergeben werden, was nachfolgend ausführlicher anhand der Fig. 7c erläutert werden wird.

Die vorstehend beschriebene vorübergehende Verrastung des Transportbehälters 1 an der Seitenwand 30 des Reinraums B kann in verschiedenster Weise erfolgen. Beispielsweise können an der Seitendwand 30 des Reinraums B verstellbare Verrastungselemente, beispielsweise schwenkbeweglich gelagerte Arme mit hakenartigen vorderen Enden, vorgesehen sein, die in einer Ausgangsstellung ein ungehindertes Annähern des Transportbehälters zulassen und anschließend in eine Verrastungsstellung überführt werden können, beispielsweise zu dem Transportbehälter hin geschwenkt werden, um dort in Ausnehmungen oder Vertiefungen formschlüssig einzugreifen. In der Verrastungsstellung ist der Transportbehälter 1 an der Seitenwand 30 des Reinraums B vorübergehend verrastet. Die Verrastung kann auch durch einen Bajonett-Mechanismus oder dergleichen erfolgen, beispielsweise indem auf der Seitenwand 30 des Reinraums B ein Drehring vorgesehen ist, der bei Verdrehung in korrespondierende Strukturen auf der vorderen Seitenwand 15 des Transportbehälters 1 eingreift, oder indem der Transportbehälter 1 nach Annähern an die Seitenwand 30 seitlich verschoben wird, was zum formschlüssigen Eingriff von Verrastungselementen, beispielsweise von Schrägflächen, auf der vorderen Seitenwand 15 des Transportbehälters 1 und auf der Seitenwand 30 des Reinraums B führt.

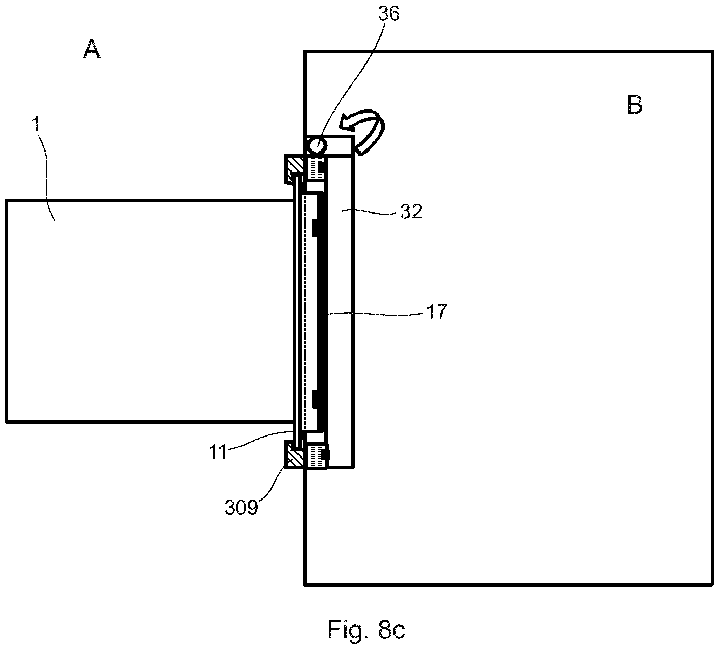

Die Fig. 8c zeigt eine weitere Vorgehensweise zur vorübergehenden Fixierung des Transportbehälters 1 an der Seitenwand 30 eines Reinraums B. Gemäß der Fig. 8c sind auf der Außenseite der Seitenwand 30 entlang des unteren Rands der Schleusentür 32 im Querschnitt L-förmige Klemmleisten 309 vorgesehen, die von der Seitenwand 30 weggeschwenkt und zu dieser hin geschwenkt werden können. In der geschlossenen Stellung gemäß der Fig. 8c, in der die Klemmleisten 309 zu der Seitenwand 30 hin geschwenkt sind, wird der über den Boden des Transportbehälters 1 hinausragende Flansch 11 von den Klemmleisten 309 hintergriffen und der Transportbehälter so zu der Seitenwand 30 hingezogen. Um die Zugriffsöffnung des Transportbehälters bzw. die Schleusentür 32 herum kann eine umlaufend ausgebildete elastische Dichtung vorgesehen sein, wie vorstehend beschrieben. Zum Lösen des Transportbehälters 1 von der Seitenwand 30 können die Klemmleisten 309 von der Seitenwand 30 weggeschwenkt werden, um den Flansch 11 freizugeben.

Selbstverständlich können die Klemmleisten 309 in entsprechender Weise auch horizontal oder vertikal verschieblich an der Seitenwand 30 vorgesehen sein, um den Flansch 11 des Transportbehälters 1 vorübergehend zu klemmen.

Alternativ könnte die vordere Seitenwand 15 des Transportbehälters auch mittels einer Saugeinrichtung, elektrisch, magnetisch oder pneumatisch vorübergehend gegen die Seitwand 30 des Reinraums B gezogen oder gedrückt werden.

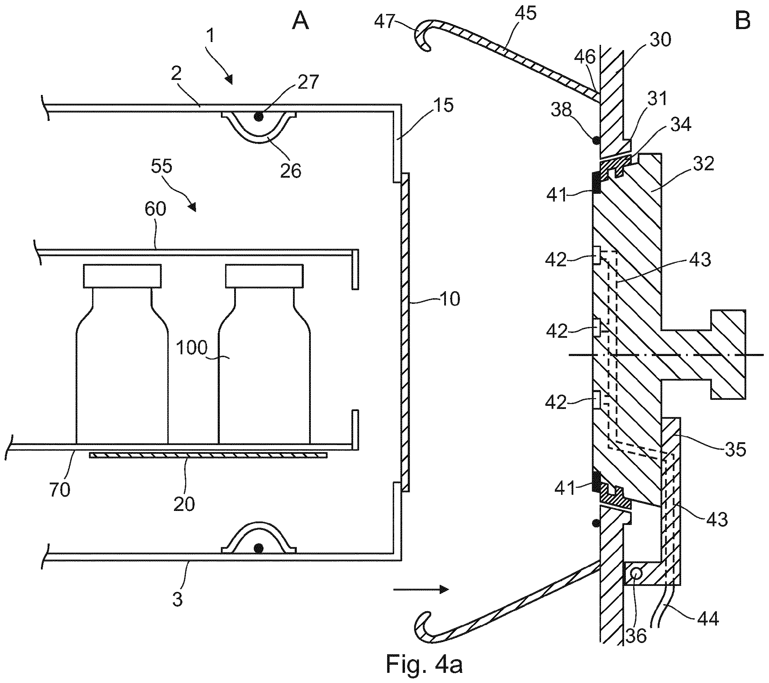

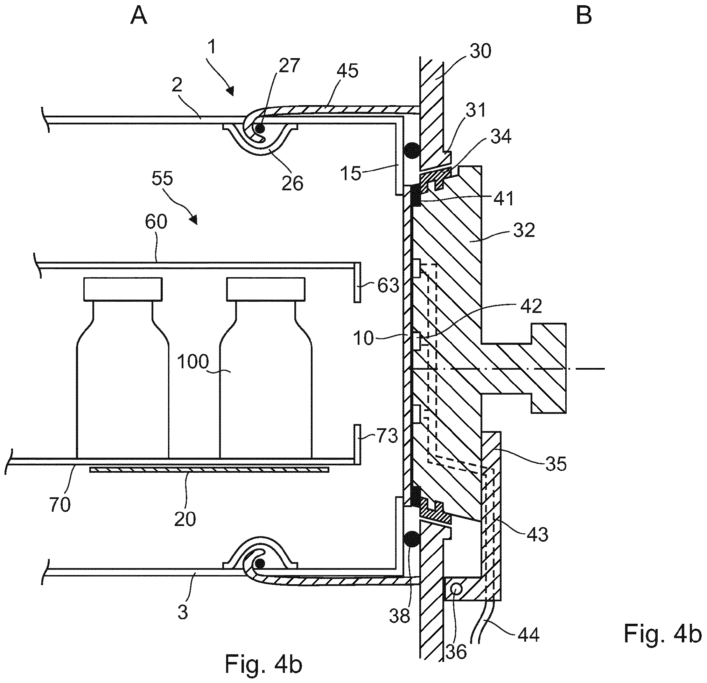

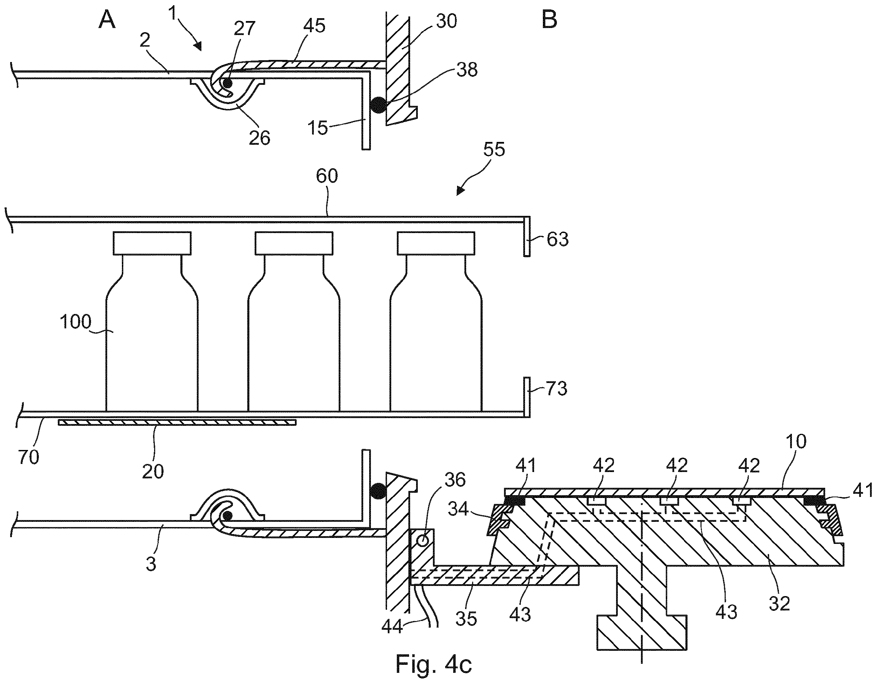

Anhand der Figuren 4a bis 4c wird nachfolgend eine weitere Variante für die vorübergehende Fixierung der Schutzfolie 10 oder Abdeckung an der Außenseite der Schleusentür 32 beschrieben. Diesem Ausführungsbeispiel liegt ein Transportbehälter 1 gemäß der Fig. 1a mit einer flachen vorderen Seitenwand 15 zugrunde, auf der eine Zugriffsöffnung ausgebildet ist und auf die die Schutzfolie 10 oder eine Abdeckung aufgeklebt ist. Gemäß der Fig. 4a sind auf der Außenseite der Schleusentür 32 in Zuordnung zu der Schutzfolie 10 oder Abdeckung mehrere Saugköpfe 42 vorgesehen, die über eine in der Schleusentür 32 und in dem Schwenkarm 35 verlaufende Saugleitung 43 mit einer Verbindungsleitung zu einer Saugeinrichtung (nicht gezeigt) verbunden sind, über die ein Unterdruck an die Saugköpfe 42 angelegt wird. Ausgehend von der Stellung gemäß der Fig. 4a wird der Transportbehälter 1 der Schleusentür 32 angenähert, bis die Stellung gemäß der Fig. 4b erreicht ist, in der die vordere Seitenwand 15 des Transportbehälters 1 unmittelbar an der Dichtung 38 anliegt und die Schutzfolie 10 bzw. Abdeckung unmittelbar an der Außenseite der Schleusentür 32 anliegt. Durch Aktivieren der Saugeinrichtung (nicht gezeigt) wird die Schutzfolie 10 bzw. Abdeckung von den Saugköpfen 42 gegen die Außenseite der Schleusentür 32 gezogen. In dieser Stellung wird der Kleberand auf der vorderen Seitenwand 15 des Transportbehälters 1 durch Aktivieren der Heizeinrichtung 41 solange erwärmt, bis der Kleber ausreichend erweicht ist, sodass beim Öffnen der Schleusentür die von den Saugköpfen 42 ausgeübte Kraft die Haftkraft des Klebers überschreitet und die Schutzfolie 10 bzw. Abdeckung von der vorderen Seitenwand 15 des Transportbehälters 1 abgezogen wird, wie in der Fig. 4c gezeigt.

Für den Fall, dass die Schutzfolie 10 eine gasdurchlässige Schutzfolie ist, beispielsweise eine gasdurchlässige Tyvek®-Schutzfolie, können auf dieser in Zuordnung zu den Positionen der Saugköpfe 42 gasundurchlässige Abschnitte vorgesehen sein, beispielsweise ausgebildet durch Aufbringen von gasundurchlässigen kreisförmigen Bereichen, insbesondere durch Aufbringen eines gasundurchlässigen Lacks oder einer gasundurchlässigen Beschichtung an diesen Positionen, sodass die Schutzfolie 10 bzw. Abdeckung dann mittels der Saugköpfe 42 und Anlegen eines Unterdrucks gegen die Außenseite der Schleusentür 32 gezogen werden kann.

Im Vergleich zu der vorhergehenden Ausführungsform erfolgt nach den Figuren 4a bis 4c die vorübergehende Fixierung des Transportbehälters 1 mittels einer an der Außenseite des Transportbehälters 1 ausgebildete muldenartige Vertiefung 26, in der ein Haltesteg 27 vorgesehen ist. Durch Verstellen von schwenkbeweglich gelagerten Verrastungsarmen 45 ausgehend von der geöffneten Stellung gemäß der Fig. 4a in die Verrastungsstellung gemäß der Fig. 4b hintergreifen die hakenförmigen vorderen Enden 47 der Verrastungsarme 45 die Haltestege 27 auf der Außenseite des Transportbehälters 1 zur vorübergehenden Fixierung des Transportbehälters 1. Hierzu kann auch die Länge der Verrastungsarme 45 verstellbar sein, um den Transportbehälter 1 fest gegen die Seitenwand 30 zu ziehen. Anschließend erfolgt die Kopplung der Abdeckung 10 mit der Außenseite der Schleusentür 32. Dies kann gleichfalls durch Aktivieren der dargestellten Saugköpfe 42 sowie der Heizeinrichtung 41 erfolgen. Dadurch kann zunächst ein Kleber, mit dem die Abdeckung 10 auf die vordere Seitenwand 15 des Transportbehälters 1 aufgeklebt ist, erweicht werden. Wenn die von den Saugköpfen 42 ausgeübte Zugkraft die Haftkraft des Klebers 10 übersteigt, wird die Abdeckung 10 gegen die Außenseite der Schleusentür 42 gesaugt.

Selbstverständlich können die Kopplung und das Abziehen der Abdeckung 10 auch in anderer Weise erfolgen. Ausdrücklich angedacht ist beispielsweise eine mechanische Kopplung der Außenseite der Abdeckung 10 mit verstellbaren Greifern, mit Formschlussgebilden auf der Außenseite der Schleusentür 32, wie beispielsweise Rastnoppen und zugeordnete, gegenüberliegend vorgesehene Ausnehmungen, oder dergleichen. Grundsätzlich angedacht ist ferner eine vorübergehende elektrische, magnetische oder pneumatische Kopplung der Außenseite der Abdeckung 10 mit der Außenseite der Schleusentür 32.

Anschließend wird die Schleusentür 32 geöffnet, wie in der Fig. 4c dargestellt, wodurch die Abdeckung 10 von der vorderen Seitenwand 15 des Transportbehälters 1 abgenommen wird. Anschließend erfolgt die Übergabe der Haltestruktur 55 mit den darin aufgenommenen Behältern 100 über die Zugriffsöffnung 9 des Transportbehälters 1 in den Innenraum des Reinraums B. Weil die Außenseite der Abdeckung 10 vor dem Öffnen der Schleusentür 32 in Anlage zur Außenseite der Schleusentür 32 gebracht wurde, können in der geöffneten Stellung der Schleusentür 32 gemäß der Fig. 4c Keime oder Verunreinigungen weder von der Außenseite der Abdeckung 10 noch von der Außenseite der Schleusentür 32 in den Innenraum des Reinraums B gelangen oder die Behälter 100 bei deren Überführung in den Reinraum B verunreinigen. Dieser Effekt wird noch weiter begünstigt, wenn auf der Außenseite der Schleusentür 32 eine umlaufende Dichtung vorgesehen ist, wie diese bei der Ausführungsform gemäß den Figuren 2a bis 2d vorgesehen ist (Bezugszeichen 39), und die Abdeckung 10 vor dem Öffnen der Schleusentür 32 in Anlage mit dieser gebracht wird, um den Spalt 29 (vgl. Fig. 2b) zwischen der Außenseite der Abdeckung 10 und der Außenseite der Schleusentür 32 abzudichten.

Nach Schließen der Schleusentür 32 können die Behälter 100 dann weiter in dem Reinraum B verarbeitet werden. werden Anschließend werden die Verrastungselemente 47 durch Zurückschwenken der Verrastungsarme 45 in deren Ausgangsstellung außer Eingriff mit den korrespondierend ausgebildeten Gegenelementen (Vertiefungen) 25 gebracht, Anschließend wird der Transportbehälter 1 wieder von der Seitenwand 30 des Reinraums B gelöst, wie vorstehend ausgeführt.

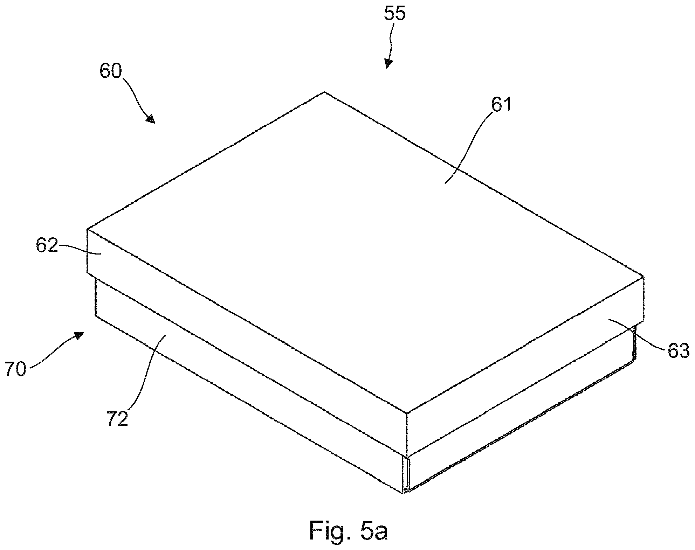

Die Behälter 100 können in dem Transportbehälter 1 in einer Haltestruktur 55 aufgenommen sein, wie nachfolgend anhand der Figuren 5a bis 5e beschrieben, die einer einfacheren Handhabung der Mehrzahl von Behältern 100 dienen soll und die auch einem weiteren Schutz der Behälter 100 vor einem unbeabsichtigten Eindringen von Verunreinigungen und Keimen dienen soll.

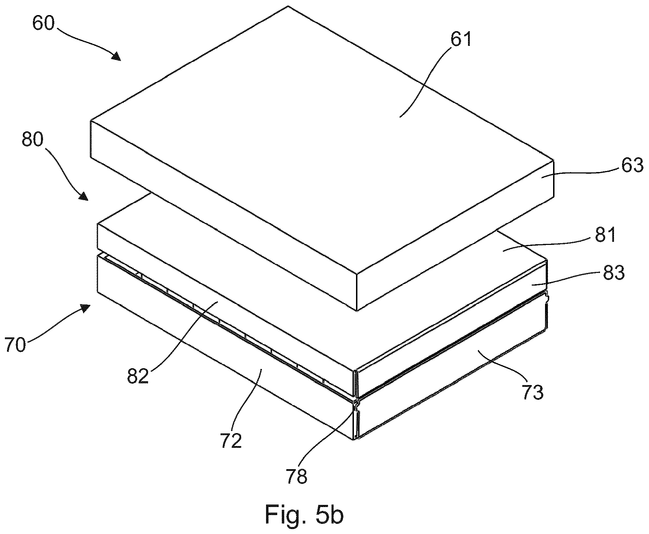

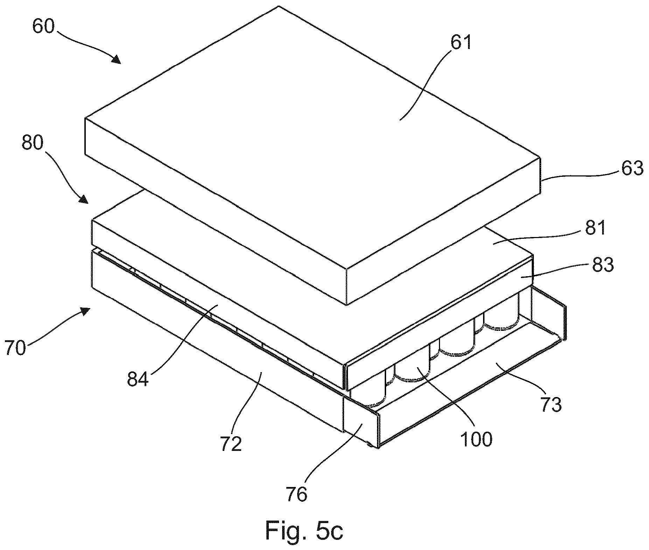

Gemäß der Fig. 5a und 5b umfasst die Haltestruktur 55 ein kastenförmiges Oberteil 60 und ein kastenförmiges Unterteil 70. Das Oberteil 60 weist einen ebenen rechteckförmigen Boden 61 sowie zwei Seitenwände 62 und eine vordere und hintere Seitenwand 63 auf, die jeweils senkrecht von dem Boden 61 abragen. Das Unterteil 70 ist in entsprechender Weise ausgebildet und weist einen ebenen rechteckförmigen Boden 71 sowie zwei Seitenwände 72 auf, die jeweils senkrecht von dem Boden 71 abragen. Die vordere und hintere Seitenwand 73 des Oberteils 70 kann gemäß der Fig. 5c heruntergeklappt werden und bildet mit dem Boden 71 dann eine gemeinsame Ebene aus (ebene Unterlage) und wird zur Ausbildung des kastenförmigen Unterteils 70 zu einem geeigneten Zeitpunkt wieder hochgeklappt.

Wenn die vordere Seitenwand 73 in der Stellung gemäß der Fig. 5c heruntergeklappt ist, können die Behälter 100 von einer Ablagefläche (nicht gezeigt), die bevorzugt bündig mit der Oberseite des Bodens 71 und der heruntergeklappten, vorderen Seitenwand 73 abschließt, einfach in das Unterteil 70 eingeschoben werden. Nach dem Einschieben der Behälter 100 in das Unterteil 70 werden die Seitenlaschen 76, die an den vorderen Enden der Seitenwände 72 ausgebildet sind, und die vordere Seitenwand 73 umgeklappt und miteinander verbunden, um eine vordere Seitenwand auszubilden, die ebenfalls senkrecht vom Boden 71 des Unterteils 70 abragt. Die Höhe der Seitenwände 72, 73 ist dabei geringer als die Höhe der Behälter 100.

Die Behälter 100 sind dabei bevorzugt spielfrei in der Haltestruktur 1 aufgenommen. Die Behälter 100 können in dem Unterteil 70 mit unmittelbarem Wand-zu-Wand-Kontakt aufgenommen sein, um eine größtmögliche Packungsdichte zu erzielen. Gemäß weiteren Ausführungsformen können zwischen sämtlichen Behältern 100 elastische oder unelastische Einlagen vorgesehen sein, beispielsweise in Form von Trennwänden, die eine Berührung von unmittelbar benachbarten Behältern in dem Unterteil 70 verhindern. Gemäß weiteren Ausführungsformen können solche Trennwände auch als elastische oder unelastische Trennstreifen vorgesehen sein, die Reihen von Behältern voneinander trennen und zum Verschieben von Reihen von Behältern verwendet werden können.

Bei der Ausführungsform gemäß der Fig. 5b kann das kastenförmige Oberteil 60 auf die oberen Enden der Behälter 100 unmittelbar aufgesetzt sein, um ein Eindringen von Verunreinigungen von oben in die noch nicht verschlossenen Behälter zu verhindern. Wie in der Fig. 5b gezeigt, kann jedoch auch eine weitere Zwischenlage, beispielsweise eine dünne Kunststoffplatte oder Kunststofffolie, oder das in der Fig. 5b dargestellte kastenförmige Zwischenteil 80 auf die oberen Enden der Behälter 100 aufgelegt sein, bevor das Oberteil 60 ausgesetzt wird, um ein Eindringen von Partikeln in die noch offenen Enden der Behälter 100 auch dann noch zu verhindern, wenn das Oberteil 60 abgenommen ist, wie in der Fig. 5c gezeigt. Die Seitenwände 83, 84 verhindern dabei ein versehentliches seitliches Abrutschen des Zwischenteils 80 von den Behältern 100.

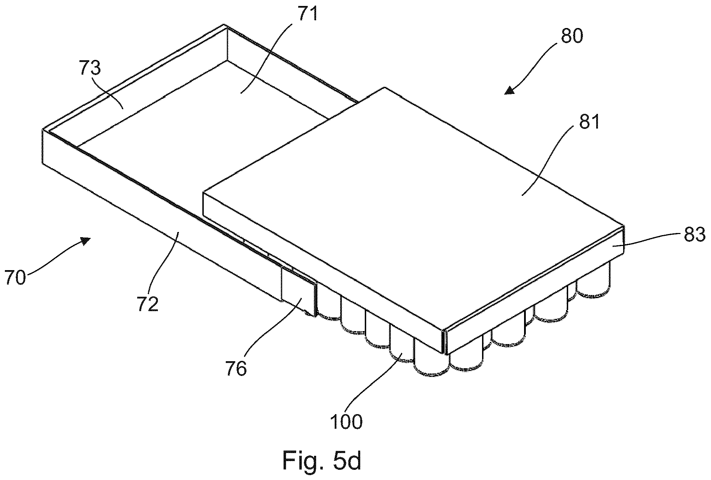

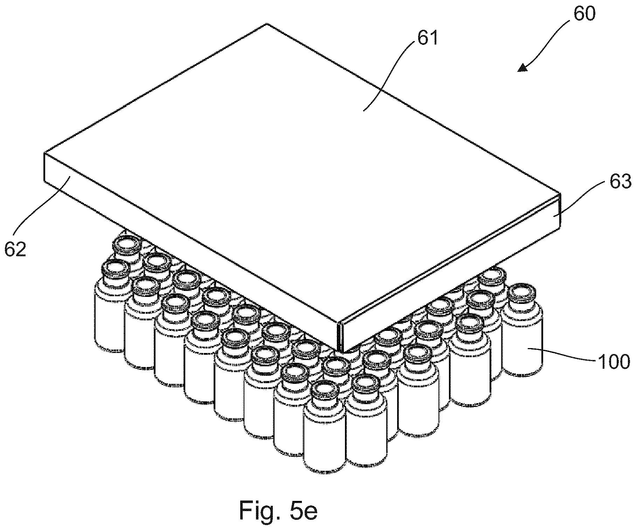

Nach Abnehmen des kastenförmigen Oberteils 60 und Herunterklappen der vorderen Seitenwand 73 des Unterteils 70 gemäß der Fig. 5d können die Behälter 100 durch Verschieben des Zwischenteils 80 aus dem Unterteil 70 herausgeschoben werden. Abschließend wird durch Abheben des Zwischenteils 80 ein Zugriff auf die Behälter 100 zur Behandlung oder Weiterverarbeitung ermöglicht, wie in der Fig. 5e dargestellt. Diese Schritte zum Öffnen der Haltestruktur werden in dem Reinraum B mit der niedrigeren Partikelkonzentration ausgeführt.

Nach der Behandlung oder Weiterverarbeitung der Behälter 100 in dem Reinraum B und ggf. Verschließen der Behälter 100 können die vorstehend ausgeführten Schritte in umgekehrter Reihenfolge ausgeführt werden, um die in der Fig. 5a gezeigte Haltestruktur 55 erneut auszubilden. Während der Behandlung oder Weiterverarbeitung der Behälter 100 in dem Reinraum B werden die Bestandteile dieser Haltestruktur 55 aufgrund der im Reinraum B bestehenden niedrigen Partikelkonzentration nicht weiter verunreinigt, sodass die Haltestruktur 55 mit den darin aufgenommenen Behältern 100 nach deren Behandlung oder Weiterverarbeitung auch weiterhin höchsten Anforderungen an die Sterilität genügen kann.

Der Boden 71 insbesondere des Unterteils 70 kann mit einer Gleitschicht beschichtet sein, um das vorstehend beschriebene Einschieben der Behälter 100 zu erleichtern. Die Gleitschicht kann aus einem Polymer und einer Haftvermittlerschicht bestehen. Die Gleitschicht besteht vorzugsweise mindestens aus einer Mischung aus einem aromatischen Silan und einem aliphatischen Silan bestehen. Weitere geeignete Materialien für das Unterteil 70 sind beispielsweise Polyamid oder Polyoxymethylen (POM).

Die in der Fig. 5a dargestellte Haltestruktur 55 kann zum weiteren Transport in einen Schlauch oder Verpackungsbeutel aus Kunststoff steril eingeschweißt werden, wie in der Fig. 1h gezeigt, was unmittelbar in dem Reinraum B erfolgen kann. Grundsätzlich kann die Haltestruktur 55 jedoch durch umgekehrte Ausführung der Schritte, die vorstehend anhand der Figuren 2 bis 4 beschrieben wurden, auch in den vorgeschalteten Reinraum A zurück überführt werden, insbesondere in einen dort befindlichen Transportbehälter, was nachfolgend näher anhand der Fig. 7c beschrieben werden soll.

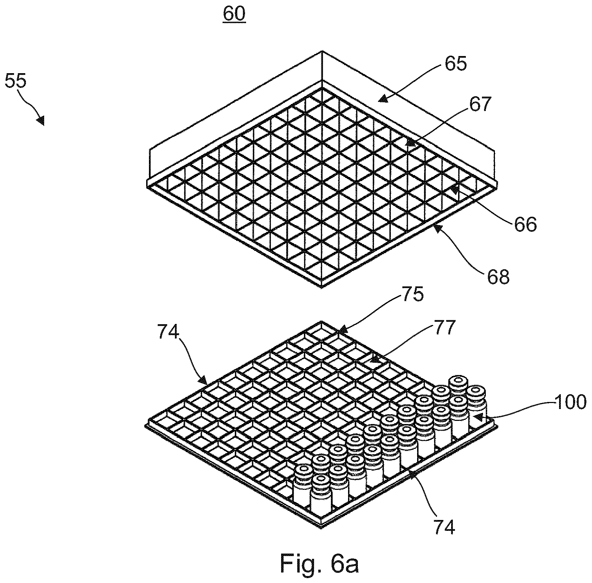

Zur vorübergehenden Aufbewahrung der Behälter in einer vorbestimmten geometrischen Anordnung, insbesondere in einer zweidimensionalen Matrixanordnung, können auch beliebige andere Haltestrukturen verwendet werden, wie nachfolgend beispielhaft anhand der Figuren 6a bis 6c beschrieben. Derartige Haltestukturen können die oberen Enden der darin aufgenommenen Behälter abdecken, wie vorstehend beschrieben, was jedoch nicht unbedingt erforderlich ist.

So zeigt die Fig. 6a ein Beispiel für eine zweiteilig ausgebildete Haltestruktur mit einem flachen Unterteil 70 und einem kastenförmigen Oberteil 60. Auf dem flachen Unterteil 70 werden von der umlaufenden Seitenwand 74 und der Mehrzahl von sich parallel zueinander erstreckenden und sich senkrecht kreuzenden Querstegen 75 eine Mehrzahl von jeweils quadratischen Aufnahmen 77 ausgebildet, in denen die Behälter 100 aufgenommen sind. Auch an dem kastenförmigen Oberteil 60 werden von der umlaufenden Seitenwand 65 und der Mehrzahl von sich parallel zueinander erstreckenden und sich senkrecht kreuzenden Querstegen 66 eine Mehrzahl von jeweils quadratischen Aufnahmen 67 ausgebildet. Die Querstege 66 des Oberteils 60 sind entsprechend den Querstegen 75 des Unterteils 70 angeordnet, sodass beim Aufsetzen des Oberteils 60 auf das Unterteil 70 die darauf angeordneten Behälter 100 in Aufnahmen aufgenommen sind, die gemeinsam von den Querstegen 66 des Oberteils 60 und den Querstegen 75 des Unterteils 70 ausgebildet werden, wodurch eine Kollision von unmittelbar benachbarten Behältern 100 in der so ausgebildeten regelmäßigen Matrixanordnung von Behältern 100 verhindert werden kann.

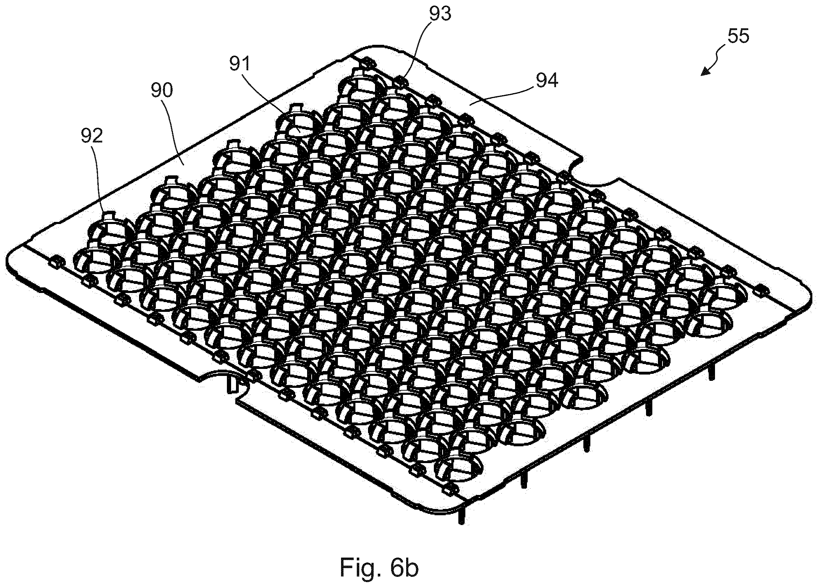

Die Fig. 6b zeigt ein weiteres Beispiel für eine einteilig ausgebildete Haltestruktur 55 zum Halten einer Mehrzahl von Behältern, die einen flächigen rechteckförmigen Träger 90 umfasst, der aus einem Kunststoff ausgebildet ist, beispielsweise ausgestanzt oder spritzgegossen ist, und eine Mehrzahl von Öffnungen 91 zur Aufnahme der Behälter (nicht gezeigt) aufweist. Die Öffnungen 91 sind in einer regelmäßigen zweidimensionalen Matrix-Anordnung aus Reihen und sich rechtwinklig dazu erstreckenden Spalten angeordnet, die unter gleichen Abständen zueinander und regelmäßig zueinander versetzt in einer wiederkehrenden Anordnung angeordnet sind. Von der Oberseite des Trägers 90 ragen elastische Haltezungen 92 bogenförmig ab und, in Draufsicht betrachtet, in die zugeordneten Öffnungen 91 hinein. Die elastischen Haltezungen 92 sind bevorzugt einstückig mit dem flächigen Träger 90 ausgebildet sind, beispielsweise durch 1K- oder 2K-Kunststoff-Spritzgussverfahren. Die Behälter können zumindest mit radialem Spiel und bevorzugt sowohl mit radialem als auch mit axialem Spiel lose auf den elastischen Haltezungen 92 abgestützt sein, um Toleranzen auszugleichen. Die Ränder 94 des flächigen Trägers 90 können weggeklappt werden, um die Grundfläche des Trägers 90 weiter zu reduzieren, beispielsweise dann, wenn dieser mit den Behältern an eine Weiterverarbeitungsstation mit begrenzter Grundfläche übergeben werden soll, beispielsweise an einen Gefriertrockenschrank. Zu diesem Zweck sind die Ränder 94 über Scharniere 93 mit dem jeweiligen Träger 90 verbunden, beispielsweise ausgebildet als Filmscharniere oder Schnapp- bzw. Federscharniere aus einem Kunststoff und einstückig mit dem Träger 90 ausgebildet.

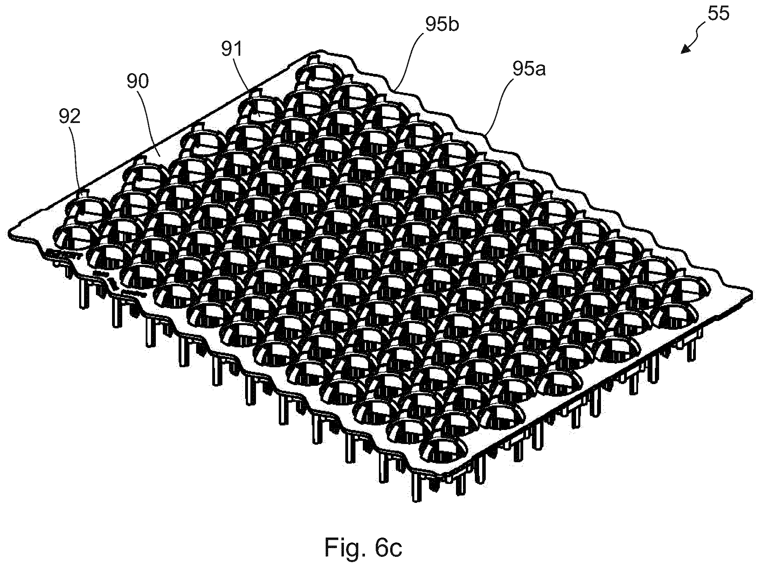

Die Fig. 6c zeigt eine weitere Variante zur Haltestruktur gemäß der Fig. 6b, bei der mehrere Vorsprünge 95b und Aussparungen 95a am Rand des flächigen Trägers 90 ausgebildet sind, die mit entsprechenden Vorsprüngen und Aussparungen eines benachbarten Trägers (nicht gezeigt), der identisch ausgebildet ist, vorübergehend formschlüssig in Eingriff gebracht werden können, etwa um diese miteinander vorübergehend zu koppeln und gemeinsam zu verschieben.

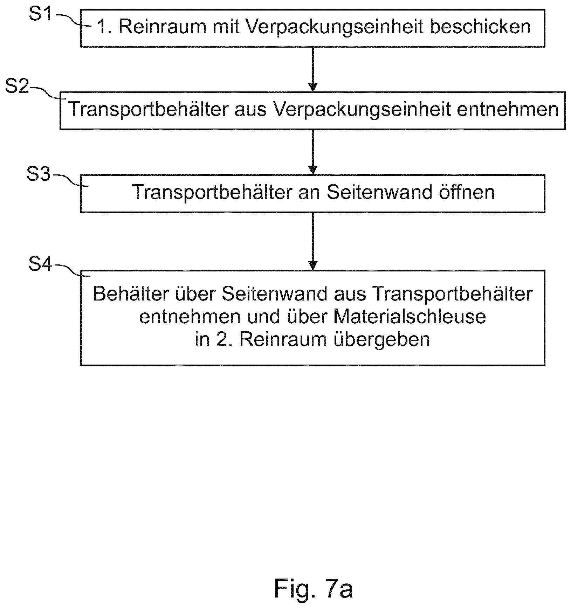

Nachfolgend wird anhand des Flussdiagramms gemäß der Fig. 7a ein Verfahren zum Überführen einer Mehrzahl von Behältern aus einem Transport- und Verpackungsbehälter in einen Reinraum gemäß einer ersten Ausführungsform der vorliegenden Erfindung beschrieben. Dabei wird davon ausgegangen, dass die Behälter in einem Transport- und Verpackungsbehälter bereitgestellt werden, der in einer Verpackungseinheit steril verpackt ist, beispielsweise in einen Kunststoffbeutel eingeschweißt. Bei den Behältern kann es sich um Vials, Ampullen, Karpulen oder Spritzen- oder Injektionsbehältnisse oder beliebige andere Behälter aus einem Glas oder Kunststoff handeln. Die Verpackung der Behälter in den Transport- und Verpackungsbehälter und zu der Verpackungseinheit erfolgt zweckmäßig auf Seiten des Herstellers. Die Verpackungseinheit wird anschließend an einen weiterverarbeitenden Betrieb geliefert, beispielsweise an einen Pharmaabfüllbetrieb. Hierzu können die vorstehend beschriebenen Transport- und Verpackungsbehälter in einen Kunststoffschlauch steril verpackt eingeschweißt sein, wie in der Fig. 1h gezeigt.

Diese Verpackungseinheit wird zunächst in einen ersten Raum eingebracht, bei dem es sich um einen Reinraum mit einer relativ hohen Partikelkonzentration handeln kann (Schritt S1).

Zur Übergabe an einen Reinraum mit einer niedrigeren Partikelkonzentration wird der Transport- und Verpackungsbehälter mit der Mehrzahl von darin aufgenommenen Behältern zunächst aus der Verpackungseinheit entnommen (Schritt S2) und der Transport- und Verpackungsbehälter so in der Nähe der Seitenwand des Reinraums angeordnet, dass eine Seitenwand des Transport- und Verpackungsbehälter nahe der Schleusentür des Reinraums angeordnet ist (Schritt S3). Dies kann in dem vorgeschalteten Reinraum mit einer höheren Partikelkonzentration als in dem Reinraum erfolgen, in dem die Behälter später weiterverarbeitet werden sollen, der zu diesem Zweck eine niedrigere Partikelkonzentration aufweist. Grundsätzlich kann es für eine sterile Übergabe der Behälter ausreichend sein, wenn der Spalt zwischen der Seitenwand des Transport- und Verpackungsbehälters und der Schleusentür bzw. Seitenwand des Reinraums mit der niedrigeren Partikelkonzentration vergleichsweise schmal ausgebildet ist, sodass die Wahrscheinlichkeit des Eindringens von Verunreinigungen in diesen Spalt und somit in den Reinraum mit der niedrigeren Partikelkonzentration gering ist. Bevorzugt wird die Seitenwand des Transport- und Verpackungsbehälter jedoch so nahe an der Seitenwand des Reinraums mit der niedrigeren Partikelkonzentration angeordnet, dass der vorgenannte Spalt mittels Dichtungsmitteln, wie vorstehend beschrieben, abgedichtet ist und ein Eindringen von Verunreinigungen in diesen Spalt verhindert ist.

Anschließend erfolgt das gleichzeitige Öffnen der Seitenwand des Transport- und Verpackungsbehälters und der Schleusentür, sodass eine Zugriffsöffnung des Transport- und Verpackungsbehälters mit einem Innenraum des Reinraums mit der niedrigeren Partikelkonzentration in Verbindung steht und die Mehrzahl von Behältern aus dem Transport- und Verpackungsbehälter in den Innenraum des Reinraums überführt werden kann (Schritt S4). Bevorzugt wird zu diesem Zweck die auf der Seitenwand des Transport- und Verpackungsbehälters vorgesehene Abdeckung oder Schutzfolie vorübergehend mit der Schleusentür gekoppelt, sodass die Schleusentür gemeinsam mit der daran vorübergehend fixierten Abdeckung oder Schutzfolie geöffnet werden kann, wie vorstehend beschrieben.

Anschließend erfolgt das Schließen der Schleusentür, sodass die Behälter dann in dem Reinraum mit der niedrigeren Partikelkonzentration weiterverarbeitet werden können. Anschließend wird der Transport- und Verpackungsbehälter von der Seitenwand des Reinraums wieder gelöst, um die Schleusentür für die Übergabe einer weiteren Mehrzahl von Behältern aus einem weiteren Transport- und Verpackungsbehälter in den Reinraum freizugeben.

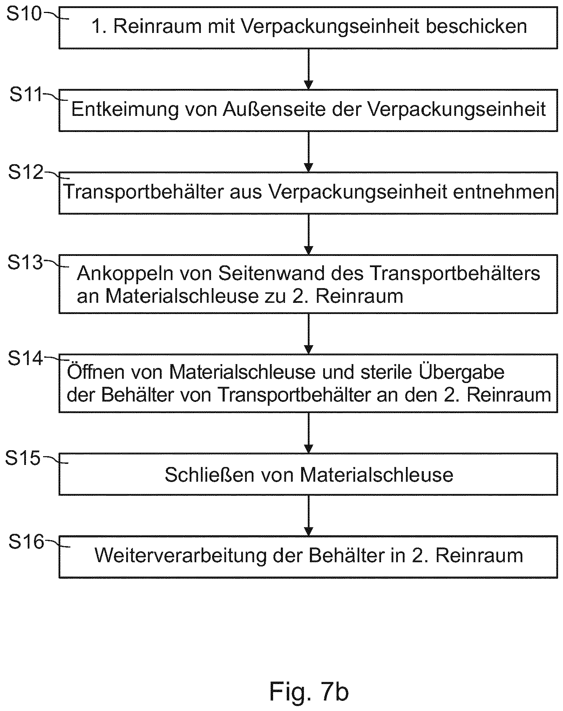

Das Flussdiagramm gemäß der Fig. 7b fasst die Schritte eines Verfahrens gemäß einer zweiten Ausführungsform der vorliegenden Erfindung zusammen. Dabei wird angenommen, dass die Behälter in einer Verpackungseinheit verpackt bereitgestellt werden, beispielsweise in einen sterilen Verpackungsbeutel aus einem Kunststoff eingeschweißt, in welchem zumindest ein Transport- und Verpackungsbehälter aufbewahrt ist, wie vorstehend ausgeführt. Die Außenseite dieser Verpackungseinheit wird in einen ersten Raum, insbesondere in einen ersten Reinraum mit einer höheren Partikelkonzentration, eingebracht (Schritt S10). Anschließend wird die Außenseite der Verpackungseinheit entkeimt (Schritt S11), beispielsweise mittels Dampfsterilisation, mittels eines Gases (beispielsweise mittels eines Ethylenoxid-(EO)- Sterilisationsprozesses) oder durch Bestrahlung. Anschließend wird die Verpackungseinheit geöffnet und der zumindest eine Transport- und Verpackungsbehälter aus der Verpackungseinheit entnommen (Schritt S12). Die Übergabe der Behälter in den Reinraum in den Schritten S13 und S14 erfolgt in ähnlicher Weise, wie vorstehend anhand der Fig. 7a beschrieben. Der Fig. 7b ist dabei zugrunde gelegt, dass die Seitenwand des Transportbehälters oder die an dieser Seitenwand vorgesehene Abdeckung oder Schutzfolie zunächst mit der Außenseite der Schleusentür gekoppelt wird (Schritt S13), bevor die Schleusentür geöffnet wird. Zur sterilen Entnahme und Übergabe der Behälter kann es jedoch ausreichend sein, wenn die Seitenwand des Transport- und Verpackungsbehälters an die Materialschleuse des Reinraums geeignet angekoppelt wird, was beispielsweise auch durch Abdichten des Spalts zwischen der Abdeckung oder Schutzfolie und der Schleusentür mittels einer umlaufend um die Abdeckung oder Schutzfolie vorgesehenen Dichtung erfolgen kann, die beispielhaft in den Figuren 1a bis 1c gezeigt ist, die jedoch auch an der Seitenwand des Reinraums vorgesehen sein kann.

Eine sterile Entnahme und Übergabe der Behälter kann zuverlässiger durch vorübergehendes Koppeln der Abdeckung oder Schutzfolie auf der Seitenwand des Transport- und Verpackungsbehälters mit der Schleusentür erfolgen, wie vorstehend ausgeführt.

Anschließend erfolgt das Öffnen der Seitenwand des Transport- und Verpackungsbehälters und der Schleusentür und die Überführung der Mehrzahl von Behältern aus dem Transport- und Verpackungsbehälter in den Innenraum des Reinraums mit der niedrigeren Partikelkonzentration (Schritt S14). Nach Schließen der Materialschleuse (Schritt S15) können die Behälter dann in diesem Reinraum weiterverarbeitet werden (Schritt S16), wie vorstehend ausgeführt.

Abschließend wird der Transport- und Verpackungsbehälter von der Seitenwand des Reinraums wieder gelöst, um die Schleusentür für die Übergabe einer weiteren Mehrzahl von Behältern von einem weiteren Transport- und Verpackungsbehälter in den Reinraum freizugeben.

Zwischen dem Schritt S13 (Ankoppeln des Transportbehälters ...) und S14 (Öffnen der Materialschleuse ...) kann optional ein weiterer Schritt zur Entkeimung speziell der vorderen Seitenwand des Transportbehälters mit der darauf vorgesehenen Abdeckung bzw. Schutzfolie vorgesehen sein. Geeignete Prozesse hierfür sind insbesondere: Beströmen der vorderen Seitenwand mit Ethylenoxid (ETO), Bestrahlen der vorderen Seitenwand mittels Gammastrahlung oder Elektronenstrahlen, Beströmen der vorderen Seitenwand mit Dampf (unter kontrollierten Bedingungen), oder neuere Verfahren, wie beispielsweise oxidative Niedertemperatur-Sterilisation. Zu diesem Zweck können im Bereich der Schleusentür auf der Außenseite des Reinraums mit der niedrigeren Partikelkonzentration entsprechend ausgelegte Entkeimungseinrichtungen vorgesehen sein, insbesondere Gasauslässe oder Bestrahlungseinrichtungen. Die Entkeimung erfolgt bevorzugt erst, wenn der Spalt zwischen der vorderen Seitenwand des Transportbehälters und der Seitenwand des Reinraums gegen die Umgebung abgedichtet ist. Grundsätzlich kann die Entkeimung jedoch auch erfolgen, ohne dass der Spalt abgedichtet ist, insbesondere wenn der Transportbehälter bereits soweit an die Seitenwand angenähert wurde, dass die Spaltbreite im Vergleich zu den Abmessungen des Transportbehälters bereits vergleichsweise gering ist.

Die vorstehend beschriebene Schrittfolge kann im Wesentlichen in umgekehrter Reihenfolge ausgeführt werden, um die Behälter aus dem Reinraum mit der niedrigeren Partikelkonzentration nach deren Weiterverarbeitung darin zurück in einen Transport- und Verpackungsbehälter zu überführen, der dann, sofern notwendig, erneut in einer Verpackungseinheit steril verpackt wird. Dies wird beispielhaft nachfolgend anhand der Fig. 7c beschrieben.

Nach der Weiterverarbeitung der Behälter in dem Reinraum mit der niedrigeren Partikelkonzentration (Schritt S20) kann vor der Übergabe der Behälter in den Transport- und Verpackungsbehälter zunächst der Innenraum des Transport- und Verpackungsbehälters in dem vorgeschalteten Reinraum mit der höheren Partikelkonzentration entkeimt werden (Schritt S21). Dieser Schritt ist jedoch nicht zwingend notwendig oder kann erst nach der Übergabe der Behälter in den Transport- und Verpackungsbehälter erfolgen, beispielsweise durch Einströmen eines Gases mittels eines Ethylenoxid-(ETO)- Sterilisationsprozesses (insbesondere, wenn an dem Transport- und Verpackungsbehälter eine gasdurchlässige Schutzfolie vorgesehen ist).

Anschließend erfolgt in dem Schritt S22 das Ankoppeln einer öffenbaren Seitenwand des Transport- und Verpackungsbehälters an die Materialschleuse zu dem Reinraum mit der niedrigeren Partikelkonzentration in der gleichen Weise, wie vorstehend beschrieben. Anschließend erfolgt das Öffnen der Materialschleuse und die Übergabe der Behälter aus dem Reinraum mit der niedrigeren Partikelkonzentration in den Transport- und Verpackungsbehälter (Schritt S23). Hierzu wird es bevorzugt, wenn die Seitenwand des Transportbehälters oder die an dieser vorgesehene Abdeckung oder Schutzfolie zunächst mit der Außenseite der Schleusentür gekoppelt wird, bevor die Schleusentür geöffnet wird, wie vorstehend beschrieben. Nach dem Überführen der Behälter in den Transport- und Verpackungsbehälter wird die Schleusentür wieder geschlossen (Schritt S24) und die Zugriffsöffnung auf der Seitenwand des Transport- und Verpackungsbehälters wieder mittels einer Abdeckung oder Schutzfolie steril verschlossen. Zu diesem Zweck kann vorgesehen sein, die mit der Schleusentür gekoppelte Abdeckung oder Schutzfolie nach dem Schließen der Schleusentür freizugeben und die Abdeckung oder Schutzfolie geeignet an der Seitenwand des Transport- und Verpackungsbehälters zu befestigen, um die Zugriffsöffnung wieder zu verschließen.

Wenn die Abdeckung beispielsweise als gasdurchlässiger oder gasundurchlässiger Verschlussdeckel ausgebildet ist, kann zu diesem Zweck das Aufdrücken des Verschlussdeckels auf die Seitenwand des Transport- und Verpackungsbehälters oder eine andere geeignete mechanische Verbindung des Verschlussdeckels mit der Seitenwand des Transport- und Verpackungsbehälters ausreichend sein, beispielsweise durch Aufschrauben des Verschlussdeckels. Zu diesem Zweck können auf der Außenseite der Schleusentür oder der Seitenwand des Reinraums mit der niedrigeren Partikelkonzentration geeignete Verstell- oder Handhabungseinrichtungen vorgesehen sein, beispielsweise mechanische Greifer, wie vorstehend ausgeführt. Natürlich kann ein solcher Verschlussdeckel auch auf die Seitenwand des Transport- und Verpackungsbehälters geeignet aufgeklebt werden.

Wenn die Abdeckung dagegen beispielsweise als gasdurchlässige Schutzfolie ausgebildet ist, kann die Schutzfolie in geeigneter Weise auf die Seitenwand des Transport- und Verpackungsbehälters aufgeklebt werden. Hierzu kann es ausreichend sein, die vorstehend anhand der Figuren 3a bis 3e bzw. 4a bis 4c beschriebenen Heizeinrichtungen 41 erneut zu betätigen, um den Kleberand auf der Seitenwand des Transport- und Verpackungsbehälters, der um die Zugriffsöffnung herum umlaufend ausgebildet ist, erneut zu erweichen, die Schutzfolie dann geeignet auf die Seitenwand aufzudrücken und den Kleberand wieder abkühlen zu lassen. Hierzu können auf der Außenseite der Schleusentür geeignete Verstelleinrichtungen vorgesehen sein, beispielsweise ein umlaufend und korrespondierend zu dem Kleberand ausgebildeter Druckstempel, um die Schutzfolie im Bereich des Kleberands auf die Seitenwand des Transport- und Verpackungsbehälters aufzudrücken.

Anschließend wird die Außenseite des Transport- und Verpackungsbehälters in dem Schritt S25 in dem vorgeschalteten Reinraum mit der höheren Partikelkonzentration entkeimt, was zweckmäßig erfolgt, nachdem der Transport- und Verpackungsbehälter von der Seitenwand des Reinraums mit der niedrigeren Partikelkonzentration abgelöst wurde.

Anschließend kann eine weitere Entkeimung des Innenraums des Transport- und Verpackungsbehälters erfolgen, beispielsweise zur Entkeimung der Außenseiten der darin aufgenommenen Behälter. Dies kann insbesondere durch Einströmen eines Gases mittels eines Ethylenoxid-(ETO)- Sterilisationsprozesses erfolgen, wenn an dem Transport- und Verpackungsbehälter eine gasdurchlässige Schutzfolie als Abdeckung der Zugriffsöffnung vorgesehen ist.

Der Weitertransport der Behälter kann unmittelbar in dem Transport- und Verpackungsbehälter erfolgen. Zweckmäßig wird der Transport- und Verpackungsbehälter oder werden mehrere Transport- und Verpackungsbehälter jedoch in dem Schritt S26 in eine Verpackungseinheit eingebracht, beispielsweise in einen Verpackungsbeutel aus einem sterilen Kunststoff eingeschweißt. Anschließend kann diese Verpackungseinheit in dem Schritt S27 geschlossen werden, beispielsweise zugeschweißt werden, und die Außenseite der Verpackungseinheit entkeimt werden. Abschließend wird diese Verpackungseinheit dann in dem Schritt S28 aus dem Reinraum mit der höheren Partikelkonzentration entnommen und an einen Endkunden ausgeliefert.

Zwischen dem Schritt S12 (Ankoppeln des Transportbehälters...) und S14 (Öffnen der Materialschleuse ...) kann optional ein weiterer Schritt zur Entkeimung speziell der vorderen Seitenwand des Transportbehälters mit der darauf vorgesehenen Abdeckung bzw. Schutzfolie vorgesehen sein. Geeignete Prozesse hierfür sind insbesondere: Beströmen der vorderen Seitenwand mit Ethylenoxid (ETO), Bestrahlen der vorderen Seitenwand mittels Gammastrahlung oder Elektronenstrahlen, Beströmen der vorderen Seitenwand mit Dampf (unter kontrollierten Bedingungen), oder neuere Verfahren, wie beispielsweise oxidative Niedertemperatur-Sterilisation. Zu diesem Zweck können im Bereich der Schleusentür auf der Außenseite des Reinraums mit der niedrigeren Partikelkonzentration entsprechend ausgelegte Entkeimungseinrichtungen vorgesehen sein, insbesondere Gasauslässe oder Bestrahlungseinrichtungen. Die Entkeimung erfolgt bevorzugt erst, wenn der Spalt zwischen der vorderen Seitenwand des Transportbehälters und der Seitenwand des Reinraums gegen die Umgebung abgedichtet ist. Grundsätzlich kann die Entkeimung jedoch auch erfolgen, ohne dass der Spalt abgedichtet ist, insbesondere wenn der Transportbehälter bereits soweit an die Seitenwand angenähert wurde, dass die Spaltbreite im Vergleich zu den Abmessungen des Transportbehälters bereits vergleichsweise gering ist.

Zur Klassifizierung der vorgenannten Reinräume wird die Norm ISO 14644-1 hiermit ausdrücklich in Bezug genommen. Als Reinraumklasse für den vorgenannten Reinraum mit der niedrigeren Partikelkonzentration wird je nach der Art der Weiterverarbeitung der Behälter und/oder der zur Aufbewahrung bestimmten Substanz in diesem Reinraum eine geeignete Reinraumklasse gewählt. Im Rahmen der vorliegenden Erfindung angedacht ist hier insbesondere die Reinraumklasse 3 gemäß ISO 14644-1, bevorzugter die Reinraumklasse 2 gemäß ISO 14644-1 und noch bevorzugter die Reinraumklasse 1 gemäß ISO 14644-1. Der Transport- und Verpackungsbehälter und die darin aufgenommenen Behälter können in einer entsprechenden Reinraumklasse angeliefert und/oder nach der Weiterverarbeitung in dem Reinraum mit der niedrigeren Partikelkonzentration wieder steril verpackt in einer entsprechenden Reinraumklasse ausgeliefert werden.

Zweckmäßig ist die Reinraumklasse des dem Reinraum mit der niedrigeren Partikelkonzentration vorgeschalteten Raums oder Reinraums schlechter als diese Reinraumklasse, bevorzugt eine Klasse gemäß ISO 14644-1 schlechter als die Reinraumklasse des Reinraums mit der niedrigeren Partikelkonzentration. Die Klassifizierung der vorgenannten Reinräume kann in entsprechender Weise auch gemäß GMP (engl. Good Manufacturing Practice) vorgenommen werden, wobei GMP Klasse A die beste Reinraumklasse und GMP Klasse D die schlechteste Reinraumklasse darstellt.

Selbstverständlich kann die kontrollierte Übergabe der Behälter in einen Reinraum gemäß der vorliegenden Erfindung auch mehrmals nacheinander ausgeführt werden.

Wie dem Fachmann beim Studium der vorstehenden Beschreibung ersichtlich sein wird, kann als Abdeckung auf der vorderen Stirnseite des Transportbehälters grundsätzlich statt einer gasdurchlässigen Schutzfolie auch eine gasundurchlässige Schutzfolie, beispielsweise aus Aluminium oder Kunststoff oder einem Metall-Kunststoff-Verbundmaterial, verwendet werden, die auf eine oder zwei vordere Stirnseiten des Transportbehälters aufgeklebt ist. Alternativ können Deckel, Klappen oder öffenbare Klappen, Türen oder öffenbare Klappmechanismen verwendet werden, die aus einem Kunststoff oder Metall gefertigt sind und die Zugriffsöffnung auf der vorderen Stirnseite des Transportbehälters geeignet verschließen, beispielsweise in diese eingesteckt sind, oder in eine Verschlussstellung durch eine Schwenk-, Klapp- oder Schiebebewegung verstellt werden können.

Wie dem Fachmann beim Studium der vorstehenden Beschreibung ersichtlich sein wird, können erfindungsgemäß insbesondere die folgenden Vorteile erzielt werden:
- die Verpackung kann nach dem Zwiebelprinzip verpackt- und entpackt werden;
- die Verpackung kann an einen Port, beispielsweise zwischen zwei Reinräumen oder Sterilräumen, angedockt werden, von innen über eine gasdurchlässige Schutzfolie mit einem Gas desinfiziert und weiter entpackt werden;
- die Be- und Entladung geschieht bevorzugt seitlich, sodass die Behälter nicht gekippt werden;
- die Verwendung von Wendeplatten ist erfindungsgemäß nicht notwendig;
- quaderförmig ausgebildete Transport- und Verpackungsbehälter sind stabil und stapelbar;
- die Transport- und Verpackungsbehälter ist hermetisch dicht und können bei Verwendung einer Klappe an der vorderen Seitenfläche in einfacher Weise geöffnet, entladen und wieder verschlossen werden;
- die Transport- und Verpackungsbehälter sind einfach und kostengünstig fertigbar, bedingt durch eine einfache Kontur/Geometrie (beispielsweise Kastenform)
- die Transport- und Verpackungsbehälter können insbesondere mit einer gasdurchlässigen Schutzfolie versehen sein, wie vorstehend beschrieben, sodass dieses sich auch im geschlossenen Zustand desinfizieren lassen, insbesondere durch Einströmen eines desinfizierenden Gases oder Dampfs;
- Schutzfolien oder Abdeckungen, wie beispielsweise Klappen, können an beiden Stirnseiten eines Transport- und Verpackungsbehälters vorhanden sein, sodass diese wahlweise von beiden Stirnseiten beladen und entladen werden können, beispielsweise durch Verschieben einer Haltestruktur, wie vorstehend beschrieben.

Wie dem Fachmann beim Studium der vorstehenden Beschreibung ersichtlich sein wird, können mit dem vorgenannten Verfahren nicht nur Pharmabehälter und/oder Verschlusselemente in einen Reinraum aseptisch eingeschleust werden, sondern mit dem Abdocken in entsprechender Weise auch Verpackungsmaterialien (also Abfall) wieder aus einem Reinraum ausgeschleust werden. Die Beutel/ Tubs und ggfs. auch Nester können so den zur Weiterverarbeitung der Pharmabehälter verwendeten Isolator oder Reinraum auch unkompliziert wieder verlassen.

Wenngleich die Schleusentür des Reinraums vorstehend als schwenkbeweglich gelagert beschrieben wurde, kann die Zugriffsöffnung auf den Reinraum in entsprechender Weise auch mittels anderer aus dem Stand der Technik bekannter Verschlussorgane verschlossen und abgedichtet werden, beispielsweise hydraulisch vertikal oder horizontal verschoben werden, um die Zugriffsöffnung auf den Reinraum freizugeben, ähnlich wie bei einem Plattenventil bei Vakuumanlagen.

Wie dem Fachmann beim Studium der vorstehenden Beschreibung ohne weiteres ersichtlich sein wird, können die vorstehend beschriebenen Merkmale und Aspekte nach der vorliegenden Erfindung auch in anderer Weise miteinander kombiniert werden, als konkret vorstehend beschrieben. Solche weiteren Ausführungsformen sollen von dem Schutzbereich der beigefügten Patentansprüche als mit umfasst gelten, sofern diese von dem allgemeinen Lösungsgedanken der vorliegenden Erfindung Gebrauch machen, wie in den beigefügten Patentansprüchen beansprucht.

### Bezugszeichenliste

- 1: Transportbehälter
- 2: Oberseite
- 3: Unterseite
- 4: Längsseite
- 5: Seitenwand
- 5a: vordere Seitenwand
- 5b: hintere Seitenwand
- 6: Rand
- 7: elastische Dichtung
- 8: Kleberand
- 9: Zugriffsöffnung
- 10: Abdeckung / Schutzfolie
- 11: Flansch
- 12: Vorsprung
- 120: Stufe
- 13: Aussparung
- 130: weitere Aussparung
- 14: Durchbrechung mit gasdurchlässiger Schutzfolie
- 15: vordere Seitenwand
- 16: abgeschrägter Rand
- 17: Deckel
- 18: abgeschrägter Rand
- 19: elastische Lasche
- 20: Führungsschiene

- 24: äußerer abgedichteter Raum

- 26: Vertiefung
- 27: Haltesteg

- 29: Spalt
- 30: Seitenwand des zweiten Reinraums B
- 300: Öffnung des zweiten Reinraums B
- 301: Klemmschiene
- 302: vordere Begrenzungswand der Klemmschiene 301
- 303: Seitenwand der Klemmschiene 301
- 304: Klemmkanal
- 305: Anschlag oder Ablage

- 308: verstellbare Klemmeinrichtung
- 309: verstellbare Klemmeinrichtung

- 31: abgeschrägter Rand
- 32: Schleusentür
- 33: abgeschrägter Rand
- 34: Dichtungselement
- 35: Schwenkarm
- 36: Schwenkachse
- 37: umlaufender Vorsprung
- 38: elastisches Dichtungselement
- 39: elastisches Dichtungselement
- 40: Haltearm
- 41: Heizelement
- 42: Saugöffnungen
- 43: Saugleitung
- 44: Verbindungsleitung zu Saugeinrichtung (nicht gezeigt)
- 45: Verrastungsarm
- 46: Schwenkachse
- 47: Verrastungselement

- 50: Führungsschiene

- 55: Haltestruktur

- 58: Verpackungsbeutel
- 59: Innenvolumen von Verpackungsbeutel 58
- 60: Oberteil
- 61: Boden des Oberteils 60
- 62: Seitenwand des Oberteils 60

- 63: vordere / hintere Seitenwand des Oberteils 60

- 65: Seitenwand
- 66: Querstege
- 67: Aufnahmen
- 68: unterer Rand

- 70: Unterteil
- 71: Boden des Unterteils 70
- 72: Seitenwand des Unterteils 70
- 73: vordere / hintere Seitenwand des Unterteils 70
- 74: Seitenwand
- 75: Querstege
- 76: vordere Seitenlasche
- 77: Aufnahmen
- 78: Vorsprung

- 80: Zwischenteil
- 81: Boden des Zwischenteils 80
- 83: vordere Seitenwand des Zwischenteils 80
- 84: Seitenwand des Zwischenteils 80

- 85: Randverstärkung
- 86: Sollbruchlinie / linienförmiger Schwächungsbereich
- 87: innerer umlaufender Abschnitt
- 88: äußerer umlaufender Abschnitt

- 90: Haltestruktur
- 91: Öffnungen
- 92: Haltearm
- 93: Scharnier
- 94: wegschwenkbarer Rand von Haltestruktur 90
- 95a: Vorsprung
- 95b: Vertiefung

- 100: Behälter
- 101: Boden des Behälters 100
- 102: Seitenwand des Behälters 100
- 103: verengter Halsabschnitt des Behälters 100
- 104: verbreiterter oberer Rand des Behälters 100
- 105: Befüllöffnung des Behälters 100

- A: erster Reinraum
- B: zweiter Reinraum
- C: dritter Reinraum

## Patentansprüche

1. Verfahren zum Überführen einer Mehrzahl von Behältern (100) zur Aufbewahrung von Substanzen für medizinische, pharmazeutische oder kosmetische Zwecke und/oder von Verschlusselementen hierfür aus einem Transport- und Verpackungsbehälter (1) in einen Reinraum (B), wobei der Transport- und Verpackungsbehälter (1) zumindest eine Seitenwand (5, 2, 4; 5a, 5b) aufweist, in der eine Zugriffsöffnung (9) ausgebildet ist, die mittels einer gasdurchlässigen Schutzfolie (10) steril abgedichtet ist, oder in der eine Öffnung ausgebildet ist, die durch einen rahmenartigen gasundurchlässigen Deckel (17) mit einer Zugriffsöffnung zum Innenraum des Transport- und Verpackungsbehälters (1) verschlossen ist, die mittels einer gasdurchlässigen Schutzfolie (10) steril abgedichtet ist, mit den Schritten:
Anordnen des Transport- und Verpackungsbehälters (1) mit der Mehrzahl von darin aufgenommenen Behältern (100) und/oder Verschlusselementen, sodass die Seitenwand (5, 2, 4; 5a, 5b) des Transport- und Verpackungsbehälters (1) unmittelbar an einer Seitenwand (30) des Reinraums (B) und in unmittelbarer Nähe zu einer Schleusentür (32) des Reinraums (B) angeordnet ist;
Öffnen der Schleusentür (32), wobei durch Koppeln der gasdurchlässigen Schutzfolie (10) mit der Schleusentür (32) gleichzeitig die gasdurchlässige Schutzfolie (10) von der Seitenwand (5, 2, 4; 5a, 5b) oder dem rahmenartigen gasundurchlässigen Deckel (17) des Transport- und Verpackungsbehälters (1) getrennt wird und die Zugriffsöffnung (9) des Transport- und Verpackungsbehälters (1) mit einem Innenraum des Reinraums (B) in Verbindung steht;
Überführen der Mehrzahl von Behältern (100) und/oder Verschlusselementen aus dem Transport- und Verpackungsbehälter (1) in den Innenraum des Reinraums (B); und
Schließen der Schleusentür (32).

2. Verfahren nach Anspruch 1, wobei die Seitenwand (5, 2, 4; 5a, 5b) des Transport- und Verpackungsbehälters (1) so nahe an der Seitenwand (30) des Reinraums (B) angeordnet wird, dass ein Spalt (29) zwischen der Seitenwand des Transport- und Verpackungsbehälters (1) und der Seitenwand (30) des Reinraums (B) mittels eines Dichtungselements (37, 38; 7) abgedichtet ist, wobei das Dichtungselement (37, 38; 7) bevorzugt ein elastisches Dichtungselement ist, das auf der Seitenwand (5, 2, 4; 5a, 5b) des Transport- und Verpackungsbehälters (1) und/oder auf der Seitenwand (30) des Reinraums (B) angeordnet ist.

3. Verfahren nach einem der vorherigen Ansprüche, wobei die Kopplung der gasdurchlässigen Schutzfolie (10) mit der Schleusentür (32) durch Verrastung, durch vorübergehende Fixierung der Schutzfolie an der Außenseite der Schleusentür (32) mittels verstellbaren Greifern (40) oder durch Ansaugen der Schutzfolie (10) gegen die Außenseite der Schleusentür (32) mittels Saugeinrichtungen (42) erfolgt.

4. Verfahren nach einem der vorhergehenden Ansprüche, wobei die gasdurchlässige Schutzfolie (10) eine gasdurchlässige Kunststofffolie, bestehend aus einem Geflecht von Kunststofffasem, beispielsweise aus Polypropylen-Fasern (PP), die auf die Seitenwand (5, 2, 4; 5a, 5b) oder den rahmenartigen gasundurchlässigen Deckel (17) des Transport- und Verpackungsbehälters (1) aufgeklebt ist.

5. Verfahren nach Anspruch 4, mit dem weiteren Schritt,
dass der Innenraum des Transport- und Verpackungsbehälters (1) und/oder die darin aufgenommenen Behälter (100) und/oder Verschlusselemente zur Bereitstellung des Transport- und Verpackungsbehälters (1) durch Einströmen eines Gases oder Dampfes durch die sterile, gasdurchlässige Schutzfolie (10) hindurch sterilisiert wird bzw. werden.

6. Verfahren nach Anspruch 4 oder 5, wobei an der Schleusentür (32) in Entsprechung zu einem Kleberand (8), entlang dem die gasdurchlässige Kunststofffolie auf den Rand (6) der Seitenwand (5, 2, 4; 5a, 5b) des Transport- und Verpackungsbehälters (1) oder auf den rahmenartigen gasundurchlässigen Deckel aufgeklebt ist, eine Heizeinrichtung (41) angeordnet ist, wobei der Kleberand (8) durch Aktivieren der Heizeinrichtung (41) erwärmt und erweicht wird, und
die gasdurchlässige Kunststofffolie so mit der Schleusentür (32) gekoppelt wird, dass die gasdurchlässige Kunststofffolie nach dem Erweichen des Kleberands (8) durch Öffnen der Schleusentür (32) von der Seitenwand (5, 2, 4; 5a, 5b) oder dem rahmenartigen gasundurchlässigen Deckel des Transport- und Verpackungsbehälters (1) abgezogen wird.

7. Verfahren nach einem der Ansprüche 4 bis 6, wobei
die gasdurchlässige Schutzfolie (10) auf der Seitenwand (5, 2, 4; 5a, 5b) des Transport- und Verpackungsbehälters (1) oder auf einem darauf ausgebildeten rahmenartigen Vorsprung (12) angeordnet ist,
in der Seitenwand (5, 2, 4; 5a, 5b) oder dem rahmenartigen Vorsprung (12) eine Mehrzahl von Ausnehmungen oder Vertiefungen (13) ausgebildet sind, und
auf der Außenseite der Schleusentür (32) korrespondierend zu den Ausnehmungen oder Vertiefungen (13) verstellbare Greifer (40) angeordnet sind, wobei
die Greifer (40) so verstellt werden, dass
der Transport- und Verpackungsbehälter (1) in einer ersten Stellung (Fig. 3b) der Greifer (40) ungehindert in die Nähe der Schleusentür (32) des Reinraums (B) gebracht wird,
die Greifer (40) anschließend in eine zweite Stellung (Fig. 3c) verstellt werden, in welcher die Greifer (40) in die entsprechenden Ausnehmungen oder Vertiefungen (13) in der Seitenwand (5, 2, 4; 5a, 5b) oder dem rahmenartigen Vorsprung (12) eingreifen und dabei die gasdurchlässige Schutzfolie (10) hintergreifen, um die gasdurchlässige Schutzfolie (10) vorübergehend an der Schleusentür (32) zu fixieren, und wobei
die Schleusentür (32) in der zweiten Stellung der Greifer (40) geöffnet wird und
die Greifer (40) nach Schließen der Schleusentür (32) zurück in die erste Stellung (Fig. 3b) verstellt werden.

8. Verfahren nach einem der vorhergehenden Ansprüche, wobei die Behälter (100) und/oder Verschlusselemente gemeinsam in einer Haltestruktur (55) gehalten sind, die in dem Transport- und Verpackungsbehälter (1) aufgenommen ist, und wobei die Haltestruktur (55) gemeinsam mit den daran gehaltenen Behältern (100) durch Verschieben aus dem Transport- und Verpackungsbehälter (1) in den Innenraum des Reinraums (B) überführt werden, wobei
die Haltestruktur (55) bevorzugt ein kastenförmiges Unterteil (70) mit einem Boden (71) aufweist, auf dem die Behälter (100) unmittelbar abgestützt sind, wobei das kastenförmige Unterteil (70) bevorzugt unmittelbar auf einem Boden des Transport- und Verpackungsbehälters (1) verschoben und in den Reinraum (B) überführt werden.

9. Verfahren nach Anspruch 7 oder 8, wobei die Haltestruktur (55) weiter ein kastenförmiges Oberteil (60) aufweist, das unmittelbar oder mittelbar unter Zwischenschaltung eines Zwischenteils (80) auf den oberen Enden (104) der Behälter (100) aufliegt.

10. Verfahren nach einem der vorgehenden Ansprüche, wobei der Transport- und Verpackungsbehälter (1) in einem sterilen Verpackungsbeutel (58) steril verpackt bereitgestellt wird und dem Reinraum (B) ein erster Raum, insbesondere ein erster Reinraum (A), mit einer höheren Partikelkonzentration vorgeschaltet ist, mit den weiteren Schritten:
Entkeimen einer Außenseite des Verpackungsbeutels (58) in dem ersten Raum (A); und
Entnehmen des Transport- und Verpackungsbehälters (1) aus dem sterilen Verpackungsbeutel (58) nach dem Entkeimen.

11. Verfahren nach Anspruch 10, bei dem die Mehrzahl von Behältern (100) nach einer weiteren Verarbeitung in dem Reinraum (B) mittels der folgenden Schritte zurück in den Transport- und Verpackungsbehälter überführt wird:
Anordnen des Transport- und Verpackungsbehälters (1) in dem ersten Raum, insbesondere in dem ersten Reinraum (A), mit der höheren Partikelkonzentration, sodass die Seitenwand (5, 2, 4; 5a, 5b) des Transport- und Verpackungsbehälters (1) mit der Zugriffsöffnung unmittelbar an der Seitenwand (30) des Reinraums (B) und in unmittelbarer Nähe zu der Schleusentür (32) des Reinraums (B) angeordnet ist;
Öffnen der Seitenwand (5, 2, 4; 5a, 5b) des Transport- und Verpackungsbehälters (1) und der Schleusentür (32), sodass die Zugriffsöffnung (9) des Transport- und Verpackungsbehälters (1) mit dem Innenraum des Reinraums (B) in Verbindung steht;
Überführen der Mehrzahl von Behältern (100) aus dem Innenraum des Reinraums (B) in den Transport- und Verpackungsbehälter (1);
Schließen der Schleusentür (32); und
Schließen des Transport- und Verpackungsbehälters (1) mittels einer Abdeckung (10; 17), insbesondere bewirkt durch ein bevorzugt gleichzeitiges Schließen der Schleusentür (32).

12. Verfahren nach Anspruch 10, weiterhin umfassend:
Entkeimen der Außenseite des Transport- und Verpackungsbehälters (1) in dem ersten Raum, insbesondere in dem ersten Reinraum (A), mit der höheren Partikelkonzentration nach dem Überführen der Mehrzahl von Behältern in den Transport- und Verpackungsbehälter (1);
Einbringen des Transport- und Verpackungsbehälters (1) nach dem Entkeimen der Außenseite in einen sterilen Verpackungsbeutel (58); und
Verschließen des sterilen Verpackungsbeutels (58).

13. Transport- und Verpackungsbehälter (1) zur Aufbewahrung von Behältern für Substanzen für medizinische, pharmazeutische oder kosmetische Zwecke und/oder von Verschlusselementen hierfür in einer vertikalen Ausrichtung, insbesondere zur Verwendung in einem Verfahren nach einem der vorhergehenden Ansprüche, wobei
der Transport- und Verpackungsbehälter (1) quaderförmig ist und eine Oberseite (2), eine Unterseite (3) und vier Seitenwände (5, 2, 4; 5a, 5b) aufweist,
die Behälter und/oder von Verschlusselemente in der vertikalen Ausrichtung senkrecht zu der Oberseite (2) und der Unterseite (3) ausgerichtet sind,
jeweils zwei Seitenwände parallel und beabstandet zueinander gegenüberliegend angeordnet sind,
zumindest eine der Seitenwände (5, 2, 4; 5a, 5b) eine Zugriffsöffnung (9) aufweist, die mittels einer Abdeckung (10; 17) steril abgedichtet ist, und
die jeweilige Abdeckung (10; 17) abnehmbar ist, um einen Innenraum des Transport- und Verpackungsbehälters (1) zur Entnahme der in dem Transport- und Verpackungsbehälter (1) aufgenommenen Behälter über die Zugriffsöffnung (9) freizugeben,
**dadurch gekennzeichnet, dass** die Abdeckung eine gasdurchlässige Schutzfolie (10) ist, die die Zugriffsöffnung in der zumindest einen Seitenwand oder in einem rahmenartigen gasundurchlässigen Deckel (17) steril abdichtet, der eine Öffnung in der zumindest einen Seitenwand verschließt, sodass der Innenraum des Transport- und Verpackungsbehälters (1) und/oder der darin aufgenommenen Behälter und/oder Verschlusselemente durch Einströmen eines Gases durch die gasdurchlässige Schutzfolie sterilisiert werden können und die Behälter und/oder Verschlusselemente durch Ablösen der gasdurchlässigen Schutzfolie (10) von der zumindest einen Seitenwand oder dem rahmenartigen gasundurchlässigen Deckel (17) durch die Zugriffsöffnung seitlich und in der vertikalen Ausrichtung aus dem Transport- und Verpackungsbehälter (1) entnommen werden können.

14. Transport- und Verpackungsbehälter nach Anspruch 13, wobei an dem Transport- und Verpackungsbehälter (1) zumindest eine Kopplungseinrichtung (11; 130) zur vorübergehenden Kopplung des Transport- und Verpackungsbehälters (1) an einer Seitenwand eines Reinraums (B) vorgesehen ist, wobei die jeweilige Kopplungseinrichtung (11; 130) außerhalb der Abdeckung (10; 17) und der Zugriffsöffnung (9) des Transport- und Verpackungsbehälters (1) vorgesehen ist.

15. Transport- und Verpackungsbehälter (1) nach Anspruch 14, wobei um die gasdurchlässige Schutzfolie oder Abdeckung herum auf der jeweiligen Seitenwand des Transport- und Verpackungsbehälters ein umlaufender, flacher oder ebener Anlageabschnitt (6) ausgebildet ist, sodass bei Anlage des umlaufenden Anlageabschnitts (6) an einem korrespondierend ausgebildeten umlaufenden Anlageabschnitt (30) der Seitenwand eines Reinraums (B) ein Spalt (29) zwischen der jeweiligen Abdeckung (10; 17) und der Seitenwand des Reinraums (B) mittels eines elastischen Dichtungselements (37, 38; 7) gegen die Umgebung hin abdichtbar ist.

## Claims

1. A method for transferring a plurality of containers (100) for storage of substances for medical, pharmaceutical or cosmetic purposes and/or of closure elements for such containers from a transport and packaging container (1) into a clean room (B), wherein the transport and packaging container (1) has at least one side wall (5, 2, 4; 5a, 5b) in which an access opening (9) is formed, which is sterile sealed by means of a gas-permeable protective sheet (10), or in which an opening is formed, which is sealed by a frame-like, gas-impermeable cover (17) having an access opening against the interior of the transport and packaging container (1), which is sterile sealed by means of a gas-permeable sheet (10), comprising the steps of:
placing the transport and packaging container (1) together with the plurality of containers (100) and/or closure elements accommodated therein, so that the side wall (5, 2, 4; 5a, 5b) of the transport and packaging container (1) is disposed directly at a side wall (30) of the clean room (B) and in close proximity to a transfer port door (32) of the clean room (B);
opening the transfer port door (32), wherein by coupling the gas-permeable protective sheet (10) with the transfer port door (32) the gas-permeable protective sheet (10) is separated from the side wall (5, 2, 4; 5a, 5b) or from the frame-like, gas-impermeable cover (17) of the transport and packaging container (1) at the same time and the access opening (9) of the transport and packaging container (1) is in communication with an inside space of the clean room (B);
transferring the plurality of containers (100) and/or of closure elements from the transport and packaging container (1) into the inside space of the clean room (B); and
closing the transfer port door (32).

2. The method as claimed in claim 1, wherein the side wall (5, 2, 4; 5a, 5b) of the transport and packaging container (1) is placed so close to the side wall (30) of the clean room (B) that a gap (29) between the side wall of the transport and packaging container (1) and the side wall (30) of the clean room (B) is sealed by means of a sealing element (37, 38; 7), wherein the sealing element (37, 38; 7) is preferably an elastic sealing element, which is disposed on the side wall (5, 2, 4; 5a, 5b) of the transport and packaging container (1) and/or on the side wall (30) of the clean room (B).

3. The method as claimed in any of the preceding claims, wherein the coupling of the gas-permeable protective sheet (10) with the transfer port door (32) is performed by latching, by temporarily fixing the gas-permeable protective sheet on the outside of the transfer port door (32) by means of adjustable grippers (40) or by sucking the gas-permeable protective sheet (10) against the outside of the transfer port door (32) by means of suction devices (42).

4. The method as claimed in any of the preceding claims, wherein the gas-permeable protective sheet (10) is a gas-permeable plastic film consisting of a mesh of plastics fibers such as polypropylene fibers (PP) that is bonded on the side wall (5, 2, 4; 5a, 5b) or on the frame-like, gas-impermeable cover (17) of the transport and packaging container (1).

5. The method as claimed in claim 4, further comprising
sterilizing the inside space of the transport and packaging container (1) and/or the containers (100) accommodated therein and/or closure elements by a flow of a gas or vapor through the sterile, gas-permeable protective sheet (10), for providing the transport and packaging container (1).

6. The method as claimed in claim 4 or 5, wherein a heating device (41) is disposed at the transfer port door (32) in correspondence with an adhesive rim (8) along which the gas-permeable plastic foil is bonded to the edge (6) of the side wall (5, 2, 4; 5a, 5b) of the transport and packaging container (1) or to the frame-like, gas-impermeable cover, wherein
the adhesive rim (8) is heated and softened by activating the heating device (41), and
the gas-permeable plastic film is coupled with the transfer port door (32) in such a manner that the gas-permeable plastic film is pulled off from the side wall (5, 2, 4; 5a, 5b) of the transport and packaging container (1) or from the frame-like, gas-impermeable cover by opening the transfer port door (32) after the softening of the adhesive rim (8).

7. The method as claimed in any of claims 4 to 6, wherein
the gas-permeable protective sheet (10) is disposed on the side wall (5, 2, 4; 5a, 5b) of the transport and packaging container (1) or on a frame-like protrusion (12) formed thereon, ,
a plurality of recesses or depressions (13) is formed in the side wall (5, 2, 4; 5a, 5b) or on the frame-like protrusion (12), and
adjustable gripping devices (40) are disposed on the outside of the transfer port door (32) corresponding to the recesses or depressions (13), wherein
the gripping devices (40) are adjusted such that
, in a first position (Fig. 3b) of the gripping devices (40), the transport and packaging container (1) is brought freely to the vicinity of the transfer port door (32) of the clean room (B),
the gripping devices (40) are then adjusted to a second position (Fig. 3c), in which the gripping devices (40) engage with the corresponding recesses or depressions (13) in the side wall (5, 2, 4; 5a, 5b) or frame-like protrusion (12) while engaging behind the gas-permeable protective sheet (10) for temporarily fixing the gas-permeable protective sheet (10) at the transfer port door (32), and wherein
the transfer port door (32) is opened in the second position of the gripping devices (40) and
the gripping devices (40) are adjusted back to the first position (Fig. 3b) after closing the transfer port door (32).

8. The method as claimed in any of the preceding claims, wherein all the containers (100) and/or closure elements are supported in a supporting structure (55) which is accommodated in said transport and packaging container (1) and wherein the supporting structure (55) together with the containers (100) and/or closure elements supported by it are transferred from the transport and packaging container (1) into the inside space of the clean room (B) by shifting, wherein
the supporting structure (55) preferably comprises a box-shaped bottom part (70) having a bottom (71), on which the containers (100) are directly supported, wherein the box-shaped bottom part (70) is preferably shifted directly on a bottom of the transport and packaging container (1) and transferred to the clean room (B).

9. The method as claimed in claim 7 or 8, wherein the supporting structure (55) further comprises a box-shaped upper part (60), which rests directly, or indirectly with the interposition of an intermediate part (80), on the upper ends (104) of the containers (100).

10. The method as claimed in any of the preceding claims, wherein the transport and packaging container (1) is provided sterile packaged inside a sterile packaging bag (58) and wherein a first space, in particular a first clean room (A) having a higher concentration of particles, is disposed upstream of the clean room (B), said method further comprising the steps of:
sterilizing an outside of the packaging bag (58) in the first space (A); and
removing the transport and packaging container (1) from the sterile packaging bag (58) after the step of sterilizing.

11. The method as claimed in claim 10, wherein the plurality of containers (100) is transferred back into the transport and packaging container after a further processing in the clean room (B) by means of the following steps:
placing the transport and packaging container (1) in the first space, in particular in the first clean room (A) having the higher concentration of particles, so that the side wall (5, 2, 4; 5a, 5b) of the transport and packaging container (1) with the access opening is arranged directly at the side wall (30) of the clean room (B) and in close proximity to the transfer port door (32) of the clean room (B);
opening the side wall (5, 2, 4; 5a, 5b) of the transport and packaging container (1) and the transfer port door (32) so that the access opening (9) of the transport and packaging container (1) communicates with the inside space of the clean room (B);
transferring the plurality of containers (100) from the inside space of the clean room (B) into the transport and packaging container (1);
closing the transfer port door (32); and
closing the transport and packaging container (1) by means of a cover (10; 17), in particular effected by a preferably simultaneous closing of the transfer port door (32).

12. The method as claimed in claim 10, further comprising:
sterilizing the outside of the transport and packaging container (1) in the first space, in particular in the first clean room (A) having the higher concentration of particles, after transferring the plurality of containers into the transport and packaging container (1);
inserting the transport and packaging container (1) into a sterile packaging bag (58) after the step of sterilizing the outside; and
sealing the sterile packaging bag (58).

13. A transport and packaging container (1) for the storage of containers for substances for medical, pharmaceutical or cosmetic purposes and/or for the storage of closure elements for such containers in a vertical orientation, in particular for use in a method as claimed in any of the preceding claims, wherein
the transport and packaging container (1) is box-shaped and comprises an upper side (2), a bottom (3) and four side walls (5, 2, 4; 5a, 5b),
the containers and/or closure elements are aligned in the vertical orientation perpendicular to the upper side (2) and to the bottom (3),
respective pairs of side walls are arranged in parallel with each other and spaced to each other,
at least one of the side walls (5, 2, 4; 5a, 5b) has an access opening (9), which is sealed sterile by means of a cover (10; 17), and
the respective cover (10; 17) is removable for providing access to an inside space of the transport and packaging container (1) for the removal of the containers accommodated in the transport and packaging container (1) via the access opening (9),
**characterized in that** the cover is a gas-permeable protective sheet (10) which sterile seals the access opening in the at least one side wall or in a frame-like, gas-impermeable cover (17), which seals an opening in the at least one side wall, so that the inside space of the transport and packaging container (1) and/or containers accommodated therein and/or closure elements can be sterilized by a gas flowing in through the gas-permeable protective sheet and so that the containers and/or closure elements can be removed via the access opening laterally and in the vertical orientation from the transport and packaging container (1) by removing the gas-permeable sheet from the at least one side wall or from the frame-like, gas-impermeable cover (17)

14. The transport and packaging container of claim 13, wherein at least one coupling device (11; 130) is provided on the transport and packaging container (1) for the temporary coupling of the transport and packaging container (1) with a side wall of a clean room (B), wherein the respective coupling device (11; 130) is disposed outside of the cover (10; 17) and of the access opening (9) of the transport and packaging container (1).

15. The transport and packaging container (1) of claim 14, wherein a circumferential, flat or planar abutment portion (6) is formed around the gas-permeable protective sheet or cover and on the respective side wall of the transport and packaging container, so that a gap (29) formed between the respective cover (10; 17) and the side wall of the clean room (B) is sealed against the environment by means of an elastic sealing member (37, 38; 7), when the circumferential abutment portion (6) abuts to a corresponding circumferential contact portion (30) of the side wall of a clean room (B).

## Revendications

1. Un procédé pour transférer une pluralité de récipients (100) pour stocker des substances à des fins médicales, pharmaceutiques ou cosmétiques et/ou des éléments de fermeture de tels récipients à partir d'un conteneur de transport et d'emballage dans une enceinte blanche (B), dans laquelle le conteneur de transport et d'emballage (1) comporte au moins une paroi latérale (5, 2, 4; 5a, 5b) dans laquelle est formée une ouverture d'accès (9), qui est scellée de manière stérile au moyen d'un film protecteur perméable aux gaz (10), ou dans laquelle est formée une ouverture, qui est scellée par un couvercle imperméable aux gaz (17) en forme de cadre ayant une ouverture d'accès contre l'intérieure du récipient de transport et d'emballage (1), qui est scellé de manière stérile au moyen d'une feuille perméable au gaz (10), comprenant les étapes consistant à :
placer le récipient de transport et d'emballage (1) avec la pluralité de récipients (100) et/ou des éléments de fermeture y logés, de telle façon que la paroi latérale (5, 2, 4 ; 5a, 5b) du récipient de transport et d'emballage (1) est disposé directement à une paroi latérale (30) dans la chambre blanche (B) et à proximité proche d'une porte de transfert (32) de la chambre blanche (B) ;
ouvrir la porte de transfert (32), dans lequel, en couplant la feuille protectrice perméable au gaz (10) avec la porte de transfert (32), la feuille protectrice perméable au gaz (10) est séparée de la paroi latérale (5, 2, 4 ; 5a, 5b) ou du couvercle imperméable au gaz (17) en forme de cadre du récipient de transport et d'emballage (1) en même temps et l'ouverture d'accès (9) du récipient de transport et d'emballage (1) est en communication avec l'espace intérieur de la chambre blanche (B) ;
transférer la pluralité de récipients (100) et/ou les éléments de fermeture du récipient de transport et d'emballage (1) à l'intérieur de l'espace intérieur de la chambre blanche (B) ;
fermer la porte de transfert (32).

2. Le procédé tel que revendiqué dans la revendication 1, dans lequel la paroi latérale (5, 2, 4; 5a, 5b) du récipient de transport et d'emballage (1) est disposée si près de la paroi latérale (30) de la chambre blanche (B) qu'un espace (29), entre la paroi latérale du récipient de transport et d'emballage (1) et la paroi latérale (30) de la chambre blanche (B), est scellé au moyen d'un élément de scellement (37, 38, 7), dans lequel l'élément de scellement (37, 38, 7) est de préférence un élément de scellement élastique, qui est disposé sur la paroi latérale (5, 2, 4 ; 5a, 5b) du récipient de transport et d'emballage (1) et/ou sur la paroi latérale (30) de la chambre blanche (B).

3. Le procédé tel que revendiqué dans l'une quelconque des revendications précédentes, dans lequel l'accouplement du film protecteur perméable aux gaz (10) avec la porte de transfert (32) est effectué par encliquetage, par fixation temporaire du film protecteur perméable au gaz sur l'extérieur de la porte de transfert (32) au moyen de pinces réglables (40) ou en aspirant le film protecteur perméable au gaz (10) contre l'extérieur de la porte de transfert (32) au moyen de dispositifs d'aspiration (52).

4. Le procédé tel que revendiqué dans l'une quelconque des revendications précédentes, dans lequel le film protecteur perméable aux gaz (10) est un film plastique perméable aux gaz, constitué d'une maille de fibres plastiques, par exemple de fibres de polypropylène (PP) qui est collé sur la paroi latérale (5, 2, 4, 5a, 5b) ou sur le couvercle imperméable au gaz (17), de type cadre, du récipient de transport et d'emballage (1).

5. Le procédé tel que revendiqué dans la revendication 4, comprenant en outre : la stérilisation de l'espace intérieure du récipient de transport et d'emballage (1) et/ou les récipients (100) y logées, et/ou les éléments de fermeture au moyen d'un flux de gaz ou de vapeur au travers le film protecteur stérile (10), perméable au gaz, pour fournir le récipient de transport et d'emballage (1).

6. Le procédé tel que revendiqué dans la revendication 4 ou 5, dans lequel un dispositif de chauffage (41) est disposé à la porte de transfert (32) en correspondance avec un bord adhésif (8) le long duquel le film plastic perméable au gaz est collé au bord (6) de la paroi latérale (5, 2, 4, 5a, 5b) du récipient de transport et d'emballage (1) ou du couvercle imperméable au gaz, de type cadre, dans lequel
le bord adhésif (8) est chauffé et ramolli par l'activation du dispositif de chauffage (41), et
le film plastic perméable au gaz est couplé avec la porte de transfert (32) de telle façon que le film plastic perméable au gaz est retiré de la paroi latérale (5, 2, 4 ; 5a, 5b) du récipient de transport et d'emballage (1) ou du couvercle imperméable au gaz de type cadre, par l'ouverture de la porte de transfert (32) à la suite du ramollissement du bord adhésif (8).

7. Le procédé tel que revendiqué dans l'une quelconque des revendications 4 à 6, dans lequel
le film protecteur perméable aux gaz (10) est disposé sur la paroi latérale (5, 2, 4; 5a, 5b) du récipient de transport et d'emballage (1) ou sur une saillie en forme de cadre (12) formée sur celle-ci,
une pluralité d'évidements ou de creux (13) sont formés dans la paroi latérale (5, 2, 4 ; 5a, 5b) ou sur la saille en forme de cadre (12), et
des dispositifs de pinces ajustables (40) sont disposés sur l'extérieur de la porte de transfert (32) correspondant aux évidements ou creux (13), dans lequel
les dispositifs de pinces (40) sont ajustées de telle façon que,
dans une première position (Fig. 3b) des dispositifs de pinces (40), le récipient de transport et d'emballage (1) est apporté librement à proximité de la porte de transfert (32) de la chambre blanche (B),
les dispositifs de pinces (40) sont ensuite ajustées dans une deuxième position (figure 3c), dans laquelle les dispositifs de pinces (40) sont insérées dans les évidements ou creux correspondants (13) dans la paroi latérale (5, 2, 4, 5a, 5b) ou de la saille en forme de cadre (12) tandis que mis en prise derrière le film protecteur perméable aux gaz (10) pour fixer temporairement le film protecteur perméable aux gaz (10) sur la porte de transfert (32), et dans lequel
la porte de transfert (32) est ouverte dans la seconde position des dispositifs de pinces (40) et
les dispositifs de pince (40) sont ajustés en arrière vers la première position ( Fig. 3B) après avoir fermé la porte de transfert (32).

8. Le procédé tel que revendiqué dans l'une quelconque des revendications précédentes, dans lequel les récipients (100) et/ou les éléments de fermeture sont maintenus ensemble dans une structure de maintien (55) logée dans le récipient de transport et d'emballage (1) et dans lequel la structure de maintien (55) avec les récipients (100) et/ou les éléments de fermeture supportés par lui sont transférés depuis le récipient de transfert et d'emballage (1) dans l'espace interne de la chambre blanche (B) par déplacement, dans lequel
la structure de support (55) comporte de préférence une partie inférieure (70) en forme de boîte ayant un fond (71), sur lequel les récipients (100) sont directement supportés, dans lequel la partie inférieure en forme de boîte (70) est de préférence déplacée directement sur un fond du récipient de transport et d'emballage (1) et transférée vers la chambre blanche.

9. Le procédé tel que revendiqué dans la revendication 7 ou 8, dans lequel la structure de maintien (55) comprend en outre une partie supérieure (60) en forme de boîte qui repose directement ou indirectement sur une partie intermédiaire (80) sur les extrémités supérieures (104) des récipients (100).

10. Le procédé tel que revendiqué dans l'une quelconque des revendications précédentes, dans lequel le récipient de transport et d'emballage (1) est fourni stérile à l'intérieur d'un sac d'emballage stérile (58) et dans lequel un premier espace, en particulier une première chambre blanche (A) ayant une concentration de particules, est disposé en amont de la chambre blanche (B), ledit procédé comprenant en outre les étapes consistant à :
stériliser un extérieur du sac d'emballage (58) dans la première chambre blanche (A) ; et
retirer le récipient de transport et d'emballage (1) du sac d'emballage stérile (58) postérieurement à l'étape de stérilisation.

11. Un procédé tel que revendiqué dans la revendication 10, dans lequel la pluralité de récipients (100) est renvoyé dans le récipient de transport et d'emballage postérieure à un traitement additionnel dans la chambre blanche (B) au moyens des étapes suivantes :
placer le récipient de transport et d'emballage (1) dans le premier espace, en particulier la première chambre blanche (A) ayant une concentration plus élevée de particules, de telle façon que la paroi latérale (5, 2, 4 ; 5a, 5b) du récipient de transport et d'emballage (1) est disposé, avec l'ouverture d'accès, directement à la paroi latérale (30) de la chambre blanche (B) et à proximité de la porte de transfert (B) de la chambre blanche (B) ;
ouvrir la paroi latérale (5, 2, 4 ; 5a, 5b) du récipient de transport et d'emballage (1) et la porte de transfert (32) de telle façon que l'ouverture d'accès (9) du récipient de transport et d'emballage (1) communiquent avec l'espace intérieur de la chambre blanche (B) ;
transférer la pluralité de récipients (100) depuis l'espace intérieur de la chambre blanche (B) dans le récipient de transport et d'emballage (1) ;
refermer la porte de transfert (32) ; et
refermer le récipient de transport et d'emballage (1) au moyen d'un couvercle (10 ; 17), en particulier réalisé de préférence simultanément avec la fermeture de la porte de transfert (32).

12. Le procédé tel que revendiqué dans la revendication 10, comprenant en outre :
- stériliser l'extérieur du récipient de transport et d'emballage (1) dans un premier espace, en particulier une première chambre blanche (A) ayant la concentration la plus élevée de particules, postérieurement au transfert de la pluralité des récipients à l'intérieur du récipient de transport et d'emballage (1) ;
- insérer le récipient de transport et d'emballage (1) à l'intérieur d'un sac d'emballage stérile (58) postérieurement à l'étape de stérilisation de l'extérieur ; et
- le scellement du sac d'emballage stérile (58).

13. Un récipient de transport et d'emballage (1) pour le stockage de récipients pour des substances à des fins médicales, pharmaceutiques ou cosmétiques et/ou des éléments de fermeture pour de tels récipients disposés suivant une orientation verticale, en particulier pour une utilisation pour un procédé tel que revendiqué dans l'une quelconque des revendications précédentes, dans lequel
le récipient de transport et d'emballage (1) est en forme de boîte et comporte une paroi supérieure (2), un fond (3) et quatre parois latérales (5, 2, 4 ; 5a, 5b),
les récipients et/ou les éléments de fermeture sont alignés suivant une orientation verticale perpendiculaire à la paroi supérieure (2) et au fond (3),
des paires respectives de parois latérales sont disposées en parallèle et espacées, l'une vis-à-vis de l'autre,
au moins l'une des parois latérales (5, 2, 5 ; 5a, 5b) a une ouverture d'accès (9), qui est scellée stérile au moyen d'un couvercle (10 ; 17), et
le couvercle respectif (10, 17) est détachable pour fournir un accès à un espace interne du récipient de transport et d'emballage pour l'enlèvement des récipients logés dans le récipient de transport et d'emballage (1) via une ouverture d'accès (9),
**caractérisé en ce que** le couvercle est un film protecteur perméable au gaz (10) qui scelle stérilement l'ouverture d'accès and ladite au moins une paroi latérale ou, dans le couvercle imperméable au gaz (17), qui scelle une ouverture dans ladite au moins une paroi latérale, de telle façon que l'espace interne du récipient de transport et d'emballage (1) et/ou des récipients y logés et/ou des éléments de fermeture peuvent être stérilisés par un gaz s'écoulant au travers le film protecteur perméable au gaz et de telle manière que les récipients et/ou les éléments de fermeture peuvent être enlevés latéralement du récipient de transport et d'emballage (1) via l'ouverture d'accès et suivant l'orientation verticale, en enlevant le film perméable au gaz de ladite paroi latérale au moins ou du couvercle imperméable au gaz (17) en forme de cadre.

14. Le récipient de transport et d'emballage de la revendication 13, dans lequel au moins un dispositif de couplage (11 ; 30) est fourni sur le récipient de transport et d'emballage (1) pour le couplage temporaire du récipient de transport et d'emballage (1) avec une paroi latérale d'une chambre blanche (B), dans lequel le dispositif de couplage respectif (11 ; 130) est disposé à l'extérieur du couvercle (10 ; 17) et de l'ouverture d'accès (9) du récipient de transport et d'emballage (1).

15. Le récipient de transport et d'emballage de la revendication 14, dans lequel une partie de butée plate pou plane, circonférentielle est formée autour du film protecteur perméable au gaz ou du couvercle et sur la paroi latérale respective du récipient de transport et d'emballage, de telle façon qu'un espace (29) formé entre le couvercle respectif (10 ; 17) et la paroi latérale de la chambre blanche (B) est scellée vis-à-vis de l'environnement au moyen d'un élément de scellement élastique (37, 38, 7), lorsque la partie de butée circonférentielle (6) est en butée contre une partie de contact circonférentielle correspondante (30) de la paroi latérale de la chambre blanche (B).
